# EUROPEAN PATENT APPLICATION

(11) **EP 2 261 361 A2**
(43) Date of publication of application: **15.12.2010**
(21) Application number: 10008864.0
(22) Date of filing: 15.05.2006
(51) Int. Cl.: C12N 15/82

(54) **Methods for improving crop plant architecture and yield**

(30) Priority: 25.05.2005 US 684617 P
(62) Divisional of application: 06770338.9
(71) Applicant: Pioneer Hi-Bred International Inc., Johnston, IA 50131-1014 (US)
(72) Inventor: Danilevskaya, Olga N., Johnston, Iowa 50131 (US); Ananiev, Evgueni, Johnston, Iowa 50131 (US); Simmons, Carl R., Des Moines, Iowa 50310 (US); Hermon, Pedro, Johnston, Iowa 50131 (US)
(74) Representative: Bentham, Andrew

(57) **Abstract**

The present invention provides methods for altering plant characteristics by introducing into plants isolated nucleic acid molecules encoding plant developmental regulator (PDR) polypeptides that can be used to produce transgenic plants characterized by altered plant architecture, plant maturity, carbon and nitrogen partitioning and/or improved harvestable yield. Also provided are isolated nucleic acids that encode PDR polypeptides, vectors capable of expressing such nucleic acid molecules, host cells containing such vectors, and polypeptides encoded by such nucleic acids. The PDR genes are homologues of the CETS gene family.

## Description

### FIELD OF THE INVENTION

The present invention is drawn to plant genetics and molecular biology. More particularly, the methods involve improving architecture and yield in plants by modulating the expression of nucleic acids within plants. This invention describes a method for improving crop plant architecture and yield in transgenic plants by manipulation of PDR genes which are homologues of the CETS gene family.

### BACKGROUND

The CETS gene family (Pnueli et al., 2001) was named for the three plant genes: Antirrhinum CENTRORADIALIS (CEN) (Bradley *et al*., 1996), Arabidopsis TERMINAL FLOWER 1 (TF1) (Bradley *et al*., 1997), and tomato SELF-PRUING (SP) (Pnueli *et al*., 1998). The CETS homologues are designated PDR (for Plant Developmental Regulators), based upon the expanded knowledge gained about these genes. The three-letter designation is in accordance with plant gene naming standards (Plant Journal, 1997, 12:247-253). The CETS (PDR) genes encoded closely related proteins with similarity to mammalian phosphatidylethanolamine-binding proteins (PEBPs) (Kardailsky *et al*., 1999; Kobayashi *et al*., 1999). Mammalian PEBS proteins have been found to act as inhibitors of MAP kinase signaling. The first studied is RKIP (Raf kinase inhibitor protein) which plays a pivotal role in several protein kinase signaling cascades (Yeung *et al*., 1999; Lorenz et al., 2003). The 3-dimensional structure of the CEN protein suggests that plant CETS/PDR protein interfering with kinases like their mammalian counterparts (Banfield *et al*., 1998; Banfield and Brady, 2000). Biochemical properties of the CETS/PDR protein family indicate their potential roles as modulators of hormone signaling cascades controlling cell growth and differentiation. Being kinase inhibitors/effectors, the CETS/PDR might be involved in regulation of diverse genetic pathways working as modulators of signaling from hormones to target genes in the various cell types or tissues.

Mutational analysis of the CETS/PDR genes in several dicot species has revealed their function in determining the fate of meristem. One group of CETS/PDR genes, such as the LF (Late Flowering) from pea (Foucher *et al*., 2003) and the TFL1 from Arabidopsis (Bradley *et al*., 1997), act as repressors of flowering by maintaining the apical meristem in the vegetative state. The second group of CETS/PDR genes, including DET (DETERMINATE) from pea (Foucher *et al*., 2003), CEN from snapdragon (Cremer *et al*., 2001), SP (SELF-PRUNING) from tomato (Pnueli *et al*., 1998), and TFL1 from Arabidopsis, maintain indeterminancy of the inflorescence meristem by delaying its transition to the flowers. The Arabidopsis TFL1 gene plays a dual role by controlling the length of both vegetative and floral phases (Ratcliffe *et al*., 1998). The Arabidopsis FT (FLOWERING LOCUS T) gene belongs to the CETS gene family as well, but it has a TFL1-antagonist role by promoting flowering, hence, accelerating the transition from vegetative to reproductive phase (Kardailsky *et al*., 1999; Kobayashi *et al*., 1999). The cited literature describes the role of the PDR genes in maintaining the indeterminancy of the shoot apical and inflorescence meristems explaining their roles in controlling flowering time, and the "determinate habit" of shoot growth.

Dicotocyledoneous plants such as arabidopsis, tomato and poplar appear to possess a small PDR gene family of six to eight genes depending upon the species (Mimida *et al*., 2001; Carmel-Goren *et al*., 2003; Kotada, 2005). Consistent with this observation, further analysis revealed 7 PDR genes in soybean. Additional studies have revealed a larger family of the PDR genes in monocot genomes. There are more than 22 PDR genes in the rice and maize genomes. Gene expression analysis performed on the genome-wide scale by MPSS RNA profiling suggests functional diversity of the maize PDR genes. Based on a tissue specific pattern of expression, one finds novel functions for the PDR genes, namely involvement in kernel, leaf and vascular bundle development, and drought stress response. Because of their apparently wider functional roles, the maize PDR genes may be used in genetically modified plants for more diverse outcomes, ranging from improving grain yield, stalk strength, plant biomass, canopy shape and drought tolerance. Because of the high similarity of amino acid sequences of the PDR proteins, judiciously altered ectopic expression of the gene family members may allow for the genes to cross their normal functional roles and affect a number of agronomic traits.

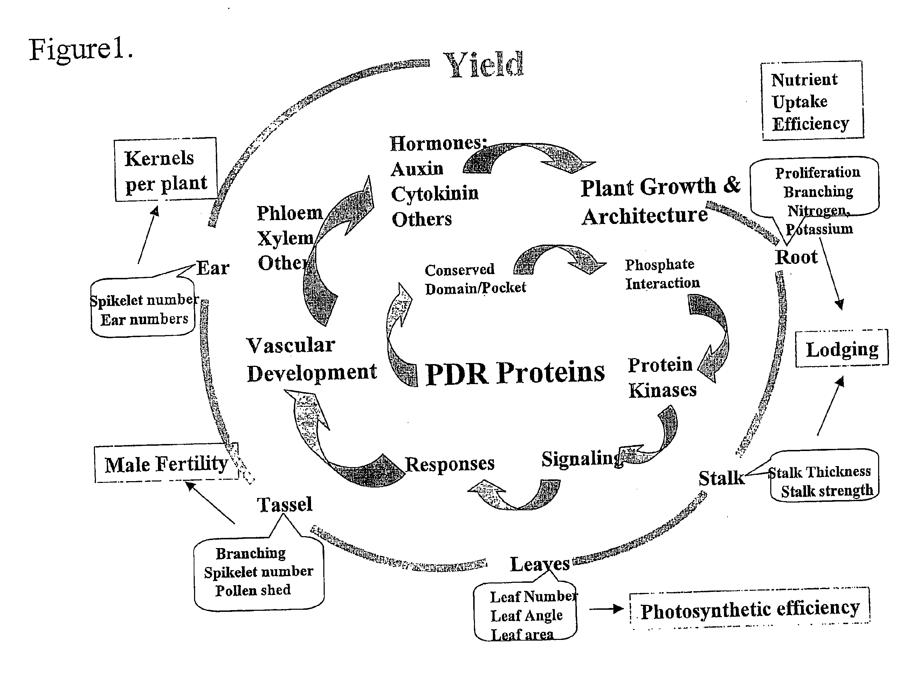

Experiments have demonstrated that ZmPDR01 and ZmPDR02 when linked to a constitutive promoter such as UBI lead to enhancement of multiple agronomic traits in transgenic plants. Maize ZmPDR01 transgenic plants showed on average 22% more spikelets per ear, 78% more spikelets per tassel, 20% larger leaf area, 17% leaf angle increase, and 30% stronger stalks. A large number of valuable agronomic traits have been changed by the action of one protein. The spikelet count per ear is a primary grain yield component. The ZmPDR01 gene, therefore, acts as a genetic factor regulating the ear length which increases the yield potential. Transgenic plants also have a favorable canopy shape, copious pollen and increased stalk strength. Together, these transgene-induced traits will support development of higher yielding varieties and hybrids (Figure 1).

Grain yield in corn is defined as weight of grain harvested per unit area (Duvick, 1992). Yield is one of the most complex agronomic traits and is determined by the interaction of specific genetics within the crops with environmental factors.

There are two general approaches to increasing yield potential: 1) increasing overall plant productivity to increase harvestable yield, and 2) overcoming the negative consequences of any abiotic stresses. Several yield components are critical for harvestable yield in maize: kernel number per ear, photosynthetic capacity, canopy shape, and standability. In the past yield increases have been achieved by breeding efforts via a number of incremental, consecutive steps (Duvick, 1992). The transgenic manipulation of the PDR genes provide a method for improving several yield components, such as kernel number, canopy shape, stalk strength, and vegetative biomass in a single larger step. Also, there is a potential for increased drought tolerance by manipulation of the appropriate class of the maize PDR genes responsive to water availability. Therefore, PDR genes are powerful morphology controlling genes that allow genetic modification of several critical yield components causing increases in both plant productivity and stress tolerance.

### SUMMARY OF THE INVENTION

Compositions and methods for improving crop plant architecture and yield by manipulation of PDR gene family in transgenic plants are provided (Figure 1).

The present invention provides polynucleotides, related polypeptides and conservatively modified variants of the PDR sequences. The polynucleotides and polypeptides of the invention include PDR genes, proteins and functional fragments or variants thereof.

The methods of the invention comprise introducing into a plant a polynucleotide and expressing the corresponding polypeptide within the plant. The sequences of the invention can be used to alter plant cell growth, leading to changes in plant structural architecture, thereby improving plant yield. The methods of the invention find use in improving plant structural characteristics, leading to increased yield.

Additionally provided are transformed plants, plant tissues, plant cells, seeds, and leaves. Such transformed plants, tissues, cells, seeds, and leaves comprise stably incorporated in their genomes at least one polynucleotide molecule of the invention.

One embodiment of the invention is a method for plant characteristics, the method comprising:
a. introducing into a plant cell a recombinant expression cassette comprising a polynucleotide whose expression, alone or in combination with additional polynucleotides, functions as a plant developmental regulator polypeptide within the plant;
b. culturing the plant cell under plant forming conditions to produce a plant; and,
c. inducing expression of the polynucleotide for a time sufficient to alter the architecture of the plant.

A second embodiment would be a method for increasing plant harvestable yield, the method comprising:
a. introducing into a plant cell a recombinant expression cassette comprising a polynucleotide whose expression, alone or in combination with additional polynucleotides, functions as a plant developmental regulator polypeptide within the plant;
b. culturing the plant cell under plant forming conditions to produce a plant; and,
c. inducing expression of the polynucleotide for a time sufficient to increase the harvestable yield of the plant.

A third embodiment would include an isolated polynucleotide selected from the group consisting of:
a. a polynucleotide having at least 80% sequence identity, as determined by the GAP algorithm under default parameters, to the full length sequence of a polynucleotide selected from the group consisting of SEQ ID NOS: 51, 89, 91, 97, 119, 127, 139, 147, 165 and 171; wherein the polynucleotide encodes a polypeptide that has PDR functions; and
b. a polynucleotide encoding a polypeptide selected from the group consisting of SEQ ID NO: 50, 90, 92, 98, 120, 128, 140, 148, 166, and 170; and
c. a polynucleotide selected from the group consisting of SEQ ID NOS: 51, 89, 91, 97, 119, 127, 139, 147, 165 and 171; and
d. a polynucleotide which is complementary to the polynucleotide of (a), (b), or (c).

### BRIEF DESCRIPTION OF THE FIGURES

Figure 1 - Diagram depicting improving crop plant architecture and yield by manipulation the PDR genes in transgenic plants.
Figure 2 - Photographic demonstration of the altered traits of genetically modified ZmPDR01 (PHP21051) transgenic plants. Transgenic plants (T) showed a distinct appearance difference from non-transgenic (NT) siblings. The transgenic plants showed a distinct canopy shape with upright wide leaves, tassels with high spikelet density and copious pollen shed, and elongated ears.
Figure 3 - Diagrammatic representation and photographic evidence of increased spikelet density on tassel branches of ZmPDR01 (PHP21051). Side by side comparison of the central spikes in control Gaspe (GASPE) and transgenic Gaspe (GASPE UBI::ZmPDR01) revealed that the distance between adjacent whorls of rachillas in transgenic Gaspe is almost half that of control plants, leading to a doubled number of spikelets. Gaspe UBI::ZmPDR1 tassel inflorescence meristems produced approximately 2 times more SPMs (spikelet pair meristems) per unit length than control GASPE plants.

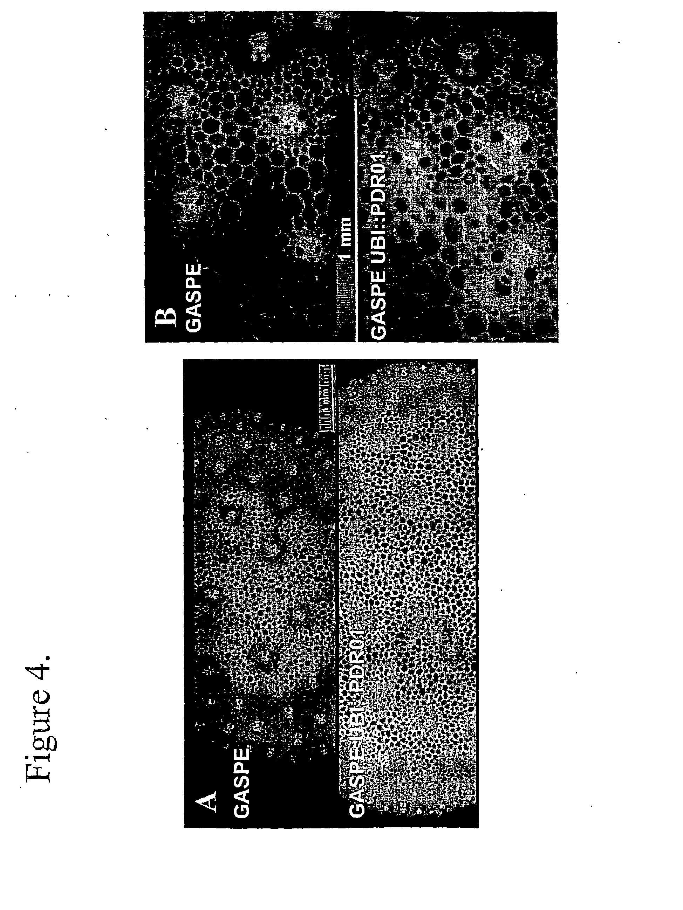

Figure 4 - Photographic evidence of increased vascular bundle size in a stalk of ZmPDR01 (PHP21051) transgenic plants. Side by side comparison of the stalk cross-sections at the 1^{st} internode in control Gaspe (GASPE) and transgenic Gaspe (GASPE UBI::ZmPDR01) revealed that numbers of vascular bundles, as well as their size are increased in transgenic plants.
Figure 5 - Structural superimposition of (a) and (d) CEN/ZmPDR01 and (b) and (e) ZmPDR01/ZmPDR14 and (c) ZmPDR01's ligand binding cavity. The three-dimensional structure of ZmPDR01 and ZmPDR14 proteins suggests their function as kinase effectors/regulators.
Figure 6 - Phylogenetic tree representing the Arabidopsis (At) PDR proteins. Mouse PEPB protein was used to outgroup. Three clades were delineated: the FT clade, the PDR1 clade, and the MFT clade.
Figure 7 - Phylogenetic tree for the Soybean (Gm) and Arabidopsis (At) PDR proteins. Seven soybean PDR genes were identified. The GmPDR genes are grouped into one of 3 Arabidopsis clades (FT, PDR and MFT).
Figure 8 - Phylogenetic tree for the Rice (Os) and Arabidopsis (At) PDR proteins. Twenty-two full-length proteins from Rice were identified. The phylogenetic tree includes 4 clades, three clades as described for dicots (FT, PDR1, MFT) and a fourth monocot clade (MC).
Figure 9 - Phylogenetic tree for the Maize (Zm) and Arabidopsis (At) PDR proteins. Eighteen full length proteins from Maize were identified. The phylogenetic tree includes 4 clades, FT, PDR1, MFT and MC.
Figure 10 - MPSS (Massively Parallel Signature Sequencing) profiling data for RNA tissue specific expression patterns/predicted function for maize PDR genes. Figure 10A is the TFL1clade, Figure 10B is the MFT clade, Figure 10C is the FT clade, and Figure 10D is the MC clade.
Figure 11 - In situ hybridization of ZmPDR01 to the shoot apical meristem. The hybridization revealed a strong signal of the ZmPDR01 antisense RNA in vascular bundles. Hybridization signal was found in the primordial provascular cells which surround mature vascular bundles with differentiated phloem and zylem. Figure 11A (transverse section) shows the ZmPDR01 signal concentrated around vascular bundles in the form of isolated islands. Figure 11B (longitudinal section) shows the ZmPDR01 hybridization signals concentrated in the form of elongated islands around vascular bundles.
Figure 12 - In situ hybridization of ZmPDR01 RNA to vascular bundles. Hybridization signal can be detected in vascular bundles with well-developed xylem vessels which are visualized by UV illumination. No obvious signal is seen in the phloem or companion cells. ZmPDR01 could be involved in the control of provascular and protoxylem cell identity.

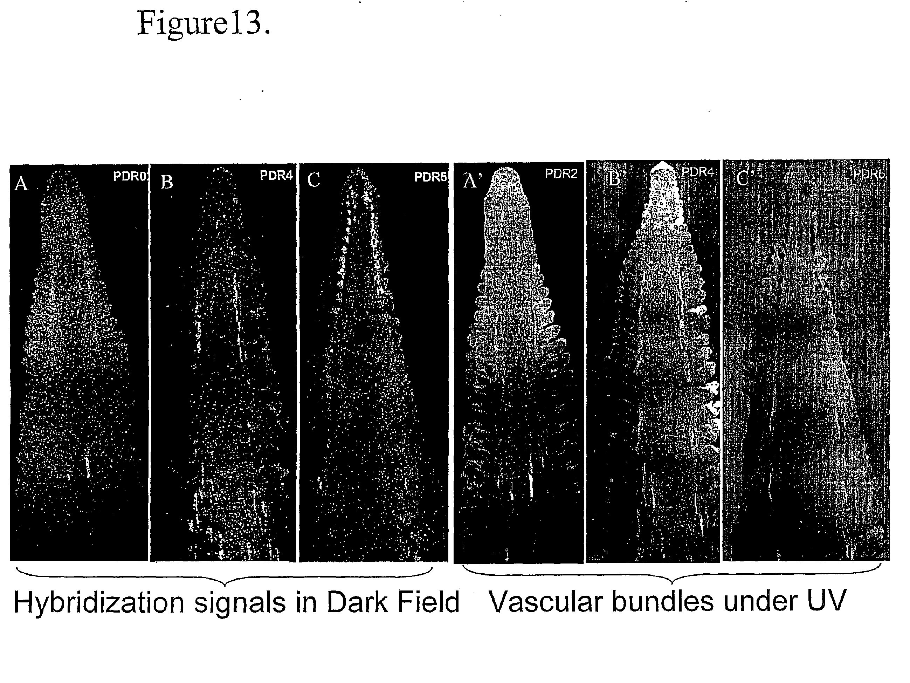

Figure 13 - Comparison of in situ hybridization of ZmPDR02, ZmPDR04 and ZmPDR05 to ear tips. The hybridization patterns of these three genes from the TFL1clade were analyzed under dark field to visualize hybridization signals and UV illumination to visualize vascular bundles 13A, B, A', B'. ZmPDR02 and ZmPDR04 are expressed in groups of cells underlying the first 8-9 consecutive spikelets from the top in each row. At the lower part of the ear hybridization signals are overlapped with lignified xylems. 13C,C' - ZmPDR05 is expressed in groups of cells underlying the earliest spikelet pair meristems as well as in the first 8-10 consecutive spikelets from the top of each row. At the lower part of the ear expression of the ZmPDR05 can be detected mostly in groups of cells tightly associated with vascular bundles (phloem).
Figure 14 - In situ hybridization of ZmPDR02 to the inner side of the vascular bundles and spikelet vasculature. Cells showing expression of ZmPDR02 include protoxylem (px), spikelet vascular bundles (svb), and gynocium (gy). Figure 14A is dark field, Figure 14B is UV illumination.
Figure 15 - In situ hybridization of ZmPDR05 to the outer side of the vascular bundles. PDR ZmPDR05 expressing cells are seen in vascular bundles in both 15A (dark field) and 15B (UV illumination) views.

### DETAILED DESCRIPTION OF THE INVENTION

Unless defined otherwise, all technical and scientific terms used herein have the same meaning as commonly understood by one of ordinary skill in the art to which this invention belongs. Unless mentioned otherwise, the techniques employed or contemplated herein are standard methodologies well known to one of ordinary skill in the art. The materials, methods and examples are illustrative only and not limiting. The following is presented by way of illustration and is not intended to limit the scope of the invention.

The present inventions now will be described more fully hereinafter with reference to the accompanying drawings, in which some, but not all embodiments of the invention are shown. Indeed, these inventions may be embodied in many different forms and should not be construed as limited to the embodiments set forth herein. Like numbers refer to like elements throughout.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation.

The practice of the present invention will employ, unless otherwise indicated, conventional techniques of botany, microbiology, tissue culture, molecular biology, chemistry, biochemistry and recombinant DNA technology, which are within the skill of the art. Such techniques are explained fully in the literature. *See*, *e.g*., Langenheim and Thimann, BOTANY: PLANT BIOLOGY AND ITS RELATION TO HUMAN AFFAIRS, John Wiley (1982); CELL CULTURE AND SOMATIC CELL GENETICS OF PLANTS, vol. 1, Vasil, ed. (1984); Stanier et al., THE MICROBIAL WORLD, 5th ed., Prentice-Hall (1986); Dhringra and Sinclair, BASIC PLANT PATHOLOGY METHODS, CRC Press (1985); Maniatis et al., MOLECULAR CLONING: A LABORATORY MANUAL (1982); DNA CLONING, vols. I and II, Glover, ed. (1985); OLIGONUCLEOTIDE SYNTHESIS, Gait, ed. (1984); NUCLEIC ACID HYBRIDIZATION, Hames and Higgins, eds. (1984); and the series METHODS IN ENZYMOLOGY, Colowick and Kaplan, eds, Academic Press, Inc., San Diego, CA.

Units, prefixes, and symbols may be denoted in their SI accepted form. Unless otherwise indicated, nucleic acids are written left to right in 5' to 3' orientation; amino acid sequences are written left to right in amino to carboxy orientation, respectively. Numeric ranges are inclusive of the numbers defining the range. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes. The terms defined below are more fully defined by reference to the specification as a whole.

In describing the present invention, the following terms will be employed, and are intended to be defined as indicated below.

By "microbe" is meant any microorganism (including both eukaryotic and prokaryotic microorganisms), such as fungi, yeast, bacteria, actinomycetes, algae and protozoa, as well as other unicellular structures.

By "amplified" is meant the construction of multiple copies of a nucleic acid sequence or multiple copies complementary to the nucleic acid sequence using at least one of the nucleic acid sequences as a template. Amplification systems include the polymerase chain reaction (PCR) system, ligase chain reaction (LCR) system, nucleic acid sequence based amplification (NASBA, Cangene, Mississauga, Ontario), Q-*Beta* Replicase systems, transcription-based amplification system (TAS), and strand displacement amplification (SDA). *See, e.g*., DIAGNOSTIC MOLECULAR MICROBIOLOGY: PRINCIPLES AND APPLICATIONS, Persing et al., eds., American Society for Microbiology, Washington, DC (1993). The product of amplification is termed an amplicon.

The term "conservatively modified variants" applies to both amino acid and nucleic acid sequences. With respect to particular nucleic acid sequences, conservatively modified variants refer to those nucleic acids that encode identical or conservatively modified variants of the amino acid sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" and represent one species of conservatively modified variation. Every nucleic acid sequence herein that encodes a polypeptide also describes every possible silent variation of the nucleic acid. One of ordinary skill will recognize that each codon in a nucleic acid (except AUG, which is ordinarily the only codon for methionine; one exception is *Micrococcus rubens*, for which GTG is the methionine codon (Ishizuka et al., J. Gen. Microbiol. 139:425-32 (1993)) can be modified to yield a functionally identical molecule. Accordingly, each silent variation of a nucleic acid, which encodes a polypeptide of the present invention, is implicit in each described polypeptide sequence and incorporated herein by reference.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" when the alteration results in the substitution of an amino acid with a chemically similar amino acid. Thus, any number of amino acid residues selected from the group of integers consisting of from 1 to 15 can be so altered. Thus, for example, 1, 2, 3, 4, 5, 7, or 10 alterations can be made. Conservatively modified variants typically provide similar biological activity as the unmodified polypeptide sequence from which they are derived. For example, substrate specificity, enzyme activity, or ligand/receptor binding is generally at least 30%, 40%, 50%, 60%, 70%, 80%, or 90%, preferably 60-90% of the native protein for it's native substrate. Conservative substitution tables providing functionally similar amino acids are well known in the art.

The following six groups each contain amino acids that are conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).
See also, Creighton, PROTEINS, W.H. Freeman and Co. (1984).

As used herein, "consisting essentially of" means the inclusion of additional sequences to an object polynucleotide where the additional sequences may not selectively hybridize, under stringent hybridization conditions, to the same cDNA as the polynucleotide and where the hybridization conditions include a wash step in 0.1X SSC and 0.1% sodium dodecyl sulfate at 65°C.

By "encoding" or "encoded," with respect to a specified nucleic acid, is meant comprising the information for translation into the specified protein. A nucleic acid encoding a protein may comprise non-translated sequences (*e.g*., introns) within translated regions of the nucleic acid, or may lack such intervening non-translated sequences (*e.g*., as in cDNA). The information by which a protein is encoded is specified by the use of codons. Typically, the amino acid sequence is encoded by the nucleic acid using the "universal" genetic code. However, variants of the universal code, such as is present in some plant, animal, and fungal mitochondria, the bacterium *Mycoplasma capricolum* (Yamao et al., Proc. Natl. Acad. Sci. USA 82:2306-9 (1985)), or the ciliate *Macronucleus*, may be used when the nucleic acid is expressed using these organisms.

When the nucleic acid is prepared or altered synthetically, advantage can be taken of known codon preferences of the intended host where the nucleic acid is to be expressed. For example, although nucleic acid sequences of the present invention may be expressed in both monocotyledonous and dicotyledonous plant species, sequences can be modified to account for the specific codon preferences and GC content preferences of monocotyledonous plants or dicotyledonous plants as these preferences have been shown to differ (Murray et al., Nucleic Acids Res. 17:477-98 (1989) and herein incorporated by reference). Thus, the maize preferred codon for a particular amino acid might be derived from known gene sequences from maize. Maize codon usage for 28 genes from maize plants is listed in Table 5 of Murray *et al*., *supra.*

As used herein, "heterologous" in reference to a nucleic acid is a nucleic acid that originates from a foreign species, or, if from the same species, is substantially modified from its native form in composition and/or genomic locus by deliberate human intervention. For example, a promoter operably linked to a heterologous structural gene is from a species different from that from which the structural gene was derived or, if from the same species, one or both are substantially modified from their original form. A heterologous protein may originate from a foreign species or, if from the same species, is substantially modified from its original form by deliberate human intervention.

By "host cell" is meant a cell, which contains a vector and supports the replication and/or expression of the expression vector. Host cells may be prokaryotic cells such as E. *coli*, or eukaryotic cells such as yeast, insect, plant, amphibian, or mammalian cells. Preferably, host cells are monocotyledonous or dicotyledonous plant cells, including but not limited to maize, sorghum, sunflower, soybean, wheat, alfalfa, rice, cotton, canola, barley, millet, and tomato. A particularly preferred monocotyledonous host cell is a maize host cell.

The term "hybridization complex" includes reference to a duplex nucleic acid structure formed by two single-stranded nucleic acid sequences selectively hybridized with each other.

The term "introduced" in the context of inserting a nucleic acid into a cell, means "transfection" or "transformation" or "transduction" and includes reference to the incorporation of a nucleic acid into a eukaryotic or prokaryotic cell where the nucleic acid may be incorporated into the genome of the cell (*e.g*., chromosome, plasmid, plastid or mitochondrial DNA), converted into an autonomous replicon, or transiently expressed (*e.g*., transfected mRNA).

The terms "isolated" refers to material, such as a nucleic acid or a protein, which is substantially or essentially free from components which normally accompany or interact with it as found in its naturally occurring environment. The isolated material optionally comprises material not found with the material in its natural environment. Nucleic acids, which are "isolated", as defined herein, are also referred to as "heterologous" nucleic acids. Unless otherwise stated, the term "PDR nucleic acid" means a nucleic acid comprising a polynucleotide ("PDR polynucleotide") encoding a PDR polypeptide.

As used herein, "nucleic acid" includes reference to a deoxyribonucleotide or ribonucleotide polymer in either single- or double-stranded form, and unless otherwise limited, encompasses known analogues having the essential nature of natural nucleotide in that they hybridize to single-stranded nucleic acids in a manner similar to naturally occurring nucleotides (*e.g*., peptide nucleic acids).

By "nucleic acid library" is meant a collection of isolated DNA or RNA molecules, which comprise and substantially represent the entire transcribed fraction of a genome of a specified organism. Construction of exemplary nucleic acid libraries, such as genomic and cDNA libraries, is taught in standard molecular biology references such as Berger and Kimmel, GUIDE TO MOLECULAR CLONING TECHNIQUES, from the series METHODS IN ENZYMOLOGY, vol. 152, Academic Press, Inc., San Diego, CA (1987); Sambrook et al., MOLECULAR CLONING: A LABORATORY MANUAL, 2nd ed., vols. 1-3 (1989); and CURRENT PROTOCOLS IN MOLECULAR BIOLOGY, Ausubel et al., eds, Current Protocols, a joint venture between Greene Publishing Associates, Inc. and John Wiley & Sons, Inc. (1994 Supplement).

As used herein "operably linked" includes reference to a functional linkage between a first sequence, such as a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame.

As used herein, the term "plant" includes reference to whole plants, plant organs (*e.g*., leaves, stems, roots, etc.), seeds and plant cells and progeny of same. Plant cell, as used herein includes, without limitation, seeds suspension cultures, embryos, meristematic regions, callus tissue, leaves, roots, shoots, gametophytes, sporophytes, pollen, and microspores. The class of plants, which can be used in the methods of the invention, is generally as broad as the class of higher plants amenable to transformation techniques, including both monocotyledonous and dicotyledonous plants including species from the genera: *Cucurbita, Rosa, Vitis, Juglans, Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Ciahorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Glycine, Pisum, Phaseolus, Lolium, Oryza, Avena, Hordeum, Secale, Allium,* and *Triticum.* A particularly preferred plant is Zea *mays.*

As used herein, "yield" includes reference to bushels per acre of a grain crop at harvest, as adjusted for grain moisture (15% typically). Grain moisture is measured in the grain at harvest. The adjusted test weight of grain is determined to be the weight in pounds per bushel, adjusted for grain moisture level at harvest.

As used herein, "polynucleotide" includes reference to a deoxyribopolynucleotide, ribopolynucleotide, or analogs thereof that have the essential nature of a natural ribonucleotide in that they hybridize, under stringent hybridization conditions, to substantially the same nucleotide sequence as naturally occurring nucleotides and/or allow translation into the same amino acid(s) as the naturally occurring nucleotide(s). A polynucleotide can be full-length or a subsequence of a native or heterologous structural or regulatory gene. Unless otherwise indicated, the term includes reference to the specified sequence as well as the complementary sequence thereof. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The term polynucleotide as it is employed herein embraces such chemically, enzymatically or metabolically modified forms of polynucleotides, as well as the chemical forms of DNA and RNA characteristic of viruses and cells, including *inter alia,* simple and complex cells.

The terms "polypeptide," "peptide," and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers.

As used herein "promoter" includes reference to a region of DNA upstream from the start of transcription and involved in recognition and binding of RNA polymerase and other proteins to initiate transcription. A "plant promoter" is a promoter capable of initiating transcription in plant cells. Exemplary plant promoters include, but are not limited to, those that are obtained from plants, plant viruses, and bacteria which comprise genes expressed in plant cells such *Agrobacterium* or *Rhizobium.* Examples are promoters that preferentially initiate transcription in certain tissues, such as leaves, roots, seeds, fibres, xylem vessels, tracheids, or sclerenchyma. Such promoters are referred to as "tissue preferred." A "cell type" specific promoter primarily drives expression in certain cell types in one or more organs, for example, vascular cells in roots or leaves. An "inducible" or "regulatable" promoter is a promoter, which is under environmental control. Examples of environmental conditions that may effect transcription by inducible promoters include anaerobic conditions or the presence of light. Another type of promoter is a developmentally regulated promoter, for example, a promoter that drives expression during pollen development. Tissue preferred, cell type specific, developmentally regulated, and inducible promoters constitute the class of "non-constitutive" promoters. A "constitutive" promoter is a promoter, which is active under most environmental conditions.

The term "PDR polypeptide" refers to one or more amino acid sequences. The term is also inclusive of fragments, variants, homologs, alleles or precursors (*e.g*., preproproteins or proproteins) thereof. A "PDR protein" comprises a PDR polypeptide. Unless otherwise stated, the term "PDR nucleic acid" means a nucleic acid comprising a polynucleotide ("PDR polynucleotide") encoding a PDR polypeptide.

As used herein "recombinant" includes reference to a cell or vector, that has been modified by the introduction of a heterologous nucleic acid or that the cell is derived from a cell so modified. Thus, for example, recombinant cells express genes that are not found in identical form within the native (non-recombinant) form of the cell or express native genes that are otherwise abnormally expressed, under expressed or not expressed at all as a result of deliberate human intervention. The term "recombinant" as used herein does not encompass the alteration of the cell or vector by naturally occurring events (*e.g*., spontaneous mutation, natural transformation/transduction/transposition) such as those occurring without deliberate human intervention.

As used herein, a "recombinant expression cassette" is a nucleic acid construct, generated recombinantly or synthetically, with a series of specified nucleic acid elements, which permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid to be transcribed, and a promoter.

The terms "residue" or "amino acid residue" or "amino acid" are used interchangeably herein to refer to an amino acid that is incorporated into a protein, polypeptide, or peptide (collectively "protein"). The amino acid may be a naturally occurring amino acid and, unless otherwise limited, may encompass known analogs of natural amino acids that can function in a similar manner as naturally occurring amino acids.

The term "selectively hybridizes" includes reference to hybridization, under stringent hybridization conditions, of a nucleic acid sequence to a specified nucleic acid target sequence to a detectably greater degree (*e.g*., at least 2-fold over background) than its hybridization to non-target nucleic acid sequences and to the substantial exclusion of non-target nucleic acids. Selectively hybridizing sequences typically have about at least 40% sequence identity, preferably 60-90% sequence identity, and most preferably 100% sequence identity (i.e., complementary) with each other.

The terms "stringent conditions" or "stringent hybridization conditions" include reference to conditions under which a probe will hybridize to its target sequence, to a detectably greater degree than other sequences (*e.g*., at least 2-fold over background). Stringent conditions are sequence-dependent and will be different in different circumstances. By controlling the stringency of the hybridization and/or washing conditions, target sequences can be identified which can be up to 100% complementary to the probe (homologous probing). Alternatively, stringency conditions can be adjusted to allow some mismatching in sequences so that lower degrees of similarity are detected (heterologous probing). Optimally, the probe is approximately 500 nucleotides in length, but can vary greatly in length from less than 500 nucleotides to at least equal to the entire length of the target sequence.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (*e.g*., 10 to 50 nucleotides) and at least about 60°C for long probes (*e.g*., greater than 50 nucleotides). Stringent conditions may also be achieved with the addition of destabilizing agents such as formamide or Denhardfs. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C. Specificity is typically the function of post-hybridization washes, the critical factors being the ionic strength and temperature of the final wash solution. For DNA-DNA hybrids, the Tₘ can be approximated from the equation of Meinkoth and Wahl, Anal. Biochem., 138:267-84 (1984): Tₘ = 81.5°C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1 °C for each 1% of mismatching; thus, Tₘ, hybridization and/or wash conditions can be adjusted to hybridize to sequences of the desired identity. For example, if sequences with ≥90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution) it is preferred to increase the SSC concentration so that a higher temperature can be used. An extensive guide to the hybridization of nucleic acids is found in Tijssen, LABORATORY TECHNIQUES IN BIOCHEMISTRY AND MOLECULAR BIOLOGY-HYBRIDIZATION WITH NUCLEIC ACID PROBES, part I, chapter 2, "Overview of principles of hybridization and the strategy of nucleic acid probe assays," Elsevier, New York (1993); and CURRENT PROTOCOLS IN MOLECULAR BIOLOGY, chapter 2, Ausubel et al., eds, Greene Publishing and Wiley-Interscience, New York (1995). Unless otherwise stated, in the present application, high stringency is defined as hybridization in 4X SSC, 5X Denhardt's (5 g Ficoll, 5 g polyvinypyrrolidone, 5 g bovine serum albumin in 500ml of water), 0.1 mg/ml boiled salmon sperm DNA, and 25 mM Na phosphate at 65°C, and a wash in 0.1X SSC, 0.1 % SDS at 65°C.

As used herein, "transgenic plant" includes reference to a plant, which comprises within its genome a heterologous polynucleotide. Generally, the heterologous polynucleotide is stably integrated within the genome such that the polynucleotide is passed on to successive generations. The heterologous polynucleotide may be integrated into the genome alone or as part of a recombinant expression cassette. "Transgenic" is used herein to include any cell, cell line, callus, tissue, plant part or plant, the genotype of which has been altered by the presence of heterologous nucleic acid including those transgenics initially so altered as well as those created by sexual crosses or asexual propagation from the initial transgenic. The term "transgenic" as used herein does not encompass the alteration of the genome (chromosomal or extra-chromosomal) by conventional plant breeding methods or by naturally occurring events such as random cross-fertilization, non-recombinant viral infection, non-recombinant bacterial transformation, non-recombinant transposition, or spontaneous mutation.

As used herein, "vector" includes reference to a nucleic acid used in transfection of a host cell and into which can be inserted a polynucleotide. Vectors are often replicons. Expression vectors permit transcription of a nucleic acid inserted therein.

The following terms are used to describe the sequence relationships between two or more nucleic acids or polynucleotides or polypeptides: (a) "reference sequence," (b) "comparison window," (c) "sequence identity," (d) "percentage of sequence identity," and (e) "substantial identity."

As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.

As used herein, "comparison window" means reference to a contiguous and specified segment of a polynucleotide sequence, wherein the polynucleotide sequence may be compared to a reference sequence and wherein the portion of the polynucleotide sequence in the comparison window may comprise additions or deletions (i.e., gaps) compared to the reference sequence (which does not comprise additions or deletions) for optimal alignment of the two sequences. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches.

Methods of alignment of nucleotide and amino acid sequences for comparison are well known in the art. The local homology algorithm (BESTFIT) of Smith and Waterman, Adv. Appl. Math 2:482 (1981), may conduct optimal alignment of sequences for comparison; by the homology alignment algorithm (GAP) of Needleman and Wunsch, J. Mol. Biol. 48:443-53 (1970); by the search for similarity method (Tfasta and Fasta) of Pearson and Lipman, Proc. Natl. Acad. Sci. USA 85:2444 (1988); by computerized implementations of these algorithms, including, but not limited to: CLUSTAL in the PC/Gene program by Intelligenetics, Mountain View, California, GAP, BESTFIT, BLAST, FASTA, and TFASTA in the Wisconsin Genetics Software Package, Version 8 (available from Genetics Computer Group (GCG® programs (Accelrys, Inc., San Diego, CA).). The CLUSTAL program is well described by Higgins and Sharp, Gene 73:237-44 (1988); Higgins and Sharp, CABIOS 5:151-3 (1989); Corpet et al., Nucleic Acids Res. 16:10881-90 (1988); Huang et al., Computer Applications in the Biosciences 8:155-65 (1992), and Pearson et al., Meth. Mol. Biol. 24:307-31 (1994). The preferred program to use for optimal global alignment of multiple sequences is PileUp (Feng and Doolittle, J. Mol. Evol., 25:351-60 (1987) which is similar to the method described by Higgins and Sharp, CABIOS 5:151-53 (1989) and hereby incorporated by reference). The BLAST family of programs which can be used for database similarity searches includes: BLASTN for nucleotide query sequences against nucleotide database sequences; BLASTX for nucleotide query sequences against protein database sequences; BLASTP for protein query sequences against protein database sequences; TBLASTN for protein query sequences against nucleotide database sequences; and TBLASTX for nucleotide query sequences against nucleotide database sequences. See CURRENT PROTOCOLS IN MOLECULAR BIOLOGY, Chapter 19, Ausubel et al., eds., Greene Publishing and Wiley-Interscience, New York (1995).

GAP uses the algorithm of Needleman and Wunsch, *supra,* to find the alignment of two complete sequences that maximizes the number of matches and minimizes the number of gaps. GAP considers all possible alignments and gap positions and creates the alignment with the largest number of matched bases and the fewest gaps. It allows for the provision of a gap creation penalty and a gap extension penalty in units of matched bases. GAP must make a profit of gap creation penalty number of matches for each gap it inserts. If a gap extension penalty greater than zero is chosen, GAP must, in addition, make a profit for each gap inserted of the length of the gap times the gap extension penalty. Default gap creation penalty values and gap extension penalty values in Version 10 of the Wisconsin Genetics Software Package are 8 and 2, respectively. The gap creation and gap extension penalties can be expressed as an integer selected from the group of integers consisting of from 0 to 100. Thus, for example, the gap creation and gap extension penalties can be 0, 1, 2, 3, 4, 5, 6, 7, 8, 9, 10, 15, 20, 30, 40, 50, or greater.

GAP presents one member of the family of best alignments. There may be many members of this family, but no other member has a better quality. GAP displays four figures of merit for alignments: Quality, Ratio, Identity, and Similarity. The Quality is the metric maximized in order to align the sequences. Ratio is the quality divided by the number of bases in the shorter segment. Percent Identity is the percent of the symbols that actually match. Percent Similarity is the percent of the symbols that are similar. Symbols that are across from gaps are ignored. A similarity is scored when the scoring matrix value for a pair of symbols is greater than or equal to 0.50, the similarity threshold. The scoring matrix used in Version 10 of the Wisconsin Genetics Software Package is BLOSUM62 (see Henikoff and Henikoff, Proc. Natl. Acad. Sci. USA 89:10915 (1989)).

Unless otherwise stated, sequence identity/similarity values provided herein refer to the value obtained using the BLAST 2.0 suite of programs using default parameters (Altschul et al., Nucleic Acids Res. 25:3389-402 (1997)).

As those of ordinary skill in the art will understand, BLAST searches assume that proteins can be modeled as random sequences. However, many real proteins comprise regions of nonrandom sequences, which may be homopolymeric tracts, short-period repeats, or regions enriched in one or more amino acids. Such low-complexity regions may be aligned between unrelated proteins even though other regions of the protein are entirely dissimilar. A number of low-complexity filter programs can be employed to reduce such low-complexity alignments. For example, the SEG (Wooten and Federhen, Comput. Chem. 17:149-63 (1993)) and XNU (Claverie and States, Comput. Chem. 17:191-201 (1993)) low-complexity filters can be employed alone or in combination.

As used herein, "sequence identity" or "identity" in the context of two nucleic acid or polypeptide sequences includes reference to the residues in the two sequences, which are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (*e.g*. charge or hydrophobicity) and therefore do not change the functional properties of the molecule. Where sequences differ in conservative substitutions, the percent sequence identity may be adjusted upwards to correct for the conservative nature of the substitution. Sequences, which differ by such conservative substitutions, are said to have "sequence similarity" or "similarity." Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, *e.g*., according to the algorithm of Meyers and Miller, Computer Applic. Biol. Sci. 4:11-17 (1988), *e.g*., as implemented in the program PC/GENE (Intelligenetics, Mountain View, California, USA).

As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The term "substantial identity" of polynucleotide sequences means that a polynucleotide comprises a sequence that has between 50-100% sequence identity, preferably at least 50% sequence identity, preferably at least 60% sequence identity, preferably at least 70%, more preferably at least 80%, more preferably at least 90%, and most preferably at least 95%, compared to a reference sequence using one of the alignment programs described using standard parameters. One of skill will recognize that these values can be appropriately adjusted to determine corresponding identity of proteins encoded by two nucleotide sequences by taking into account codon degeneracy, amino acid similarity, reading frame positioning and the like. Substantial identity of amino acid sequences for these purposes normally means sequence identity of between 55-100%, preferably at least 55%, preferably at least 60%, more preferably at least 70%, 80%, 90%, and most preferably at least 95%.

Another indication that nucleotide sequences are substantially identical is if two molecules hybridize to each other under stringent conditions. The degeneracy of the genetic code allows for many amino acids substitutions that lead to variety in the nucleotide sequence that code for the same amino acid, hence it is possible that the DNA sequence could code for the same polypeptide but not hybridize to each other under stringent conditions. This may occur, *e.g.,* when a copy of a nucleic acid is created using the maximum codon degeneracy permitted by the genetic code. One indication that two nucleic acid sequences are substantial identical is that the polypeptide, which the first nucleic acid encodes, is immunologically cross reactive with the polypeptide encoded by the second nucleic acid.

The terms "substantial identity" in the context of a peptide indicates that a peptide comprises a sequence with between 55-100% sequence identity to a reference sequence preferably at least 55% sequence identity, preferably 60% preferably 70%, more preferably 80%, most preferably at least 90% or 95% sequence identity to the reference sequence over a specified comparison window. Preferably, optimal alignment is conducted using the homology alignment algorithm of Needleman and Wunsch, *supra.* An indication that two peptide sequences are substantially identical is that one peptide is immunologically reactive with antibodies raised against the second peptide. Thus, a peptide is substantially identical to a second peptide, for example, where the two peptides differ only by a conservative substitution. In addition, a peptide can be substantially identical to a second peptide when they differ by a non-conservative change if the epitope that the antibody recognizes is substantially identical. Peptides, which are "substantially similar" share sequences as, noted above except that residue positions, which are not identical, may differ by conservative amino acid changes.

The invention discloses PDR polynucleotides and polypeptides. The novel nucleotides and proteins of the invention have an expression pattern which indicates that they regulate cell development and thus play an important role in plant development. The polynucleotides are expressed in various plant tissues. The polynucleotides and polypeptides thus provide an opportunity to manipulate plant development to alter seed and vegetative tissue development, timing or composition. This may be used to create a sterile plant, a seedless plant or a plant with altered endosperm composition.

**TABLE 1. Sequence Identification and nomenclature**

| **Sequence ID numbers (Polynucleotide polypeptide)** | **Current Name** | **Other nomenclature** |
|---|---|---|
| 1,2 | ZmPDR01 | ZmTFL1 |
| 3,4 | ZmPDR02 | ZmTFL2 |
| 5,6 | ZmPDR03 | ZmTFL3 |
| 7,8 | ZmPDR04 | ZmTFL4 |
| 9,10 | ZmPDR05 | ZmTFL5 |
| 11,12 | ZmPDR06 | ZmTFL_C10 |
| 13,14 | ZmPDR07 | ZmTFL_C04 |
| 15,16 | ZmPDR08 | ZmTFL_C14 |
| 17,18 | ZmPDR09 | ZmFT4 |
| 19,20 | ZmPDR10 | ZmFT5 |
| 21,22 | ZmPDR11 | ZmFT6 |
| 23,24 | ZmPDR12 | ZmFT2 |
| 25,26 | ZmPDR13 | ZmTFL_C05 |
| 27,28 | ZmPDR14 | ZmFT1 |
| 29,30 | ZmPDR15 | ZmFT3 |
| 31,32 | ZmPDR16 | ZmFT7 |
| 33,34 | ZmPDR17 | ZmTFL_C01 |
| 35,36 | ZmPDR18 | ZmTFL_C02 |
| 37,38 | ZmPDR19 | ZmTFL_C03 |
| 39,40 | ZmPDR20 | ZmTFL_C06 |
| 41,42 | ZmPDR21 | ZmTFL_C07 |
| 43,44 | ZmPDR22 | ZmTFL_C08 |
| 45,46 | ZmPDR23 | ZmTFL_C09 |
| 47,48 | ZmPDR24 | ZmTFL_C11 |
| 49,50 | ZmPDR25 | ZmTFL_C12 |
| 51,52 | ZmPDR26 | ZmTFL_C13 |
| 53,54 | ZmPDR27 | ZmTFL_C15 |
| 55,56 | ZmPDR28 | ZmTFL_C19 |
| 57,58 | OsPDR01 | OsTFL01 |
| 59,60 | OsPDR02 | OsTFL02 |
| 61,62 | OsPDR03 | OsTFL03 |
| 63,64 | OsPDR04 | OsTFL04 |
| 65,66 | OsPDR05 | OsTFL05 |
| 67,68 | OsPDR06 | OsTFL06 |
| 69,70 | OsPDR07 | DsTFL07 |
| 71,72 | OsPDR08 | OsTFL08 |
| 73,74 | OsPDR09 | OsTFL09 |
| 75,76 | OSPDR10 | OsTFL10 |
| 77,78 | OsPDR11 | OsTFL11 |
| 79,80 | OsPDR12 | OsTFL12 |
| 81,82 | OsPDR13 | OsTFL13 |
| 83,84 | OsPDR14 | OsTFL14 |
| 85,86 | OsPDR15 | OsTFL15 |
| 87,88 | OsPDR16 | OsTFL16 |
| 89,90 | OsPDR17 | OsTFL17 |
| 91,92 | OsPDR18 | OsTFL18 |
| 93,94 | OsPDR19 | OsTFL19 |
| 95,96 | OsPDR20 | OsTFL20 |
| 97,98 | OsPDR21 | OsTFL22 |
| 99,100 | SbPDR01 | SbTFL_01 |
| 101,102 | SbPDR02 | SbTFL_02 |
| 103,104 | SbPDR03 | SbTFL_03 |
| 105,106 | SbPDR04 | SbTFL 04 |
| 107,108 | SbPDR05 | SbTFL_05 |
| 109,110 | SbPDR06 | SbTFL_ 06 |
| 111,112 | SbPDR07 | SbTFL_07 |
| 113,114 | SbPDR08 | SbTFL_08 |
| 115,116 | SbPDR09 | SbTFL_09 |
| 117,118 | SBPDR10 | SbTFL_10 |
| 119,120 | SbFDRH11 | SbTFL_11 |
| 121,122 | SbPDR12 | SbTFL_12 |
| 123,124 | SbpDR13 | SbTFL_13 |
| 125,126 | SbPDR14 | SbTFL_14 |
| 127,128 | SbPDR15 | SbTFL_15 |
| 129,130 | SbPDR16 | SbTFL_16 |
| 131,132 | SbPDR17 | SbTFL_17 |
| 133,134 | SbPDR18 | SbTFL_18 |
| 135,136 | SbPDR19 | SbTFL_19 |
| 137,138 | SbPDR20 | SbTFL_20 |
| 139,140 | SbPDR21 | SbTFL_21 |
| 141,142 | SbPDR22 | SbTFL_22 |
| 143,144 | SbPDR23 | SbTFL_23 |
| 145,146 | SbPDR24 | SbTFL_24 |
| 147,148 | AcPDR01 | AcTFL_01 |
| 149,150 | TaPDR01 | TaTFL_01 |
| 151,152 | TaPDR02 | TaTFL_02 |
| 163,154 | GmPDR01 | Gm_TFL_01 |
| 155,156 | GmPDR02 | Gm_TFL_02 |
| 157,158 | GmPDR03 | Gm_TFL_03 |
| 159,160 | GmPDR04 | Gm_TFL_04 |
| 161,162 | GmPDR05 | Gm_TFL_05 |
| 163,164 | GmPDR06 | Gm_TFL_06 |
| 165,166 | GmPDR07 | Gm_TFL_07 |
| 167,168 | HaPDR01 | HaTFL_01 |
| 168,170 | HaPDR02 | HaTFL_02 |
| 171,172 | HaPDR03 | HaTFL_03 |
| 173,174 | ATPDR01 | At_TFL1 |
| 175,176 | AtPDR02 | At_CEN |
| 177,178 | AtPDR03 | At_BFT |
| 179,180 | AtPDR04 | At_FT |
| 181,182 | AtPDR05 | At_TSF |
| 183,184 | AtPDR06 | At_MFT |

### Nucleic Acids

The present invention provides, *inter alia,* isolated nucleic acids of RNA, DNA, and analogs and/or chimeras thereof, comprising a PDR polynucleotide.

The present invention also includes polynucleotides optimized for expression in different organisms. For example, for expression of the polynucleotide in a maize plant, the sequence can be altered to account for specific codon preferences and to alter GC content as according to Murray *et al*, *supra.* Maize codon usage for 28 genes from maize plants is listed in Table 5 of Murray *et al., supra.*

The PDR nucleic acids of the present invention comprise isolated PDR polynucleotides which are inclusive of:
(a) a polynucleotide encoding a PDR polypeptide and conservatively modified and polymorphic variants thereof;
(b) a polynucleotide having at least 70% sequence identity with polynucleotides of (a) or (b);
(c) complementary sequences of polynucleotides of (a) or (b).

### Construction of Nucleic Acids

The isolated nucleic acids of the present invention can be made using (a) standard recombinant methods, (b) synthetic techniques, or combinations thereof. In some embodiments, the polynucleotides of the present invention will be cloned, amplified, or otherwise constructed from a fungus or bacteria.

The nucleic acids may conveniently comprise sequences in addition to a polynucleotide of the present invention. For example, a multi-cloning site comprising one or more endonuclease restriction sites may be inserted into the nucleic acid to aid in isolation of the polynucleotide. Also, translatable sequences may be inserted to aid in the isolation of the translated polynucleotide of the present invention. For example, a hexa-histidine marker sequence provides a convenient means to purify the proteins of the present invention. The nucleic acid of the present invention - excluding the polynucleotide sequence - is optionally a vector, adapter, or linker for cloning and/or expression of a polynucleotide of the present invention. Additional sequences may be added to such cloning and/or expression sequences to optimize their function in cloning and/or expression, to aid in isolation of the polynucleotide, or to improve the introduction of the polynucleotide into a cell. Typically, the length of a nucleic acid of the present invention less the length of its polynucleotide of the present invention is less than 20 kilobase pairs, often less than 15 kb, and frequently less than 10 kb. Use of cloning vectors, expression vectors, adapters, and linkers is well known in the art. Exemplary nucleic acids include such vectors as: M13, lambda ZAP Express, lambda ZAP II, lambda gt10, lambda gt11, pBK-CMV, pBK-RSV, pBluescript II, lambda DASH II, lambda EMBL 3, lambda EMBL 4, pWE15, SuperCos 1, SurfZap, Uni-ZAP, pBC, pBS+/-, pSG5, pBK, pCR-Script, pET, pSPUTK, p3'SS, pGEM, pSK+/-, pGEX, pSPORTI and II, pOPRSVI CAT, pOPI3 CAT, pXT1, pSG5, pPbac, pMbac, pMC1neo, pOG44, pOG45, pFRTβGAL, pNEOβGAL, pRS403, pRS404, pRS405, pRS406, pRS413, pRS414, pRS415, pRS416, lambda MOSSlox, and lambda MOSElox. Optional vectors for the present invention include but are not limited to, lambda ZAP II, and pGEX. For a description of various nucleic acids see, *e.g*., Stratagene Cloning Systems, Catalogs 1995, 1996, 1997 (La Jolla, CA); and, Amersham Life Sciences, Inc, Catalog '97 (Arlington Heights, IL).

### Synthetic Methods for Constructing Nucleic Acids

The isolated nucleic acids of the present invention can also be prepared by direct chemical synthesis by methods such as the phosphotriester method of Narang et al., Meth. Enzymol. 68:90-9 (1979); the phosphodiester method of Brown et al., Meth. Enzymol. 68:109-51 (1979); the diethylphosphoramidite method of Beaucage et al., Tetra. Letts. 22(20):1859-62 (1981); the solid phase phosphoramidite triester method described by Beaucage *et al*., *supra, e.g.,* using an automated synthesizer, *e.g.,* as described in Needham-VanDevanter et al., Nucleic Acids Res. 12:6159-68 (1984); and, the solid support method of United States Patent No. 4,458,066. Chemical synthesis generally produces a single stranded oligonucleotide. This may be converted into double stranded DNA by hybridization with a complementary sequence or by polymerization with a DNA polymerase using the single strand as a template. One of skill will recognize that while chemical synthesis of DNA is limited to sequences of about 100 bases, longer sequences may be obtained by the ligation of shorter sequences.

### UTRs and Codon Preference

In general, translational efficiency has been found to be regulated by specific sequence elements in the 5' non-coding or untranslated region (5' UTR) of the RNA. Positive sequence motifs include translational initiation consensus sequences (Kozak, Nucleic Acids Res.15:8125 (1987)) and the 5<G> 7 methyl GpppG RNA cap structure (Drummond et al., Nucleic Acids Res. 13:7375 (1985)). Negative elements include stable intramolecular 5' UTR stem-loop structures (Muesing et al., Cell 48:691 (1987)) and AUG sequences or short open reading frames preceded by an appropriate AUG in the 5' UTR (Kozak, *supra,* Rao et al., Mol. and Cell. Biol. 8:284 (1988)). Accordingly, the present invention provides 5' and/or 3' UTR regions for modulation of translation of heterologous coding sequences.

Further, the polypeptide-encoding segments of the polynucleotides of the present invention can be modified to alter codon usage. Altered codon usage can be employed to alter translational efficiency and/or to optimize the coding sequence for expression in a desired host or to optimize the codon usage in a heterologous sequence for expression in maize. Codon usage in the coding regions of the polynucleotides of the present invention can be analyzed statistically using commercially available software packages such as "Codon Preference" available from the University of Wisconsin Genetics Computer Group. See Devereaux et al., Nucleic Acids Res. 12:387-395 (1984)); or MacVector 4.1 (Eastman Kodak Co., New Haven, Conn.). Thus, the present invention provides a codon usage frequency characteristic of the coding region of at least one of the polynucleotides of the present invention. The number of polynucleotides (3 nucleotides per amino acid) that can be used to determine a codon usage frequency can be any integer from 3 to the number of polynucleotides of the present invention as provided herein. Optionally, the polynucleotides will be full-length sequences. An exemplary number of sequences for statistical analysis can be at least 1, 5, 10, 20, 50, or 100.

### Sequence Shuffling

The present invention provides methods for sequence shuffling using polynucleotides of the present invention, and compositions resulting therefrom. Sequence shuffling is described in PCT publication No. 96/19256. *See also*, Zhang et al., Proc. Natl. Acad. Sci. USA 94:4504-9 (1997); and Zhao et al., Nature Biotech 16:258-61 (1998). Generally, sequence shuffling provides a means for generating libraries of polynucleotides having a desired characteristic, which can be selected or screened for. Libraries of recombinant polynucleotides are generated from a population of related sequence polynucleotides, which comprise sequence regions, which have substantial sequence identity and can be homologously recombined in vitro or in vivo. The population of sequence-recombined polynucleotides comprises a subpopulation of polynucleotides which possess desired or advantageous characteristics and which can be selected by a suitable selection or screening method. The characteristics can be any property or attribute capable of being selected for or detected in a screening system, and may include properties of: an encoded protein, a transcriptional element, a sequence controlling transcription, RNA processing, RNA stability, chromatin conformation, translation, or other expression property of a gene or transgene, a replicative element, a protein-binding element, or the like, such as any feature which confers a selectable or detectable property. In some embodiments, the selected characteristic will be an altered Kₘ and/or K_{cat} over the wild-type protein as provided herein. In other embodiments, a protein or polynucleotide generated from sequence shuffling will have a ligand binding affinity greater than the non-shuffled wild-type polynucleotide. In yet other embodiments, a protein or polynucleotide generated from sequence shuffling will have an altered pH optimum as compared to the non-shuffled wild-type polynucleotide. The increase in such properties can be at least 110%, 120%, 130%, 140% or greater than 150% of the wild-type value.

### Recombinant Expression Cassettes

The present invention further provides recombinant expression cassettes comprising a nucleic acid of the present invention. A nucleic acid sequence coding for the desired polynucleotide of the present invention, for example a cDNA or a genomic sequence encoding a polypeptide long enough to code for an active protein of the present invention, can be used to construct a recombinant expression cassette which can be introduced into the desired host cell. A recombinant expression cassette will typically comprise a polynucleotide of the present invention operably linked to transcriptional initiation regulatory sequences which will direct the transcription of the polynucleotide in the intended host cell, such as tissues of a transformed plant.

For example, plant expression vectors may include (1) a cloned plant gene under the transcriptional control of 5' and 3' regulatory sequences and (2) a dominant selectable marker. Such plant expression vectors may also contain, if desired, a promoter regulatory region (*e.g*., one conferring inducible or constitutive, environmentally- or developmentally-regulated, or cell- or tissue-specific/selective expression), a transcription initiation start site, a ribosome binding site, an RNA processing signal, a transcription termination site, and/or a polyadenylation signal.

A plant promoter fragment can be employed which will direct expression of a polynucleotide of the present invention in all tissues of a regenerated plant. Such promoters are referred to herein as "constitutive" promoters and are active under most environmental conditions and states of development or cell differentiation. Examples of constitutive promoters include the 1'- or 2'- promoter derived from T-DNA of *Agrobacterium tumefaciens*, the Smas promoter, the cinnamyl alcohol dehydrogenase promoter (United States Patent No. 5,683,439), the *Nos* promoter, the rubisco promoter, the GRP1-8 promoter, the 35S promoter from cauliflower mosaic virus (CaMV), as described in Odell et al., Nature 313:810-2 (1985); rice actin (McElroy et al., Plant Cell 163-171 (1990)); ubiquitin (Christensen et al., Plant Mol. Biol. 12:619-632 (1992) and Christensen et al., Plant Mol. Biol. 18:675-89 (1992)); pEMU (Last et al., Theor. Appl. Genet. 81:581-8 (1991)); MAS (Velten et al., EMBO J. 3:2723-30 (1984)); and maize H3 histone (Lepetit et al., Mol. Gen. Genet. 231:276-85 (1992); and Atanassvoa et al., Plant Journal 2(3):291-300 (1992)); ALS promoter, as described in PCT Application No. WO 96/30530; and other transcription initiation regions from various plant genes known to those of skill. For the present invention ubiquitin is the preferred promoter for expression in monocot plants.

Alternatively, the plant promoter can direct expression of a polynucleotide of the present invention in a specific tissue or may be otherwise under more precise environmental or developmental control. Such promoters are referred to here as "inducible" promoters. Environmental conditions that may effect transcription by inducible promoters include pathogen attack, anaerobic conditions, or the presence of light. Examples of inducible promoters are the Adh1 promoter, which is inducible by hypoxia or cold stress, the Hsp70 promoter, which is inducible by heat stress, and the PPDK promoter, which is inducible by light.

Examples of promoters under developmental control include promoters that initiate transcription only, or preferentially, in certain tissues, such as leaves, roots, fruit, seeds, or flowers. The operation of a promoter may also vary depending on its location in the genome. Thus, an inducible promoter may become fully or partially constitutive in certain locations.

If polypeptide expression is desired, it is generally desirable to include a polyadenylation region at the 3'-end of a polynucleotide coding region. The polyadenylation region can be derived from a variety of plant genes, or from T-DNA. The 3' end sequence to be added can be derived from, for example, the nopaline synthase or octopine synthase genes, or alternatively from another plant gene, or less preferably from any other eukaryotic gene. Examples of such regulatory elements include, but are not limited to, 3' termination and/or polyadenylation regions such as those of the *Agrobacterium tumefaciens* nopaline synthase (nos) gene (Bevan et al., Nucleic Acids Res. 12:369-85 (1983)); the potato proteinase inhibitor II (PINII) gene (Keil et al., Nucleic Acids Res. 14:5641-50 (1986); and An et al., Plant Cell 1:115-22 (1989)); and the CaMV 19S gene (Mogen et al., Plant Cell 2:1261-72 (1990)).

An intron sequence can be added to the 5' untranslated region or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg, Mol. Cell Biol. 8:4395-4405 (1988); Callis et al., Genes Dev. 1:1183-200 (1987)). Such intron enhancement of gene expression is typically greatest when placed near the 5' end of the transcription unit. Use of maize introns Adh1-S intron 1, 2, and 6, the Bronze-1 intron are known in the art. *See generally*, THE MAIZE HANDBOOK, Chapter 116, Freeling and Walbot, eds., Springer, New York (1994).

Plant signal sequences, including, but not limited to, signal-peptide encoding DNA/RNA sequences which target proteins to the extracellular matrix of the plant cell (Dratewka-Kos et al., J. Biol. Chem. 264:4896-900 (1989)), such as the *Nicotiana plumbaginifolia* extension gene (DeLoose et al., Gene 99:95-100 (1991)); signal peptides which target proteins to the vacuole, such as the sweet potato sporamin gene (Matsuka et a/., Proc. Natl. Acad. Sci. USA 88:834 (1991)) and the barley lectin gene (Wilkins et al., Plant Cell, 2:301-13 (1990)); signal peptides which cause proteins to be secreted, such as that of PRIb (Lind et al., Plant Mol. Biol. 18:47-53 (1992)) or the barley alpha amylase (BAA) (Rahmatullah et al., Plant Mol. Biol. 12:119 (1989), and hereby incorporated by reference), or signal peptides which target proteins to the plastids such as that of rapeseed enoyl-Acp reductase (Verwaert et al., Plant Mol. Biol. 26:189-202 (1994)) are useful in the invention. The barley alpha amylase signal sequence fused to the PDR polynucleotide is the preferred construct for expression in maize for the present invention.

The vector comprising the sequences from a polynucleotide of the present invention will typically comprise a marker gene, which confers a selectable phenotype on plant cells. Usually, the selectable marker gene will encode antibiotic resistance, with suitable genes including genes coding for resistance to the antibiotic spectinomycin (*e.g*., the aada gene), the streptomycin phosphotransferase (SPT) gene coding for streptomycin resistance, the neomycin phosphotransferase (NPTII) gene encoding kanamycin or geneticin resistance, the hygromycin phosphotransferase (HPT) gene coding for hygromycin resistance, genes coding for resistance to herbicides which act to inhibit the action of acetolactate synthase (ALS), in particular the sulfonylurea-type herbicides (*e.g*., the acetolactate synthase (ALS) gene containing mutations leading to such resistance in particular the S4 and/or Hra mutations), genes coding for resistance to herbicides which act to inhibit action of glutamine synthase, such as phosphinothricin or basta (*e.g*., the *bar* gene), or other such genes known in the art. The *bar* gene encodes resistance to the herbicide basta, and the ALS gene encodes resistance to the herbicide chlorsulfuron.

Typical vectors useful for expression of genes in higher plants are well known in the art and include vectors derived from the tumor-inducing (Ti) plasmid of *Agrobacterium tumefaciens* described by Rogers et al., Meth. Enzymol. 153:253-77 (1987). These vectors are plant integrating vectors in that on transformation, the vectors integrate a portion of vector DNA into the genome of the host plant. Exemplary *A. tumefaciens* vectors useful herein are plasmids pKYLX6 and pKYLX7 of Schardl et al., Gene 61:1-11 (1987), and Berger et al., Proc. Natl. Acad. Sci. USA, 86:8402-6 (1989). Another useful vector herein is plasmid pBI101.2 that is available from CLONTECH Laboratories, Inc. (Palo Alto, CA).

### Expression of Proteins in Host Cells

Using the nucleic acids of the present invention, one may express a protein of the present invention in a recombinantly engineered cell such as bacteria, yeast, insect, mammalian, or preferably plant cells. The cells produce the protein in a non-natural condition (*e.g*., in quantity, composition, location, and/or time), because they have been genetically altered through human intervention to do so.

It is expected that those of skill in the art are knowledgeable in the numerous expression systems available for expression of a nucleic acid encoding a protein of the present invention. No attempt to describe in detail the various methods known for the expression of proteins in prokaryotes or eukaryotes will be made.

In brief summary, the expression of isolated nucleic acids encoding a protein of the present invention will typically be achieved by operably linking, for example, the DNA or cDNA to a promoter (which is either constitutive or inducible), followed by incorporation into an expression vector. The vectors can be suitable for replication and integration in either prokaryotes or eukaryotes. Typical expression vectors contain transcription and translation terminators, initiation sequences, and promoters useful for regulation of the expression of the DNA encoding a protein of the present invention. To obtain high level expression of a cloned gene, it is desirable to construct expression vectors which contain, at the minimum, a strong promoter, such as ubiquitin, to direct transcription, a ribosome binding site for translational initiation, and a transcription/translation terminator. Constitutive promoters are classified as providing for a range of constitutive expression. Thus, some are weak constitutive promoters, and others are strong constitutive promoters. Generally, by "weak promoter" is intended a promoter that drives expression of a coding sequence at a low level. By "low level" is intended at levels of about 1/10,000 transcripts to about 1/100,000 transcripts to about 1/500,000 transcripts. Conversely, a "strong promoter" drives expression of a coding sequence at a "high level," or about 1/10 transcripts to about 1/100 transcripts to about 1/1,000 transcripts.

One of skill would recognize that modifications could be made to a protein of the present invention without diminishing its biological activity. Some modifications may be made to facilitate the cloning, expression, or incorporation of the targeting molecule into a fusion protein. Such modifications are well known to those of skill in the art and include, for example, a methionine added at the amino terminus to provide an initiation site, or additional amino acids (*e.g*., poly His) placed on either terminus to create conveniently located restriction sites or termination codons or purification sequences.

### Expression in Prokaryotes

Prokaryotic cells may be used as hosts for expression. Prokaryotes most frequently are represented by various strains of E. *coli;* however, other microbial strains may also be used. Commonly used prokaryotic control sequences which are defined herein to include promoters for transcription initiation, optionally with an operator, along with ribosome binding site sequences, include such commonly used promoters as the beta lactamase (penicillinase) and lactose (lac) promoter systems (Chang et al., Nature 198:1056 (1977)), the tryptophan (trp) promoter system (Goeddel et al., Nucleic Acids Res. 8:4057 (1980)) and the lambda derived P L promoter and N-gene ribosome binding site (Shimatake et al., Nature 292:128 (1981)). The inclusion of selection markers in DNA vectors transfected in E. *coli* is also useful. Examples of such markers include genes specifying resistance to ampicillin, tetracycline, or chloramphenicol.

The vector is selected to allow introduction of the gene of interest into the appropriate host cell. Bacterial vectors are typically of plasmid or phage origin. Appropriate bacterial cells are infected with phage vector particles or transfected with naked phage vector DNA. If a plasmid vector is used, the bacterial cells are transfected with the plasmid vector DNA. Expression systems for expressing a protein of the present invention are available using *Bacillus sp.* and *Salmonella* (Palva et al., Gene 22:229-35 (1983); Mosbach et al., Nature 302:543-5 (1983)). The pGEX-4T-1 plasmid vector from Pharmacia is the preferred E. *coli* expression vector for the present invention.

### Expression in Eukaryotes

A variety of eukaryotic expression systems such as yeast, insect cell lines, plant and mammalian cells, are known to those of skill in the art. As explained briefly below, the present invention can be expressed in these eukaryotic systems. In some embodiments, transformed/transfected plant cells, as discussed *infra*, are employed as expression systems for production of the proteins of the instant invention.

Synthesis of heterologous proteins in yeast is well known. Sherman et al., METHODS IN YEAST GENETICS, Cold Spring Harbor Laboratory (1982) is a well recognized work describing the various methods available to produce the protein in yeast. Two widely utilized yeasts for production of eukaryotic proteins are *Saccharomyces cerevisiae* and *Pichia pastoris.* Vectors, strains, and protocols for expression in *Saccharomyces* and *Pichia* are known in the art and available from commercial suppliers (*e.g*., Invitrogen). Suitable vectors usually have expression control sequences, such as promoters, including 3-phosphoglycerate kinase or alcohol oxidase, and an origin of replication, termination sequences and the like as desired.

A protein of the present invention, once expressed, can be isolated from yeast by lysing the cells and applying standard protein isolation techniques to the lysates or the pellets. The monitoring of the purification process can be accomplished by using Western blot techniques or radioimmunoassay of other standard immunoassay techniques.

The sequences encoding proteins of the present invention can also be ligated to various expression vectors for use in transfecting cell cultures of, for instance, mammalian, insect, or plant origin. Mammalian cell systems often will be in the form of monolayers of cells although mammalian cell suspensions may also be used. A number of suitable host cell lines capable of expressing intact proteins have been developed in the art, and include the HEK293, BHK21, and CHO cell lines. Expression vectors for these cells can include expression control sequences, such as an origin of replication, a promoter (*e.g*., the CMV promoter, a HSV *tk* promoter or pgk (phosphoglycerate kinase) promoter), an enhancer (Queen et al., Immunol. Rev. 89:49 (1986)), and necessary processing information sites, such as ribosome binding sites, RNA splice sites, polyadenylation sites (*e.g*., an SV40 large T Ag poly A addition site), and transcriptional terminator sequences. Other animal cells useful for production of proteins of the present invention are available, for instance, from the American Type Culture Collection Catalogue of Cell Lines and Hybridomas (7th ed., 1992).

Appropriate vectors for expressing proteins of the present invention in insect cells are usually derived from the SF9 baculovirus. Suitable insect cell lines include mosquito larvae, silkworm, armyworm, moth, and *Drosophila* cell lines such as a Schneider cell line (see, *e.g*., Schneider, J. Embryol. Exp. Morphol. 27:353-65 (1987)).

As with yeast, when higher animal or plant host cells are employed, polyadenlyation or transcription terminator sequences are typically incorporated into the vector. An example of a terminator sequence is the polyadenlyation sequence from the bovine growth hormone gene. Sequences for accurate splicing of the transcript may also be included. An example of a splicing sequence is the VP1 intron from SV40 (Sprague et al., J. Virol. 45:773-81 (1983)). Additionally, gene sequences to control replication in the host cell may be incorporated into the vector such as those found in bovine papilloma virus type-vectors (Saveria-Campo, "Bovine Papilloma Virus DNA a Eukaryotic Cloning Vector," in DNA CLONING: A PRACTICAL APPROACH, vol. II, Glover, ed., IRL Press, Arlington, VA, pp. 213-38 (1985)).

In addition, the gene for PDR placed in the appropriate plant expression vector can be used to transform plant cells. The polypeptide can then be isolated from plant callus or the transformed cells can be used to regenerate transgenic plants. Such transgenic plants can be harvested, and the appropriate tissues (seed or leaves, for example) can be subjected to large scale protein extraction and purification techniques.

### Plant Transformation Methods

Numerous methods for introducing foreign genes into plants are known and can be used to insert a PDR polynucleotide into a plant host, including biological and physical plant transformation protocols. See, *e.g.,* Miki et al., "Procedure for Introducing Foreign DNA into Plants," in METHODS IN PLANT MOLECULAR BIOLOGY AND BIOTECHNOLOGY, Glick and Thompson, eds., CRC Press, Inc., Boca Raton, pp. 67-88 (1993). The methods chosen vary with the host plant, and include chemical transfection methods such as calcium phosphate, microorganism-mediated gene transfer such as *Agrobacterium* (Horsch et al., Science 227:1229-31 (1985)), electroporation, micro-injection, and biolistic bombardment.

Expression cassettes and vectors and *in vitro* culture methods for plant cell or tissue transformation and regeneration of plants are known and available. See, *e.g*., Gruber et al., "Vectors for Plant Transformation," in METHODS IN PLANT MOLECULAR BIOLOGY AND BIOTECHNOLOGY, supra, pp. 89-119.

The isolated polynucleotides or polypeptides may be introduced into the plant by one or more techniques typically used for direct delivery into cells. Such protocols may vary depending on the type of organism, cell, plant, or plant cell, i.e., monocot or dicot, targeted for gene modification. Suitable methods of transforming plant cells include microinjection (Crossway et al., (1986) Biotechniques 4:320-334; and U.S. Patent 6,300,543), electroporation (Riggs et al., (1986) Proc. Natl. Acad. Sci. USA 83:5602-5606, direct gene transfer (Paszkowski et al., (1984) EMBO J. 3:2717-2722), and ballistic particle acceleration (see, for example, Sanford et al., U.S. Patent No. 4,945,050; WO 91/10725; and McCabe et al., (1988) Biotechnology 6:923-926). Also see, Tomes et al., Direct DNA Transfer into Intact Plant Cells Via Microprojectile Bombardment pp.197-213 in Plant Cell, Tissue and Organ Culture, Fundamental Methods eds. O. L. Gamborg & G.C. Phillips. Springer-Verlag Berlin Heidelberg New York, 1995; U.S. Patent 5,736,369 (meristem); Weissinger et al., (1988) Ann. Rev. Genet. 22:421-477; Sanford et al., (1987) Particulate Science and Technology 5:27-37 (onion); Christou et al., (1988) Plant Physiol. 87:671-674 (soybean); Datta et al., (1990) Biotechnology 8:736-740 (rice); Klein et al., (1988) Proc. Natl. Acad. Sci. USA 85:4305-4309 (maize); Klein et al., (1988) Biotechnology 6:559-563 (maize); WO 91/10725 (maize); Klein et al., (1988) Plant Physiol. 91:440-444 (maize); Fromm et al., (1990) Biotechnology 8:833-839; and Gordon-Kamm et al., (1990) Plant Cell 2:603-618 (maize); Hooydaas-Van Slogteren & Hooykaas (1984) Nature (London) 311:763-764; Bytebier et al., (1987) Proc. Natl. Acad. Sci. USA 84:5345-5349 (Liliaceae); De Wet et al., (1985) In The Experimental Manipulation of Ovule Tissues, ed. G.P. Chapman et al., pp. 197-209. Longman, NY (pollen); Kaeppler et al., (1990) Plant Cell Reports 9:415-4.18; and Kaeppler et al., (1992) Theor. Appl. Genet. 84:560-566 (whisker-mediated transformation); U.S. Patent No. 5,693,512 (sonication); D'Halluin et al., (1992) Plant Cell 4:1495-1505 (electroporation); Li et al., (1993) Plant Cell Reports 12:250-255; and Christou & Ford (1995) Annals of Botany 75:407-413 (rice); Osjoda et al., (1996) Nature Biotech. 14:745-750; Agrobacterium mediated maize transformation (U.S. Patent 5,981,840); silicon carbide whisker methods (Frame et al., (1994) Plant J. 6:941-948); laser methods (Guo et al., (1995) Physiologia Plantarum 93:19-24); sonication methods (Bao et al., (1997) Ulfrasound in Medicine & Biology 23:953-959; Finer & Finer (2000) Lett Appl Microbiol. 30:406-10; Amoah et al., (2001) J Exp Bot 52:1135-42); polyethylene glycol methods (Krens et al., (1982) Nature 296:72-77); protoplasts of monocot and dicot cells can be transformed using electroporation (Fromm et al., (1985) Proc. Natl. Acad. Sci. USA 82:5824-5828) and microinjection (Crossway et al., (1986) Mol. Gen. Genet. 202:179-185); all of which are herein incorporated by reference.

### Agrobacterium-mediated Transformation

The most widely utilized method for introducing an expression vector into plants is based on the natural transformation system of *Agrobacterium. A. tumefaciens* and *A. rhizogenes* are plant pathogenic soil bacteria, which genetically transform plant cells. The Ti and Ri plasmids of *A. tumefaciens* and A. *rhizogenes,* respectively, carry genes responsible for genetic transformation of plants. *See, e.g.,* Kado, Crit. Rev. Plant Sci. 10:1 (1991). Descriptions of the *Agrobacterium* vector systems and methods for *Agrobacterium-mediated* gene transfer are provided in Gruber *et al*., *supra;* Miki *et al*., *supra*; and Moloney et al., Plant Cell Reports 8:238 (1989).

Similarly, the gene can be inserted into the T-DNA region of a Ti or Ri plasmid derived from *A. tumefaciens* or *A. rhizogenes,* respectively. Thus, expression cassettes can be constructed as above, using these plasmids. Many control sequences are known which when coupled to a heterologous coding sequence and transformed into a host organism show fidelity in gene expression with respect to tissue/organ specificity of the original coding sequence. *See, e.g.,* Benfey and Chua, Science 244:174-81 (1989). Particularly suitable control sequences for use in these plasmids are promoters for constitutive leaf-specific expression of the gene in the various target plants. Other useful control sequences include a promoter and terminator from the nopaline synthase gene (NOS). The NOS promoter and terminator are present in the plasmid pARC2, available from the American Type Culture Collection and designated ATCC 67238. If such a system is used, the virulence (*vir*) gene from either the Ti or Ri plasmid must also be present, either along with the T-DNA portion, or via a binary system where the *vir* gene is present on a separate vector. Such systems, vectors for use therein, and methods of transforming plant cells are described in United States Patent No. 4,658,082; United States Patent Application No. 913,914, filed Oct. 1, 1986, as referenced in United States Patent No. 5,262,306, issued November 16, 1993; and Simpson et al., Plant Mol. Biol. 6:403-15 (1986) (also referenced in the '306 patent); all incorporated by reference in their entirety.

Once constructed, these plasmids can be placed into *A. rhizogenes* or A. *tumefaciens* and these vectors used to transform cells of plant species, which are ordinarily susceptible to *Fusarium* or *Alternaria* infection. Several other transgenic plants are also contemplated by the present invention including but not limited to soybean, com, sorghum, affalfa, rice, clover, cabbage, banana, coffee, celery, tobacco, cowpea, cotton, melon and pepper. The selection of either A. *tumefaciens* or *A. rhizogenes* will depend on the plant being transformed thereby. In general *A. tumefaciens* is the preferred organism for transformation. Most dicotyledonous plants, some gymnosperms, and a few monocotyledonous plants (e.g., certain members of the *Liliales* and *Arales*) are susceptible to infection with *A. tumefaciens. A. rhizogenes* also has a wide host range, embracing most dicots and some gymnosperms, which includes members of the *Leguminosae, Compositae,* and *Chenopodiaceae.* Monocot plants can now be transformed with some success. European Patent Application No. 604 662 A1 discloses a method for transforming monocots using *Agrobacterium.* European Application No. 672 752 A1 discloses a method for transforming monocots with *Agrobacterium* using the scutellum of immature embryos. Ishida *et al*. discuss a method for transforming maize by exposing immature embryos to *A. tumefaciens* (Nature Biotechnology 14:745-50 (1996)).

Once transformed, these cells can be used to regenerate transgenic plants. For example, whole plants can be infected with these vectors by wounding the plant and then introducing the vector into the wound site. Any part of the plant can be wounded, including leaves, stems and roots. Alternatively, plant tissue, in the form of an explant, such as cotyledonary tissue or leaf disks, can be inoculated with these vectors, and cultured under conditions, which promote plant regeneration. Roots or shoots transformed by inoculation of plant tissue with A. *rhizogenes* or *A. tumefaciens,* containing the gene coding for the fumonisin degradation enzyme, can be used as a source of plant tissue to regenerate fumonisin-resistant transgenic plants, either via somatic embryogenesis or organogenesis. Examples of such methods for regenerating plant tissue are disclosed in Shahin, Theor. Appl. Genet. 69:235-40 (1985); United States Patent No. 4,658,082; Simpson, *et al*., *supra;* and United States Patent Application Nos. 913,913 and 913,914, both filed Oct. 1, 1986, as referenced in United States Patent No. 5,262,306, issued November 16, 1993, the entire disclosures therein incorporated herein by reference.

### Direct Gene Transfer

Despite the fact that the host range for *Agrobacterium-mediated* transformation is broad, some major cereal crop species and gymnosperms have generally been recalcitrant to this mode of gene transfer, even though some success has recently been achieved in rice (Hiei et al., The Plant Journal 6:271-82 (1994)). Several methods of plant transformation, collectively referred to as direct gene transfer, have been developed as an alternative to *Agrobacterium-mediated* transformation.

A generally applicable method of plant transformation is microprojectile-mediated transformation, where DNA is carried on the surface of microprojectiles measuring about 1 to 4 µm. The expression vector is introduced into plant tissues with a biolistic device that accelerates the microprojectiles to speeds of 300 to 600 mls which is sufficient to penetrate the plant cell walls and membranes (Sanford et al., Part. Sci. Technol. 5:27 (1987); Sanford, Trends Biotech 6:299 (1988); Sanford, Physiol. Plant 79:206 (1990); and Klein et al., Biotechnology 10:268 (1992)).

Another method for physical delivery of DNA to plants is sonication of target cells as described in Zang et al., BioTechnology 9:996 (1991). Alternatively, liposome or spheroplast fusions have been used to introduce expression vectors into plants. *See, e.g.,* Deshayes et al., EMBO J. 4:2731 (1985); and Christou et al., Proc. Natl. Acad. Sci. USA 84:3962 (1987). Direct uptake of DNA into protoplasts using CaCl₂ precipitation, polyvinyl alcohol, or poly-L-ornithine has also been reported. *See, e.g.,* Hain et al., Mol. Gen. Genet 199:161 (1985); and Draper et al., Plant Cell Physiol. 23:451 (1982).

Electroporation of protoplasts and whole cells and tissues has also been described. *See, e.g.,* Donn et al., in Abstracts of the Vllth Int'l. Congress on Plant Cell and Tissue Culture IAPTC, A2-38, p. 53 (1990); D'Halluin et al., Plant Cell 4:1495-505 (1992); and Spencer et al., Plant Mol. Biol. 24:51-61 (1994).

### Increasing the Activity and/or Level of a PDR Polypeptide

Methods are provided to increase the activity and/or level of the PDR polypeptide of the invention. An increase in the level and/or activity of the PDR polypeptide of the invention can be achieved by providing to the plant a PDR polypeptide. The PDR polypeptide can be provided by introducing the amino acid sequence encoding the PDR polypeptide into the plant, introducing into the plant a nucleotide sequence encoding a PDR polypeptide or alternatively by modifying a genomic locus encoding the PDR polypeptide of the invention.

As discussed elsewhere herein, many methods are known the art for providing a polypeptide to a plant including, but not limited to, direct introduction of the polypeptide into the plant, introducing into the plant (transiently or stably) a polynucleotide construct encoding a polypeptide having cell development regulator activity. It is also recognized that the methods of the invention may employ a polynucleotide that is not capable of directing, in the transformed plant, the expression of a protein or an RNA. Thus, the level and/or activity of a PDR polypeptide may be increased by altering the gene encoding the PDR polypeptide or its promoter. See, e.g., Kmiec, U.S. Patent 5,565,350; Zarling et al., PCT/US93/03868. Therefore mutagenized plants that carry mutations in PDR genes, where the mutations increase expression of the PDR gene or increase the cell development regulator activity of the encoded PDR polypeptide are provided.

### Reducing the Activity and/or Level of a PDR Polypeptide

Methods are provided to reduce or eliminate the activity of a PDR polypeptide of the invention by transforming a plant cell with an expression cassette that expresses a polynucleotide that inhibits the expression of the PDR polypeptide. The polynucleotide may inhibit the expression of the PDR polypeptide directly, by preventing translation of the PDR messenger RNA, or indirectly, by encoding a polypeptide that inhibits the transcription or translation of a PDR gene encoding a PDR polypeptide. Methods for inhibiting or eliminating the expression of a gene in a plant are well known in the art, and any such method may be used in the present invention to inhibit the expression of a PDR polypeptide.

In accordance with the present invention, the expression of a PDR polypeptide is inhibited if the protein level of the PDR polypeptide is less than 70% of the protein level of the same PDR polypeptide in a plant that has not been genetically modified or mutagenized to inhibit the expression of that PDR polypeptide. In particular embodiments of the invention, the protein level of the PDR polypeptide in a modified plant according to the invention is less than 60%, less than 50%, less than 40%, less than 30%, less than 20%, less than 10%, less than 5%, or less than 2% of the protein level of the same PDR polypeptide in a plant that is not a mutant or that has not been genetically modified to inhibit the expression of that PDR polypeptide. The expression level of the PDR polypeptide may be measured directly, for example, by assaying for the level of PDR polypeptide expressed in the plant cell or plant, or indirectly, for example, by measuring the cell development regulator activity of the PDR polypeptide in the plant cell or plant, or by measuring the cell development in the plant. Methods for performing such assays are described elsewhere herein.

In other embodiments of the invention, the activity of the PDR polypeptides is reduced or eliminated by transforming a plant cell with an expression cassette comprising a polynucleotide encoding a polypeptide that inhibits the activity of a PDR polypeptide. The cell development regulator activity of a PDR polypeptide is inhibited according to the present invention if the cell development regulator activity of the PDR polypeptide is less than 70% of the cell development regulator activity of the same PDR polypeptide in a plant that has not been modified to inhibit the cell development regulator activity of that PDR polypeptide. In particular embodiments of the invention, the cell development regulator activity of the PDR polypeptide in a modified plant according to the invention is less than 60%, less than 50%, less than 40%, less than 30%, less than 20%, less than 10%, or less than 5% of the cell development regulator activity of the same PDR polypeptide in a plant that that has not been modified to inhibit the expression of that PDR polypeptide. The cell development regulator activity of a PDR polypeptide is "eliminated" according to the invention when it is not detectable by the assay methods described elsewhere herein. Methods of determining the cell development regulator activity of a PDR polypeptide are described elsewhere herein.

In other embodiments, the activity of a PDR polypeptide may be reduced or eliminated by disrupting the gene encoding the PDR polypeptide. The invention encompasses mutagenized plants that carry mutations in PDR genes, where the mutations reduce expression of the PDR gene or inhibit the cell development regulator activity of the encoded PDR polypeptide.

Thus, many methods may be used to reduce or eliminate the activity of a PDR polypeptide. In addition, more than one method may be used to reduce the activity of a single PDR polypeptide. Non-limiting examples of methods of reducing or eliminating the expression of PDR polypeptides are given below.

### 1. Polynucleotide-Based Methods:

In some embodiments of the present invention, a plant is transformed with an expression cassette that is capable of expressing a polynucleotide that inhibits the expression of a PDR polypeptide of the invention. The term "expression" as used herein refers to the biosynthesis of a gene product, including the transcription and/or translation of said gene product. For example, for the purposes of the present invention, an expression cassette capable of expressing a polynucleotide that inhibits the expression of at least one PDR polypeptide is an expression cassette capable of producing an RNA molecule that inhibits the transcription and/or translation of at least one PDR polypeptide of the invention. The "expression" or "production" of a protein or polypeptide from a DNA molecule refers to the transcription and translation of the coding sequence to produce the protein or polypeptide, while the "expression" or "production" of a protein or polypeptide from an RNA molecule refers to the translation of the RNA coding sequence to produce the protein or polypeptide.

Examples of polynucleotides that inhibit the expression of a PDR polypeptide are given below.

### i. Sense SuppressionlCosuppression

In some embodiments of the invention, inhibition of the expression of a PDR polypeptide may be obtained by sense suppression or cosuppression. For cosuppression, an expression cassette is designed to express an RNA molecule corresponding to all or part of a messenger RNA encoding a PDR polypeptide in the "sense" orientation. Over expression of the RNA molecule can result in reduced expression of the native gene. Accordingly, multiple plant lines transformed with the cosuppression expression cassette are screened to identify those that show the greatest inhibition of PDR polypeptide expression.

The polynucleotide used for cosuppression may correspond to all or part of the sequence encoding the PDR polypeptide, all or part of the 5' and/or 3' untranslated region of a PDR polypeptide transcript, or all or part of both the coding sequence and the untranslated regions of a transcript encoding a PDR polypeptide. In some embodiments where the polynucleotide comprises all or part of the coding region for the PDR polypeptide, the expression cassette is designed to eliminate the start codon of the polynucleotide so that no protein product will be translated.

Cosuppression may be used to inhibit the expression of plant genes to produce plants having undetectable protein levels for the proteins encoded by these genes. See, for example, Broin et al. (2002) Plant Cell 14:1417-1432. Cosuppression may also be used to inhibit the expression of multiple proteins in the same plant. See, for example, U.S. Patent No. 5,942,657. Methods for using cosuppression to inhibit the expression of endogenous genes in plants are described in Flavell et al. (1994) Proc. Natl. Acad. Sci. USA 91:3490-3496; Jorgensen et al. (1996) Plant Mol. Biol. 31:957-973; Johansen and Carrington (2001) Plant Physiol. 126:930-938; Broin et al. (2002) Plant Cell 14:1417-1432; Stoutjesdijk et al. (2002) Plant Physiol. 129:1723-1731; Yu et al. (2003) Phytochemistry 63:753-763; and U.S. Patent Nos. 5,034,323, 5,283,184, and 5,942,657; each of which is herein incorporated by reference. The efficiency of cosuppression may be increased by including a poly-dT region in the expression cassette at a position 3' to the sense sequence and 5' of the polyadenylation signal. See, U.S. Patent Publication No. 20020048814, herein incorporated by reference. Typically, such a nucleotide sequence has substantial sequence identity to the sequence of the transcript of the endogenous gene, optimally greater than about 65% sequence identity, more optimally greater than about 85% sequence identity, most optimally greater than about 95% sequence identity. See U.S. Patent Nos. 5,283,184 and 5,034,323; herein incorporated by reference.

### ii. Antisense Suppression

In some embodiments of the invention, inhibition of the expression of the PDR polypeptide may be obtained by antisense suppression. For antisense suppression, the expression cassette is designed to express an RNA molecule complementary to all or part of a messenger RNA encoding the PDR polypeptide. Over expression of the antisense RNA molecule can result in reduced expression of the native gene. Accordingly, multiple plant lines transformed with the antisense suppression expression cassette are screened to identify those that show the greatest inhibition of PDR polypeptide expression.

The polynucleotide for use in antisense suppression may correspond to all or part of the complement of the sequence encoding the PDR polypeptide, all or part of the complement of the 5' and/or 3' untranslated region of the PDR transcript, or all or part of the complement of both the coding sequence and the untranslated regions of a transcript encoding the PDR polypeptide. In addition, the antisense polynucleotide may be fully complementary (i.e., 100% identical to the complement of the target sequence) or partially complementary (i.e., less than 100% identical to the complement of the target sequence) to the target sequence. Antisense suppression may be used to inhibit the expression of multiple proteins in the same plant. See, for example, U.S. Patent No. 5,942,657. Furthermore, portions of the antisense nucleotides may be used to disrupt the expression of the target gene. Generally, sequences of at least 50 nucleotides, 100 nucleotides, 200 nucleotides, 300, 400, 450, 500, 550, or greater may be used. Methods for using antisense suppression to inhibit the expression of endogenous genes in plants are described, for example, in Liu et al. (2002) Plant Physiol. 129:1732-1743 and U.S. Patent Nos. 5,759,829 and 5,942,657, each of which is herein incorporated by reference. Efficiency of antisense suppression may be increased by including a poly-dT region in the expression cassette at a position 3' to the antisense sequence and 5' of the polyadenylation signal. See, U.S. Patent Publication No. 20020048814, herein incorporated by reference.

### iii. Double-Stranded RNA Interference

In some embodiments of the invention, inhibition of the expression of a PDR polypeptide may be obtained by double-stranded RNA (dsRNA) interference. For dsRNA interference, a sense RNA molecule like that described above for cosuppression and an antisense RNA molecule that is fully or partially complementary to the sense RNA molecule are expressed in the same cell, resulting in inhibition of the expression of the corresponding endogenous messenger RNA.

Expression of the sense and antisense molecules can be accomplished by designing the expression cassette to comprise both a sense sequence and an antisense sequence. Alternatively, separate expression cassettes may be used for the sense and antisense sequences. Multiple plant lines transformed with the dsRNA interference expression cassette or expression cassettes are then screened to identify plant lines that show the greatest inhibition of PDR polypeptide expression. Methods for using dsRNA interference to inhibit the expression of endogenous plant genes are described in Waterhouse et al. (1998) Proc. Natl. Acad. Sci. USA 95:13959-13964, Liu et al. (2002) Plant Physiol. 129:1732-1743, and WO 99/49029, WO 99/53050, WO 99/61631, and WO 00/49035; each of which is herein incorporated by reference.

### iv. Hairpin RNA Interference and Intron-Containing Hairpin RNA Interference

In some embodiments of the invention, inhibition of the expression of one or a PDR polypeptide may be obtained by hairpin RNA (hpRNA) interference or intron-containing hairpin RNA (ihpRNA) interference. These methods are highly efficient at inhibiting the expression of endogenous genes. See, Waterhouse and Helliwell (2003) Nat. Rev. Genet. 4:29-38 and the references cited therein.

For hpRNA interference, the expression cassette is designed to express an RNA molecule that hybridizes with itself to form a hairpin structure that comprises a single-stranded loop region and a base-paired stem. The base-paired stem region comprises a sense sequence corresponding to all or part of the endogenous messenger RNA encoding the gene whose expression is to be inhibited, and an antisense sequence that is fully or partially complementary to the sense sequence. Thus, the base-paired stem region of the molecule generally determines the specificity of the RNA interference. hpRNA molecules are highly efficient at inhibiting the expression of endogenous genes, and the RNA interference they induce is inherited by subsequent generations of plants. See, for example, Chuang and Meyerowitz (2000) Proc. Natl. Acad. Sci. USA 97:4985-4990; Stoutjesdijk et al. (2002) Plant Physiol. 129:1723-1731; and Waterhouse and Helliwell (2003) Nat. Rev. Genet. 4:29-38. Methods for using hpRNA interference to inhibit or silence the expression of genes are described, for example, in Chuang and Meyerowitz (2000) Proc. Natl. Acad. Sci. USA 97:4985-4990; Stoutjesdijk et al. (2002) Plant Physiol. 129:1723-1731; Waterhouse and Helliwell (2003) Nat. Rev. Genet. 4:29-38; Pandolfini et al. BMC Biotechnology 3:7, and U.S. Patent Publication No. 20030175965; each of which is herein incorporated by reference. A transient assay for the efficiency of hpRNA constructs to silence gene expression *in vivo* has been described by Panstruga et al. (2003) Mol. Biol. Rep. 30:135-140, herein incorporated by reference.

For ihpRNA, the interfering molecules have the same general structure as for hpRNA, but the RNA molecule additionally comprises an intron that is capable of being spliced in the cell in which the ihpRNA is expressed. The use of an intron minimizes the size of the loop in the hairpin RNA molecule following splicing, and this increases the efficiency of interference. See, for example, Smith et a/. (2000) Nature 407:319-320. In fact, Smith *et a*/. show 100% suppression of endogenous gene expression using ihpRNA-mediated interference. Methods for using ihpRNA interference to inhibit the expression of endogenous plant genes are described, for example, in Smith et al. (2000) Nature 407:319-320; Wesley et al. (2001) Plant J. 27:581-590; Wang and Waterhouse (2001) Curr. Opin. Plant Biol. 5:146-150; Waterhouse and Helliwell (2003) Nat. Rev. Genet. 4:29-38; Helliwell and Waterhouse (2003) Methods 30:289-295, and U.S. Patent Publication No. 20030180945, each of which is herein incorporated by reference.

The expression cassette for hpRNA interference may also be designed such that the sense sequence and the antisense sequence do not correspond to an endogenous RNA. In this embodiment, the sense and antisense sequence flank a loop sequence that comprises a nucleotide sequence corresponding to all or part of the endogenous messenger RNA of the target gene. Thus, it is the loop region that determines the specificity of the RNA interference. See, for example, WO 02/00904, herein incorporated by reference.

### v. Amplicon-Mediated Interference

Amplicon expression cassettes comprise a plant virus-derived sequence that contains all or part of the target gene but generally not all of the genes of the native virus. The viral sequences present in the transcription product of the expression cassette allow the transcription product to direct its own replication. The transcripts produced by the amplicon may be either sense or antisense relative to the target sequence (i.e., the messenger RNA for the PDR polypeptide). Methods of using amplicons to inhibit the expression of endogenous plant genes are described, for example, in Angell and Baulcombe (1997) EMBO J. 16:3675-3684, Angell and Baulcombe (1999) Plant J. 20:357-362, and U.S. Patent No. 6,646,805, each of which is herein incorporated by reference.

### vi. Ribozymes

In some embodiments, the polynucleotide expressed by the expression cassette of the invention is catalytic RNA or has ribozyme activity specific for the messenger RNA of the PDR polypeptide. Thus, the polynucleotide causes the degradation of the endogenous messenger RNA, resulting in reduced expression of the PDR polypeptide. This method is described, for example, in U.S. Patent No. 4,987,071, herein incorporated by reference.

### vii. Small Interfering RNA or Micro RNA

In some embodiments of the invention, inhibition of the expression of a PDR polypeptide may be obtained by RNA interference by expression of a gene encoding a micro RNA (miRNA). miRNAs are regulatory agents consisting of about 22 ribonucleotides. miRNA are highly efficient at inhibiting the expression of endogenous genes. See, for example Javier et al. (2003) Nature 425: 257-263, herein incorporated by reference.

For miRNA interference, the expression cassette is designed to express an RNA molecule that is modeled on an endogenous miRNA gene. The miRNA gene encodes an RNA that forms a hairpin structure containing a 22-nucleotide sequence that is complementary to another endogenous gene (target sequence). For suppression of PDR expression, the 22-nucleotide sequence is selected from a PDR transcript sequence and contains 22 nucleotides of said PDR sequence in sense orientation and 21 nucleotides of a corresponding antisense sequence that is complementary to the sense sequence. miRNA molecules are highly efficient at inhibiting the expression of endogenous genes, and the RNA interference they induce is inherited by subsequent generations of plants.

### 2. Polypeptide-Based Inhibition of Gene Expression

In one embodiment, the polynucleotide encodes a zinc finger protein that binds to a gene encoding a PDR polypeptide, resulting in reduced expression of the gene. In particular embodiments, the zinc finger protein binds to a regulatory region of a PDR gene. In other embodiments, the zinc finger protein binds to a messenger RNA encoding a PDR polypeptide and prevents its translation. Methods of selecting sites for targeting by zinc finger proteins have been described, for example, in U.S. Patent No. 6,453,242, and methods for using zinc finger proteins to inhibit the expression of genes in plants are described, for example, in U.S. Patent Publication No. 20030037355; each of which is herein incorporated by reference.

### 3. Polypeptide-Based Inhibition of Protein Activity

In some embodiments of the invention, the polynucleotide encodes an antibody that binds to at least one PDR polypeptide, and reduces the cell development regulator activity of the PDR polypeptide. In another embodiment, the binding of the antibody results in increased turnover of the antibody-PDR complex by cellular quality control mechanisms. The expression of antibodies in plant cells and the inhibition of molecular pathways by expression and binding of antibodies to proteins in plant cells are well known in the art. See, for example, Conrad and Sonnewald (2003) Nature Biotech. 21:35-36, incorporated herein by reference.

### 4. Gene Disruption

In some embodiments of the present invention, the activity of a PDR polypeptide is reduced or eliminated by disrupting the gene encoding the PDR polypeptide. The gene encoding the PDR polypeptide may be disrupted by any method known in the art. For example, in one embodiment, the gene is disrupted by transposon tagging. In another embodiment, the gene is disrupted by mutagenizing plants using random or targeted mutagenesis, and selecting for plants that have reduced cell development regulator activity.

### i. Transposon Tagging

In one embodiment of the invention, transposon tagging is used to reduce or eliminate the PDR activity of one or more PDR polypeptide. Transposon tagging comprises inserting a transposon within an endogenous PDR gene to reduce or eliminate expression of the PDR polypeptide. "PDR gene" is intended to mean the gene that encodes a PDR polypeptide according to the invention.

In this embodiment, the expression of one or more PDR polypeptide is reduced or eliminated by inserting a transposon within a regulatory region or coding region of the gene encoding the PDR polypeptide. A transposon that is within an exon, intron, 5' or 3' untranslated sequence, a promoter, or any other regulatory sequence of a PDR gene may be used to reduce or eliminate the expression and/or activity of the encoded PDR polypeptide.

Methods for the transposon tagging of specific genes in plants are well known in the art. See, for example, Maes et al. (1999) Trends Plant Sci. 4:90-96; Dharmapuri and Sonti (1999) FEMS Microbiol. Left. 179:53-59; Meissner et al. (2000) Plant J. 22:265-274; Phogat et al. (2000) J. Biosci. 25:57-63; Walbot (2000) Curr. Opin. Plant Biol. 2:103-107; Gai et al. (2000) Nucleic Acids Res. 28:94-96; Fitzmaurice et al. (1999) Genetics 153:1919-1928). In addition, the TUSC process for selecting Mu insertions in selected genes has been described in Bensen et al. (1995) Plant Cell 7:75-84; Mena et al. (1996) Science 274:1537-1540; and U.S. Patent No. 5,962,764; each of which is herein incorporated by reference.

### ii. Mutant Plants with Reduced Activity

Additional methods for decreasing or eliminating the expression of endogenous genes in plants are also known in the art and can be similarly applied to the instant invention. These methods include other forms of mutagenesis, such as ethyl methanesulfonate-induced mutagenesis, deletion mutagenesis, and fast neutron deletion mutagenesis used in a reverse genetics sense (with PCR) to identify plant lines in which the endogenous gene has been deleted. For examples of these methods see Ohshima et al. (1998) Virology 243:472-481; Okubara et al. (1994) Genetics 137:867-874; and Quesada et al. (2000) Genetics 154:421-436; each of which is herein incorporated by reference. In addition, a fast and automatable method for screening for chemically induced mutations, TILLING (Targeting Induced Local Lesions In Genomes), using denaturing HPLC or selective endonuclease digestion of selected PCR products is also applicable to the instant invention. See McCallum et al. (2000) Nat. Biotechnol. 18:455-457, herein incorporated by reference.

Mutations that impact gene expression or that interfere with the function (cell development regulator activity) of the encoded protein are well known in the art. Insertional mutations in gene exons usually result in null-mutants. Mutations in conserved residues are particularly effective in inhibiting the cell development regulator activity of the encoded protein. Conserved residues of plant PDR polypeptides suitable for mutagenesis with the goal to eliminate cell development regulator activity have been described. Such mutants can be isolated according to well-known procedures, and mutations in different PDR loci can be stacked by genetic crossing. See, for example, Gruis et al. (2002) Plant Cell 14:2863-2882.

In another embodiment of this invention, dominant mutants can be used to trigger RNA silencing due to gene inversion and recombination of a duplicated gene locus. See, for example, Kusaba et al. (2003) Plant Cell 15:1455-1467.

The invention encompasses additional methods for reducing or eliminating the activity of one or more PDR polypeptides. Examples of other methods for altering or mutating a genomic nucleotide sequence in a plant are known in the art and include, but are not limited to, the use of RNA:DNA vectors, RNA:DNA mutational vectors, RNA:DNA repair vectors, mixed-duplex oligonucleotides, self-complementary RNA:DNA oligonucleotides, and recombinogenic oligonucleobases. Such vectors and methods of use are known in the art. See, for example, U.S. Patent Nos. 5,565,350; 5,731,181; 5,756,325; 5,760,012; 5,795,972; and 5,871,984; each of which are herein incorporated by reference. See also, WO 98/49350, WO 99/07865, WO 99/25821, and Beetham et al. (1999) Proc. Natl. Acad. Sci. USA 96:8774-8778; each of which is herein incorporated by reference.

### iii. Modulating plant architecture

In specific methods, the increased growth of yield improvement associated tissues in a plant is caused by increasing the level or activity of the PDR polypeptide in the plant. Methods for increasing the level and/or activity of PDR polypeptides in a plant are discussed elsewhere herein. Briefly, such methods comprise providing a PDR polypeptide of the invention to a plant and thereby increasing the level and/or activity of the PDR polypeptide. In other embodiments, a PDR nucleotide sequence encoding a PDR polypeptide can be provided by introducing into the plant a polynucleotide comprising a PDR nucleotide sequence of the invention, expressing the PDR sequence, increasing the activity of the PDR polypeptide, and thereby causing increases in the yield improvement associate related plant architecture in the plant or plant part. In other embodiments, the PDR nucleotide construct introduced into the plant is stably incorporated into the genome of the plant.

In other methods, the number and shape of a yield associated plant tissue is increased by increasing the level and/or activity of the PDR polypeptide in the plant. Such methods are disclosed in detail elsewhere herein. In one such method, a PDR nucleotide sequence is introduced into the plant and expression of said PDR nucleotide sequence increases the activity of the PDR polypeptide, and thereby increasing the size or shape of the tissue in the plant or plant part. In other embodiments, the PDR nucleotide construct introduced into the plant is stably incorporated into the genome of the plant.

As discussed above, one of skill will recognize the appropriate promoter to use to modulate the level/activity of the yield improvement associated polypeptides in the plant. Exemplary promoters for this embodiment have been disclosed elsewhere herein.

Accordingly, the present invention further provides plants having modified plant architecture when compared to the architecture of a control plant. In one embodiment, maize plants of the invention have an increased level/activity of the PDR polypeptide of the invention and thus exhibit one or more of the following phenotypic characteristics: an increased kernel number per ear, increased spikelet density, tassel branch number, pollen production, improved canopy shape and increased photosynthetic capacity in the leaf tissue, and improved stalk strength and plant standability. In other embodiments, the plants of the invention have an increased level of the PDR polypeptide of the invention resulting in an alteration of vascular bundle structure and number in the plant tissue. In other embodiments, such plants have stably incorporated into their genome a nucleic acid molecule comprising a PDR nucleotide sequence of the invention operably linked to a promoter that drives expression in the plant cell.

### iv. Modulating Root Development

Methods for modulating root development in a plant are provided. By "modulating root development" is intended any alteration in the development of the plant root when compared to a control plant. Such alterations in root development include, but are not limited to, alterations in the growth rate of the primary root, the fresh root weight, the extent of lateral and adventitious root formation, the vasculature system, meristem development, or radial expansion.

Methods for modulating root development in a plant are provided. The methods comprise modulating the level and/or activity of the PDR polypeptide in the plant. In one method, a PDR sequence of the invention is provided to the plant. In another method, the PDR nucleotide sequence is provided by introducing into the plant a polynucleotide comprising a PDR nucleotide sequence of the invention, expressing the PDR sequence, and thereby modifying root development. In still other methods, the PDR nucleotide construct introduced into the plant is stably incorporated into the genome of the plant.

In other methods, root development is modulated by altering the level or activity of the PDR polypeptide in the plant. An increase in PDR activity can result in at least one or more of the following alterations to root development, including, but not limited to, larger root meristems, increases in root growth, enhanced radial expansion, an enhanced vasculature system, increased root branching, more adventitious roots, and/or an increase in fresh root weight when compared to a control plant.

As used herein, "root growth" encompasses all aspects of growth of the different parts that make up the root system at different stages of its development in both monocotyledonous and dicotyledonous plants. It is to be understood that enhanced root growth can result from enhanced growth of one or more of its parts including the primary root, lateral roots, adventitious roots, etc.

Methods of measuring such developmental alterations in the root system are known in the art. See, for example, U.S. Application No. 2003/0074698 and Wemer et al. (2001) PNAS 18:10487-10492, both of which are herein incorporated by reference.

As discussed above, one of skill will recognize the appropriate promoter to use to modulate root development in the plant. Exemplary promoters for this embodiment include constitutive promoters and root-preferred promoters. Exemplary root-preferred promoters have been disclosed elsewhere herein.

Stimulating root growth and increasing root mass by increasing the activity and/or level of the PDR polypeptide also finds use in improving the standability of a plant. The term "resistance to lodging" or "standability" refers to the ability of a plant to fix itself to the soil. For plants with an erect or semi-erect growth habit, this term also refers to the ability to maintain an upright position under adverse (environmental) conditions. This trait relates to the size, depth and morphology of the root system. In addition, stimulating root growth and increasing root mass by decreasing the level and/or activity of the PDR polypeptide also finds use in promoting *in vitro* propagation of explants.

Furthermore, higher root biomass production due to an increased level and/or activity of PDR activity has a direct effect on the yield and an indirect effect of production of compounds produced by root cells or transgenic root cells or cell cultures of said transgenic root cells. One example of an interesting compound produced in root cultures is shikonin, the yield of which can be advantageously enhanced by said methods.

Accordingly, the present invention further provides plants having modulated root development when compared to the root development of a control plant. In some embodiments, the plant of the invention has an increased level/activity of the PDR polypeptide of the invention and has enhanced root growth and/or root biomass. In other embodiments, such plants have stably incorporated into their genome a nucleic acid molecule comprising a PDR nucleotide sequence of the invention operably linked to a promoter that drives expression in the plant cell.

### v. Modulating Shoot and Leaf Development

Methods are also provided for modulating shoot and leaf development in a plant. By "modulating shoot and/or leaf development" is intended any alteration in the development of the plant shoot and/or leaf. Such alterations in shoot and/or leaf development include, but are not limited to, alterations in shoot meristem development, in leaf number, leaf size, leaf and stem vasculature, internode length, and leaf senescence. As used herein, "leaf development" and "shoot development" encompasses all aspects of growth of the different parts that make up the leaf system and the shoot system, respectively, at different stages of their development, both in monocotyledonous and dicotyledonous plants. Methods for measuring such developmental alterations in the shoot and leaf system are known in the art. See, for example, Werner et al. (2001) PNAS 98:10487-10492 and U.S. Application No. 2003/0074698, each of which is herein incorporated by reference.

The method for modulating shoot and/or leaf development in a plant comprises modulating the activity and/or level of a PDR polypeptide of the invention. In one embodiment, a PDR sequence of the invention is provided. In other embodiments, the PDR nucleotide sequence can be provided by introducing into the plant a polynucleotide comprising a PDR nucleotide sequence of the invention, expressing the PDR sequence, and thereby modifying shoot and/or leaf development. In other embodiments, the PDR nucleotide construct introduced into the plant is stably incorporated into the genome of the plant.

In specific embodiments, shoot or leaf development is modulated by increasing the level and/or activity of the PDR polypeptide in the plant. An increase in PDR activity can result in at least one or more of the following alterations in shoot and/or leaf development, including, but not limited to, increased leaf number, increased leaf surface, increased vascularity, longer internodes and improved growth, and altered leaf senescence, when compared to a control plant.

As discussed above, one of skill will recognize the appropriate promoter to use to modulate shoot and leaf development of the plant. Exemplary promoters for this embodiment include constitutive promoters, shoot-preferred promoters, shoot meristem-preferred promoters, and leaf-preferred promoters. Exemplary promoters have been disclosed elsewhere herein.

Decreasing PDR activity and/or level in a plant results in shorter internodes and stunted growth. Thus, the methods of the invention find use in producing dwarf plants. In addition, as discussed above, modulation PDR activity in the plant modulates both root and shoot growth. Thus, the present invention further provides methods for altering the root/shoot ratio. Shoot or leaf development can further be modulated by decreasing the level and/or activity of the PDR polypeptide in the plant.

Accordingly, the present invention further provides plants having modulated shoot and/or leaf development when compared to a control plant. In some embodiments, the plant of the invention has an increased level/activity of the PDR polypeptide of the invention. In other embodiments, the plant of the invention has a decreased level/activity of the PDR polypeptide of the invention.

### vi. Modulating Reproductive Tissue Development

Methods for modulating reproductive tissue development are provided. In one embodiment, methods are provided to modulate floral development in a plant. By "modulating floral development" is intended any alteration in a structure of a plant's reproductive tissue as compared to a control plant in which the activity or level of the PDR polypeptide has not been modulated. "Modulating floral development" further includes any alteration in the timing of the development of a plant's reproductive tissue (i.e., a delayed or an accelerated timing of floral development) when compared to a control plant in which the activity or level of the PDR polypeptide has not been modulated. Macroscopic alterations may include changes in size, shape, number, or location of reproductive organs, the developmental time period that these structures form, or the ability to maintain or proceed through the flowering process in times of environmental stress. Microscopic alterations may include changes to the types or shapes of cells that make up the reproductive organs.

The method for modulating floral development in a plant comprises modulating PDR activity in a plant. In one method, a PDR sequence of the invention is provided. A PDR nucleotide sequence can be provided by introducing into the plant a polynucleotide comprising a PDR nucleotide sequence of the invention, expressing the PDR sequence, and thereby modifying floral development. In other embodiments, the PDR nucleotide construct introduced into the plant is stably incorporated into the genome of the plant.

In specific methods, floral development is modulated by increasing the level or activity of the PDR polypeptide in the plant. An increase in PDR activity can result in at least one or more of the following alterations in floral development, including, but not limited to, more rapid flowering, increased number of flowers, and increased seed set, when compared to a control plant. Inducing more rapid flowering can be used to enhance yield in forage crops such as alfalfa. Methods for measuring such developmental alterations in floral development are known in the art. See, for example, Mouradov et al. (2002) The Plant Cell S111-S130, herein incorporated by reference.

As discussed above, one of skill will recognize the appropriate promoter to use to modulate floral development of the plant. Exemplary promoters for this embodiment include constitutive promoters, inducible promoters, shoot-preferred promoters, and inflorescence-preferred promoters.

In other methods, floral development is modulated by increasing the level and/or activity of the PDR sequence of the invention. Such methods can comprise introducing a PDR nucleotide sequence into the plant and increasing the activity of the PDR polypeptide. In other methods, the PDR nucleotide construct introduced into the plant is stably incorporated into the genome of the plant. Increased expression of the PDR sequence of the invention can modulate floral development during periods of stress. Such methods are described elsewhere herein. Accordingly, the present invention further provides plants having modulated floral development when compared to the floral development of a control plant. Compositions include plants having a decreased level/activity of the PDR polypeptide of the invention and having an altered floral development. Compositions also include plants having an increased level/activity of the PDR polypeptide of the invention wherein the plant maintains or proceeds through the flowering process in times of stress.

Methods are also provided for the use of the PDR sequences of the invention to increase seed number. The method comprises increasing the activity of the PDR sequences in a plant or plant part, such as the seed. An increase in seed size and/or number comprises an increased size or number of the seed and/or an increase in the size of one or more seed part including, for example, the embryo, endosperm, seed coat, aleurone, or cotyledon.

As discussed above, one of skill will recognize the appropriate promoter to use to increase seed size and/or seed number. Exemplary promoters of this embodiment include constitutive promoters, inducible promoters, seed-preferred promoters, embryo-preferred promoters, and endosperm-preferred promoters.

Accordingly, the present invention further provides plants having an increased seed weight and/or seed number when compared to a control plant. In other embodiments, plants having an increased vigor and plant yield are also provided. In some embodiments, the plant of the invention has an increased level/activity of the PDR polypeptide of the invention and has an increased seed number and/or seed size. In other embodiments, such plants have stably incorporated into their genome a nucleic acid molecule comprising a PDR nucleotide sequence of the invention operably linked to a promoter that drives expression in the plant cell.

### vii. Method of Use for PDR promoter polynucleotides

The polynucleotides comprising the PDR promoters disclosed in the present invention, as well as variants and fragments thereof, are useful in the genetic manipulation of any host cell, preferably plant cell, when assembled with a DNA construct such that the promoter sequence is operably linked to a nucleotide sequence comprising a polynucleotide of interest. In this manner, the PDR promoter polynucleotides of the invention are provided in expression cassettes along with a polynucleotide sequence of interest for expression in the host cell of interest. As discussed in Example 2 below, the PDR promoter sequences of the invention are expressed in a variety of tissues and thus the promoter sequences can find use in regulating the temporal and/or the spatial expression of polynucleotides of interest.

Synthetic hybrid promoter regions are known in the art. Such regions comprise upstream promoter elements of one polynucleotide operably linked to the promoter element of another polynucleotide. In an embodiment of the invention, heterologous sequence expression is controlled by a synthetic hybrid promoter comprising the PDR promoter sequences of the invention, or a variant or fragment thereof, operably linked to upstream promoter element(s) from a heterologous promoter. Upstream promoter elements that are involved in the plant defense system have been identified and may be used to generate a synthetic promoter. See, for example, Rushton et al. (1998) Cunt. Opin. Plant Biol. 1:311-315. Alternatively, a synthetic PDR promoter sequence may comprise duplications of the upstream promoter elements found within the PDR promoter sequences.

It is recognized that the promoter sequence of the invention may be used with its native PDR coding sequences. A DNA construct comprising the PDR promoter operably linked with its native PDR gene may be used to transform any plant of interest to bring about a desired phenotypic change, such as modulating cell development, modulating root, shoot, leaf, floral, and embryo development, stress tolerance, and any other phenotype described elsewhere herein.

The promoter nucleotide sequences and methods disclosed herein are useful in regulating expression of any heterologous nucleotide sequence in a host plant in order to vary the phenotype of a plant. Various changes in phenotype are of interest including modifying the fatty acid composition in a plant, altering the amino acid content of a plant, altering a plant's pathogen defense mechanism, and the like. These results can be achieved by providing expression of heterologous products or increased expression of endogenous products in plants. Alternatively, the results can be achieved by providing for a reduction of expression of one or more endogenous products, particularly enzymes or cofactors in the plant. These changes result in a change in phenotype of the transformed plant.

Genes of interest are reflective of the commercial markets and interests of those involved in the development of the crop. Crops and markets of interest change, and as developing nations open up world markets, new crops and technologies will emerge also. In addition, as our understanding of agronomic traits and characteristics such as yield and heterosis increase, the choice of genes for transformation will change accordingly. General categories of genes of interest include, for example, those genes involved in information, such as zinc fingers, those involved in communication, such as kinases, and those involved in housekeeping, such as heat shock proteins. More specific categories of transgenes, for example, include genes encoding important traits for agronomics, insect resistance, disease resistance, herbicide resistance, sterility, grain characteristics, and commercial products. Genes of interest include, generally, those involved in oil, starch, carbohydrate, or nutrient metabolism as well as those affecting kernel size, sucrose loading, and the like.

In certain embodiments the nucleic acid sequences of the present invention can be used in combination ("stacked") with other polynucleotide sequences of interest in order to create plants with a desired phenotype. The combinations generated can include multiple copies of any one or more of the polynucleotides of interest. The polynucleotides of the present invention may be stacked with any gene or combination of genes to produce plants with a variety of desired trait combinations, including but not limited to traits desirable for animal feed such as high oil genes (e.g., U.S. Patent No. 6,232,529); balanced amino acids (*e.g.* hordothionins (U.S. Patent Nos. 5,990,389; 5,885,801; 5,885,802; and 5,703,409); barley high lysine (Williamson et al., (1987) Eur. J. Biochem. 165:99-106; and WO 98/20122); and high methionine proteins (Pedersen et al., (1986) J. Biol. Chem. 261:6279; Kirihara et al., (1988) Gene 71:359; and Musumura et al., (1989) Plant Mol. Biol. 12: 123)); increased digestibility (e.g., modified storage proteins (U.S. Application Serial No. 10/053,410, filed November 7, 2001); and thioredoxins (U.S. Application Serial No. 10/005,429, filed December 3, 2001)), the disclosures of which are herein incorporated by reference. The polynucleotides of the present invention can also be stacked with traits desirable for insect, disease or herbicide resistance (*e.g., Bacillus thuringiensis* toxic proteins (U.S. Patent Nos. 5,366,892; 5,747,450; 5,737,514; 5723,756; 5,593,881; Geiser et al., (1986) Gene 48:109); lectins (Van Damme et al., (1994) Plant Mol. Biol. 24:825); fumonisin detoxification genes (U.S. Patent No. 5,792,931); avirulence and disease resistance genes (Jones et al., (1994) Science 266:789; Martin et al., (1993) Science 262:1432; Mindrinos et al., (1994) Cell 78:1089); acetolactate synthase (ALS) mutants that lead to herbicide resistance such as the S4 and/or Hra mutations; inhibitors of glutamine synthase such as phosphinothricin or basta (e.g., bar gene); and glyphosate resistance (EPSPS gene)); and traits desirable for processing or process products such as high oil (e.g., U.S. Patent No. 6,232,529 ); modified oils (e.g., fatty acid desaturase genes (U.S. Patent No. 5,952,544; WO 94/11516)); modified starches (e.g., ADPG pyrophosphorylases (AGPase), starch synthases (SS), starch branching enzymes (SBE) and starch debranching enzymes (SDBE)); and polymers or bioplastics (e.g., U.S. patent No. 5.602,321; beta-ketothiolase, polyhydroxybutyrate synthase, and acetoacetyl-CoA reductase (Schubert et al., (1988) J. Bacteriol. 170:5837-5847) facilitate expression of polyhydroxyalkanoates (PHAs)), the disclosures of which are herein incorporated by reference. One could also combine the polynucleotides of the present invention with polynucleotides affecting agronomic traits such as male sterility (e.g., see U.S. Patent No. 5.583,210), stalk strength, flowering time, or transformation technology traits such as cell cycle regulation or gene targeting (e.g. WO 99/61619; WO 00/17364; WO 99/25821), the disclosures of which are herein incorporated by reference.

In one embodiment, sequences of interest improve plant growth and/or crop yields. For example, sequences of interest include agronomically important genes that result in improved primary or lateral root systems. Such genes include, but are not limited to, nutrient/water transporters and growth induces. Examples of such genes, include but are not limited to, maize plasma membrane H⁺-ATPase (MHA2) (Frias et al., (1996) Plant Cell 8:1533-44); AKT1, a component of the potassium uptake apparatus in *Arabidopsis,* (Spalding et al., (1999) J Gen Physiol 113:909-18); RML genes which activate cell division cycle in the root apical cells (Cheng et al. (1995) Plant Physiol 108:881); maize glutamine synthetase genes (Sukanya et al., (1994) Plant Mol Biol 26:1935-46) and hemoglobin (Duff et al., (1997) J. Biol. Chem 27:16749-16752, Arredondo-Peter et al., (1997) Plant Physiol. 115:1259-1266; Arredondo-Peter et al., (1997) Plant Physiol 114:493-500 and references sited therein). The sequence of interest may also be useful in expressing antisense nucleotide sequences of genes that that negatively affects root development.

Additionally, agronomically important traits such as oil, starch, and protein content can be genetically altered in addition to using traditional breeding methods. Modifications include increasing content of oleic acid, saturated and unsaturated oils, increasing levels of lysine and sulfur, providing essential amino acids, and also modification of starch. Hordothionin protein modifications are described in U.S. Patent Nos. 5,703,049, 5,885,801, 5,885,802, and 5,990,389, herein incorporated by reference. Another example is lysine and/or sulfur rich seed protein encoded by the soybean 2S albumin described in U.S. Patent No. 5,850,016, and the chymotrypsin inhibitor from barley, described in Williamson et al., (1987) Eur. J. Biochem. 165:99-106, the disclosures of which are herein incorporated by reference.

Derivatives of the coding sequences can be made by site-directed mutagenesis to increase the level of preselected amino acids in the encoded polypeptide. For example, the gene encoding the barley high lysine polypeptide (BHL) is derived from barley chymotrypsin inhibitor, U.S. Application Serial No. 08/740,682, filed November 1, 1996, and WO 98/20133, the disclosures of which are herein incorporated by reference. Other proteins include methionine-rich plant proteins such as from sunflower seed (Lilley et al., (1989) Proceedings of the World Congress on Vegetable Protein Utilization in Human Foods and Animal Feedstuffs, ed. Applewhite (American Oil Chemists Society, Champaign, Illinois), pp. 497-502; herein incorporated by reference); corn (Pedersen et al., (1986) J. Biol. Chem. 261:6279; Kirihara et al., (1988) Gene 71:359; both of which are herein incorporated by reference); and rice (Musumura et al., (1989) Plant Mol. Biol. 12:123, herein incorporated by reference). Other agronomically important genes encode latex, Floury 2, growth factors, seed storage factors, and transcription factors.

Insect resistance genes may encode resistance to pests that have great yield drag such as rootworm, cutworm, European Corn Borer, and the like. Such genes include, for example, *Bacillus thuringiensis* toxic protein genes (U.S. Patent Nos. 5,366,892; 5,747,450; 5,736,514; 5,723,756; 5,593,881; and Geiser et al., (1986) Gene 48:109); and the like.

Genes encoding disease resistance traits include detoxification genes, such as against fumonosin (U.S. Patent No. 5,792,931); avirulence (avr) and disease resistance (R) genes (Jones et al., (1994) Science 266:789; Martin et al., (1993) Science 262:1432; and Mindrinos et al., (1994) Cell 78:1089); and the like.

Herbicide resistance traits may include genes coding for resistance to herbicides that act to inhibit the action of acetolactate synthase (ALS), in particular the sulfonylurea-type herbicides (e.g., the acetolactate synthase (ALS) gene containing mutations leading to such resistance, in particular the S4 and/or Hra mutations), genes coding for resistance to herbicides that act to inhibit action of glutamine synthase, such as phosphinothricin or basta (e.g., the bar gene), or other such genes known in the art. The *bar* gene encodes resistance to the herbicide basta, the *nptll* gene encodes resistance to the antibiotics kanamycin and geneticin, and the ALS-gene mutants encode resistance to the herbicide chlorsulfuron.

Sterility genes can also be encoded in an expression cassette and provide an alternative to physical detasseling. Examples of genes used in such ways include male tissue-preferred genes and genes with male sterility phenotypes such as QM, described in U.S. Patent No. 5,583,210. Other genes include kinases and those encoding compounds toxic to either male or female gametophytic development.

The quality of grain is reflected in traits such as levels and types of oils, saturated and unsaturated, quality and quantity of essential amino acids, and levels of cellulose. In corn, modified hordothionin proteins are described in U.S. Patent Nos. 5,703,049, 5,885,801, 5,885,802, and 5,990,389.

Commercial traits can also be encoded on a gene or genes that could increase for example, starch for ethanol production, or provide expression of proteins. Another important commercial use of transformed plants is the production of polymers and bioplastics such as described in U.S. Patent No. 5,602,321. Genes such as β-Ketothiolase, PHBase (polyhydroxyburyrate synthase), and acetoacetyl-CoA reductase (see Schubert et al., (1988) J. Bacteriol. 170:5837-5847) facilitate expression of polyhyroxyalkanoates (PHAs).

Exogenous products include plant enzymes and products as well as those from other sources including procaryotes and other eukaryotes. Such products include enzymes, cofactors, hormones, and the like. The level of proteins, particularly modified proteins having improved amino acid distribution to improve the nutrient value of the plant, can be increased. This is achieved by the expression of such proteins having enhanced amino acid content.

This invention can be better understood by reference to the following non-limiting examples. It will be appreciated by those skilled in the art that other embodiments of the invention may be practiced without departing from the spirit and the scope of the invention as herein disclosed and claimed.

### EXAMPLES

### EXAMPLE 1: Enhancement of Multiple Agronomic Traits in ZmPDR1Transqenic Plants

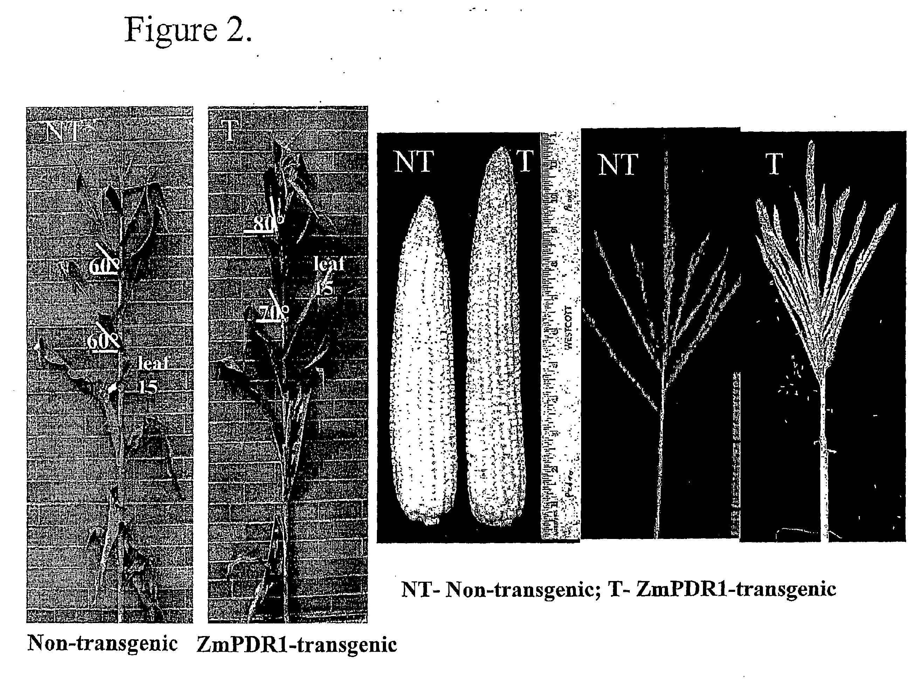

Eight maize ESTs were previously identified in the Pioneer/Dupont EST database by homology to CETS proteins (PCT patent application, publication number WO02044390). Maize ESTs p0104.cabak14rb (*ZmPDR01*) and p0118.chsaq04rb *(ZmPDR02)* were integrated into a transcriptional cassette between the Ubiquitin promoter and PINII terminator in a standard vector for Agrobacterium transformation. 25 events were generated for each construct (PHP21051, *UBI::ZmPDR01*and PHP21836, *UBI:: ZmPDR02*). In a greenhouse T0 plants exhibited extended vegetative growth, produced more and larger leaves. In addition, transgenic plants produced tassels with increased spikelet density and increased amount of pollen. The ectopic expression of *ZmPDR01*/*12* in the study demonstrated a complex phenotype with altered vegetative and reproductive characteristics. (Figure 2)

Transgenic (T1) seeds were harvested from 14 events of *ZmPDR01* (PHP21051) in a greenhouse and were planted in the field in summer 2004. Under the field conditions, transgenic plants continued to show unique characteristic differences in structure compared to non-transgenic siblings. (Figure 2) Transgenic plants showed a distinct canopy shape with upright wide leaves. They produced a tassel with high spikelet density with copious pollen shed. Transgenic plants had also elongated ears; an advantageous trait for yield enhancement.

The following traits were measured in T1 *UBI::ZmPDR01* plants at maturity: leaf number, leaf angle, leaf area, internode length, stalk strength, spikelet number and spikelet density per main tassel branch, spikelet number per ear row, and ear row numbers. The average morphometric results are shown in Table 2. Statistical analysis of all traits measured was performed with MINITAB^{®} Statistical Software, MINITAB® (Release 14.12.0). Results are shown in tables 2 - 8 below. Twelve of the 14 transgenic events showed positive changes in multiple agronomic traits in comparison with non-transgenic siblings: more and larger upright leaves, more spikelets per tassel, copious pollen, more spikelet number per ear, and stronger stalks.

**TABLE 2 Average morphometric results for T1 UBI::ZMPDR01 plants**

| Trait | Non-transgenic | Transgenic | Transgenic/NT |
|---|---|---|---|
| Leaf number | 20.9 | 22.1 | 1-2 leaves more |
| Leaf angle* | 61.7° | 72.7° | 10.4° increase |
| Leaf area, cm^{2*} | 300 | 363 | 21% increase |
| Internodes length, cm | 171.2 | 165.5 | Not significant |
| Stalk strength, by force (kg) to break | 55.5 | 71.0 | 30% increase |
| Spikelets per main tassel branch | 214 | 55.513 | 78% increase |
| Spikelets per ear row | 46 | 56 | 22% increase |
| Row number per ear | 14-16 | 16 | No differences |

| | | | |
|---|---|---|---|
| * 4 top leaves | | | |

### Ear traits

The *ZmPDR01* transgenic plants show up to a 20% increase in spikelet numbers per row. T-test confirmed that differences between the transgenic and non-transgenic spikelet number per row are statistically significant (Table 3A). The row number was not changed in transgenic ears. The estimated total number of spikelets per ear is shown in a Table 3B. The transgenic ears produced up to 24% more kernels compared to non-transgenics. Grain yield in corn is highly correlated with kernel number per ear. Therefore, the *ZmPDR01* transgene may enhance yield potential by increasing total kernel number on mature ears. The transgene-induced phenotypes would therefore show dominant gain of function trait and have a good penetrance in hybrids and increased grain yield in various backgrounds.

### TABLE 3 Statistical analysis of a spikelet number from transgenic and non-transgenic ears.

### A. Paired T-Test and Cl: Spikelets per Ear Row Non-transgenic, Transgenic

### Paired T for Non-transgenic - Transgenic

| | N | Mean | StDev | SE Mean |
|---|---|---|---|---|
| Non-transgenic | 12 | 45.0208 | 7.7221 | 2.2292 |
| Transgenic | 12 | 56.0417 | 3.1277 | 0.9029 |
| Difference | 12 | -11.0208 | 9.3319 | 2.6939 |

| | | | | |
|---|---|---|---|---|
| 95% Cl for mean difference: (-16.9501, -5.0916) T-Test of mean difference = 0 (vs not = 0): T-Value = -4.09 P-Value = 0.002 | | | | |

### B. Paired T-Test and Cl: Total Number of Spikelets per ears Non-transgenic, Transgenic

### Paired T for Estimated Non-transgenic - Transgenic

| | N | Mean | StDev | SE Mean |
|---|---|---|---|---|
| Non-transgenic | 12 | 705.104 | 143.969 | 41.560 |
| Transgenic | 12 | 876.083 | 104.092 | 30.049 |
| Difference | 12 | -170.979 | 151.040 | 43.602 |

| | | | | |
|---|---|---|---|---|
| 95% Cl for mean difference: (-266.946, -75.013) T-Test of mean difference = 0 (vs not = 0): T-Value = -3.92 P-Value = 0.002 | | | | |

### Tassel Traits

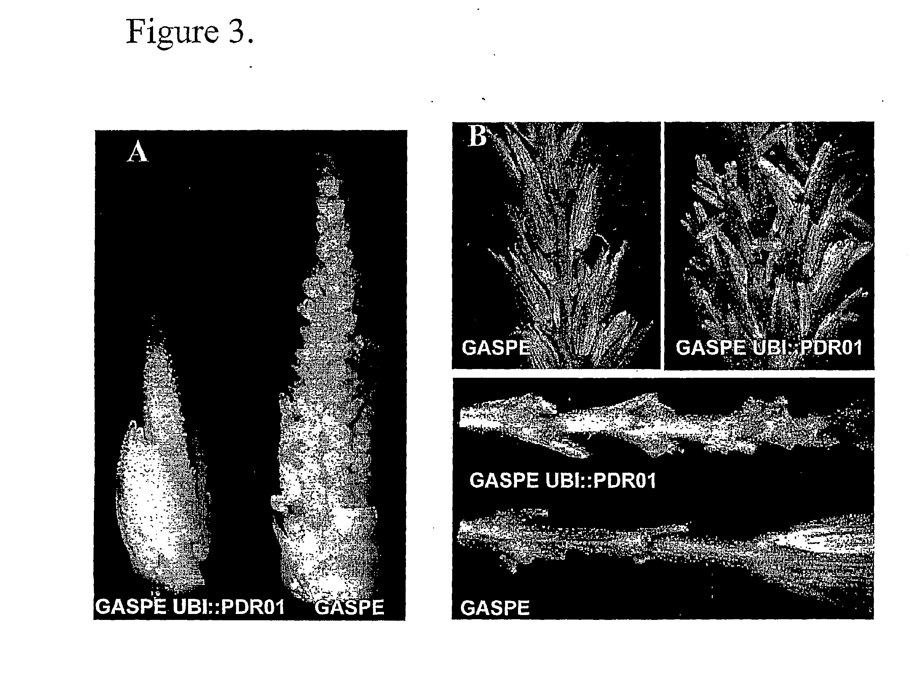

Transgenes *ZmPDR01* and *ZmPDR02,* driven by Ubiquitin promoter changed tassel morphology with respect to the number of lateral branches (Figure 3A) and spikelet density (3B). This phenotype has a strong penetrance in different transformable lines including GS3 and Gaspe flint. The number of lateral branches in Gaspe Flint background is increased at least 3 times, from 3-4 in Gaspe up to 20 in transgenics (Figure 3A). The most prominent feature of transgenic tassels is an increased spikelet density, which is most evident on the central rachis (spike). Side by side comparison (Figure 3B) of the central spikes in control Gaspe and transgenic Gaspe *UBI:: ZmPDR01* plants revealed that the distance between adjacent whorls of rachillas in transgenic Gaspe *UBI:: ZmPDR01* is nearly half that found in control plants (Figure 3B). Gaspe *UBI:: ZmPOR01* tassel inflorescence meristems produce about two times more SPM (spikelet pair meristems ) per unit length than control GASPE plants.

T-test was performed to compare the transgenic and non-transgenic spikelet number per tassels. It confirmed that differences are statistically significant (Table 4).

### TABLE 4 Statistical analysis of spikelet number from transgenic and non-transgenic tassels

### Paired T-Test and Cl: Tassel Spikelet, Tassel Spikelets per main branch

### Paired T for Tassel Spikelet NonTransgenics - Tassel Spikelets Transgenic

| | N | Mean | StDev | SE Mean |
|---|---|---|---|---|
| Tassel Spikelet | 11 | 210.364 | 45.840 | 13.821 |
| Tassel Spikelets | 11 | 351.379 | 84.151 | 25.372 |
| Difference | 11 | -141.015 | 98.156 | 29.595 |

| | | | | |
|---|---|---|---|---|
| 95% Cl for mean difference: (-206.958, -75.073) T-Test of mean difference = 0 (vs not = 0): T-Value = -4.76 P-Value = 0.001 | | | | |

### Paired T-Test and Cl: Spikelet Density, Spikelet Density per cm of length

### Paired T for Spikelet Density NonTransgenics - Spikelet Density Transgenics

| | N | Mean | StDev | SE Mean |
|---|---|---|---|---|
| Spikelet Density | 11 | 7.9491 | 1.4408 | 0.4344 |
| Spikelet Density | 11 | 11.9973 | 1.8509 | 0.5581 |
| Difference | 11 | -4.04818 | 2.19032 | 0.66041 |

| | | | | |
|---|---|---|---|---|
| 95% Cl for mean difference: (-5.51966, -2.57670) T-Test of mean difference = 0 (vs not = 0): T-Value = -6.13 P-Value = 0.000 | | | | |

Plants with a large tassel having high spikelet density and copious amount of pollen, as seen in *ZmPDR01* transgenics, will improve hybrid seed production. Many hybrid seed production fields are planted in a 4:1 row pattern in which 4 rows are planted with the seed-bearing parent (female) and 1 row is planted with the pollen-bearing parent (male). Only the female rows are harvested for seed, thus only 80% of the land area is harvested. Seed companies must pay the grower for 100% of the acres under production. A large, prolific tassel such as that produced by over-expression of the *ZmPDR10* gene would increase the percent of acres used to grow the female parent, thereby decreasing the total number of acres necessary for production. In addition, the large, highly branched tassel will release pollen over a longer period, thereby increasing the pollen shed window. This would improve seed set and reduce the risk of adventitious presence in seed production fields. Many of the best male inbreds have small tassels with a limited period of pollen shed. This requires delayed plantings or other expensive methods to extend the pollen-shed period. Moreover, many superior yielding inbred combinations are never used because male plants do not efficiently "nick" with the female. Incorporating *ZmPDR01* into male inbreds would solve this problem by producing males that shed pollen for an extended period of time.

### Leaf traits

The size, shape and number of leaves are important components of efficient light interception affecting photosynthetic capacity of the plants. The leaf area in *ZmPDR01* transgenics plants above the ear increased about 20% over non-transgenic control plants. Those leaves are responsible for fixing 70-80% of the carbohydrates that ultimately end up in the ear. The *ZmPDR01* transgenics have enhanced potential for both the source (more photosynthesis) and sink (more kernels to fill) sources as a means of increasing yield.

T-test was performed to compare the transgenic and non-transgenic leaf areas for 4 top leaves. The analysis confirmed that differences are statistically significant (Table 5).

### TABLE 5: Statistical analysis of the leaf area from 4 top leaves for transgenic and non-transgenic plants.

### Paired T- Test and Cl: Leaf Area Non-transgenic, Leaf Area Transgenic

### Paired T for Leaf Area Non-transgenic - Leaf Area Transgenic

| | N | Mean | StDev | SE Mean |
|---|---|---|---|---|
| Leaf Area Non-tr | 12 | 300.948 | 53.077 | 15.322 |
| Leaf Area Transg | 12 | 362.798 | 57.735 | 16.667 |
| Difference | 12 | -61.8508 | 83.4149 | 24.0798 |

| | | | | |
|---|---|---|---|---|
| 95% Cl for mean difference: (-114.8502, -8.8515) T-Test of mean difference = 0 (vs not = 0): T-Value = -2.57 P-Value = 0.026 | | | | |

The *ZmPDR01* transgenics also exhibited upright top leaves (Table 6). Increases in the upright leaf habit is a trait that has been positively associated with hybrid yielding ability during the many years of hybrid selection (Duvick, 1992). This trait may result from breeding for high density planting and/or from more rigid stalks that increased rigidity of leaf mid-ribs causing a more upright leaf habit (Duvick, 1992). This allows for increased density in planting crops. Also, when coupled with increased pollen shed, the yield per acre production fields would increase. A statistical T-test was performed to compare the transgenic and non-transgenic leaf angles for 4 top leaves. It confirmed that the leaf inclination differences are statistically significant (Table 6).

### TABLE 6: Statistical analysis of the leaf angles for 4 top leaves from transgenic and non-transgenic plants.

### Paired T-Test and Cl: Leaf Angle NonTransgenic, Leaf Angle Transgenic

### Paired for Leaf Angle NonTransgenic- Leaf Angle Transgenic

| | N | Mean | StDev | SEMean |
|---|---|---|---|---|
| Leaf Angle NonTr | 12 | 7.19250 | 0.46014 | 0.13283 |
| Leaf Angle Trans | 12 | 7.88167 | 0.34604 | 0.09989 |
| Difference | 12 | -0.689167 | 0.629523 | 0.181728 |

| | | | | |
|---|---|---|---|---|
| 95% Cl for mean difference: (-1.089147, -0.289187) T-Test of mean difference = 0 (vs not = 0): T-Value = -3.79 P-Value = 0.003 | | | | |

Transgenic plants also produced 1-2 leaves more than non-transgenic plants (Table 7), indicating that *ZmPDR01* delayed the meristem transition from vegetative to reproductive phase. The delay is less than that found in Arabidopsis *TFL1* expressing lines (Ratcliffe et al., 1998).

### TABLE 7: Statistical analysis of the leaf numbers from transgenic and non-transgenic plants.

### Paired T-Test and Cl: Leaf Number Non Transgenic, Leaf Number Transgenic

### Paired T for Leaf Number Non Transgenic - Leaf Number Transgenic

| | N | Mean | StDev | SE Mean |
|---|---|---|---|---|
| Leaf Number Non | 12 | 20.8650 | 1.0095 | 0.2914 |
| Leaf Number Tran | 12 | 22.1433 | 0.7640 | 0.2205 |
| Difference | 12 | -1.27833 | 1.18876 | 0.34317 |

| | | | | |
|---|---|---|---|---|
| 95% Cl for mean difference: (-2.03364, -0.52303) | | | | |

### Stalk traits

Stalk strength was tested by an Instron, model 4411 (Instron Corporation, 100 Royall Street, Canton, MA 02021), which measures force required to break stalks and also measures the stalk bend before breaking (Appenzeller et al., Cellulose, 11: 287-299, 2004). Stalks were sampled from mature plants in a field, fully dried at room temperature and measurements were taken for two internodes below the ear. Stalk diameters, flexibility and strength were measured.

**TABLE 8: Stalk strength measured by force (kg) to break a stalk**

| | Count | Mean for load(kg) | Standard deviation | Standard error |
|---|---|---|---|---|
| Transgenic | 19 | 70.971 | 21.866 | 5.016 |
| Non-transgenic | 15 | 55.513 | 15.479 | 3.997 |

**TABLE 9: Stalk flexibility measured bv displacement (mm)**

| | Count | Mean (mm) | Standard deviation | Standard error |
|---|---|---|---|---|
| Transgenic | 19 | .236 | .071 | .016 |
| Non-transgenic | 15 | .305 | .062 | .016 |

**TABLE 10: Stalk diameter (mm)**

| | Count | Mean (mm) | Standard deviation | Standard error |
|---|---|---|---|---|
| Transgenic | 19 | 22.305 | 2.606 | .598 |
| Non-transgenic | 15 | 21.707 | 2.219 | .573 |

Maximum force to break a stalk was significantly different between transgenic and non-transgenic plants (Table 8). Transgenic stalks are up to 29% stronger than non-transgenic stalks. Stalk flexibility, measured as the stalk bend before breaking, statistically was not different (Table 9).

Intrernode diameters differed slightly between transgenics and non-transgenics (Table 10) therefore, the observed strength difference may not be due to merely a bigger stalk, a thicker rind, or some dry matter composition. One explanation could be differences in mechanical properties of the transgenic stalks, which are modified as the result of increased number or strength of vascular bundles. The increased stalk strength is a valuable agronomic trait, which reduces stalk lodging under certain environmental conditions. This trait is associated with improved standability and increased harvestable yield.

Morphometric analysis of vascular bundles in internodes was performed in control Gaspe plants and transgenic GASPE UBI::ZmPDR01 plants. Cross-sections were photographed under UV illumination to visualize cellular composition of stem tissues and vascular bundles based on auto-fluorescence. The number of vascular bundles was increased on average by a factor 1.28; the area of vascular bundles was increased by a factor of 1.43; and the area of metaxylem vessels was increased by a factor of 1.96, in ZmPDR01 transgenics compared with non-transgenic GASPE siblings. The ZmPDR01 gene affects vascular bundles in multiple ways by increasing overall numbers of vascular bundles, as well as their size. The analysis of bundles and vessels show the vascular bundles thickness increasing and themetaxylem vessels have a larger diameter.

This data is consistent with the *in situ* hybridization which shows that the *ZmPDR01* gene is expressed in the vascular bundles. ZmPDR01 protein expressed under the ubiquitin promoter initiates more vascular bundles (or bigger bundles) in transgenic plants. Increased bundle strength could explain both the upright leaf habit and stronger stalks in transgenic plants. A more developed vasculature enhances a flux of nutrients across the plants and improves the overall plant vigor.

### EXAMPLE 2: Three-Dimensional Structure of ZMPDR01 and ZMPDR14 proteins suggest their function as kinase effectors/regulators.

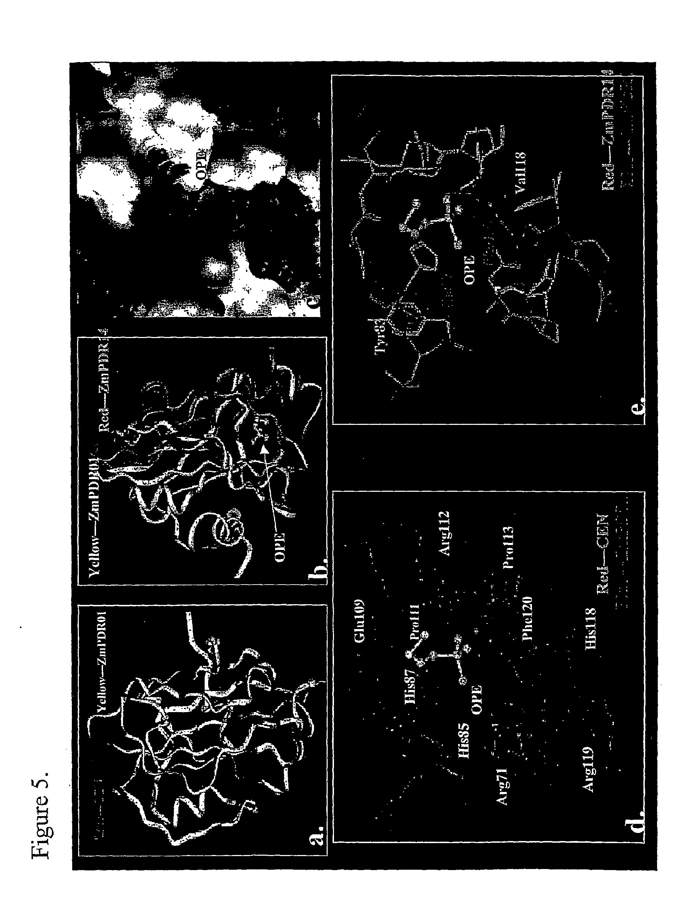

In order to predict biochemical function of maize PDR proteins, ZMPDR01 and ZMPDR14 (ESTs p0104.cabak14rb and cbn10.pk0052.f5 as described in PCT application WO02044390) were chosen for modeling. The crystallographic structure comparison demonstrated that the general fold and the anion binding-site are extremely well conserved among mammal, plant, and bacterial RKIP proteins (Banfield and Brady, 2000; Odabaei *et al*., 2004). Similar to those experimentally determined structures, the ZmPDR01 and ZMPDR14 models each have the signature fold and the strongly conserved anion recognition pocket (Figure 5a, b, d). The structural similarity at both ligand binding site and overall fold indicates the biological functions of ZmPDR01 and ZMPDR14, like other RKIP members, stem from the ability to form complexes with phosphorylated ligand, hence interfering with protein kinases and/or their effectors.

The ZmPDR01/ZMPDR14 structures were modeled with MODELERE (Sali & Blundell, 1993), an Insight II package for structural modeling. Two protein structures, PDB:1qou (Berman, *et al*., 2000) of *Antirrhinum* CENTRORADIALIS protein and PDB: 1 b7a of the phosphatidylethanolamine-binding protein from bovine, were used as templates. The template structures were first structurally aligned together and then the maize sequences were aligned to the structures with a structure-based sequence alignment tool, in which the structural information was mainly captured in the position specific substitution score matrix and gap-penalty. The atom coordinates of modeling protein were assigned based on template, and subsequently underwent an energy minimization procedure to remove the bad contacts. The overall structures of ZMPDR011ZMPDR14 are similar to the folding of other the RKIP members characterized as two anti-parallel β-sheets and long stranded-connecting loops (Figure 5b). Superposition of either ZMPDR01 or ZmPDR14 to template structures gives an r.m.s.d (root mean square of deviation) of < 1 Å.

The most striking feature among the RKIP family is the high conservation of the ligand binding-site. Extensive mutagenesis data especially from the mammalian and yeast proteins showed that the binding site and its surrounding region are crucial for protein activities. To identify the binding site in the modeled structures, a ligand surrogate OPE was mapped, phosphoric acid mono-(2-aminoethyl), into the structural frame based on the overall structural alignment between the bovine RKIP complexed with OPE and ZmPDR1/ZMPDR14. After transformation, OPE fell in a well-defined binding pocket of modeled structures and its phosphate group formed an extensive hydrogen-bond network with the recognition residues (Figure 5b, c, d, e). The comparison between CEN and ZmPDR01 revealed the near consensus of the major residues contributing the construction of the binding sites, including Asn71, His85, His87, Glu109, Pro111, Arg112, Pro113, His118, and Phe120 as in ZmPDR01 (Figure 5d). However, the ZMPDR's anion recognition site is slightly different from that of ZmPDR01 or CEN. A large hydrophobic residue, Phe120 of ZMPDR14, is replaced with a smaller hydrophobic Val in ZMPDR14, and in an offset, the ZMPDR14 uses a large aromatic Tyr83 to substitute His83 of ZmPDR01 on another side of the binding-site. As a consequence, ZMPDR14 and ZM PDR01 have a ligand binding-pocket of equivalent size. A similar variation was also observed in the Arabidopsis proteins, atTFL1/atFT (data not shown). This binding-site variation may be associated with the antagonistic effects of TFL1 and FT. Analysis of the geometric and electric property of putative anion binding site in ZmPDR01 (Figure 5c) was performed. The binding site topology indicates it is able to well accommodate phosphorylated ligand. The Arg and a few His residues (possibly in protonated state upon phosphate group binding) may play a key role for ligand recognition.

### EXAMPLE 3: Identification of the CETS (PDR) gene family in maize, rice, Arabidopsis and sorghum

The identification of genes for this gene family was focused upon sequences from the following plant species: *Zea mays* (maize), *Oryza sativa* (rice), *Hordeum vulgare* (barley), *Sorghum bicolor* (sorghum), *Triticum aestivum* (wheat), *Allium cepa* (onion), *Arabidopsis thaliana, Glycine max* (soybean), and *Helianthus ssp.* (sunflower species). Related sequences were found from all but barley. The identification of genes relied upon searches of available genomic and/or cDNA sequences for these species. The sequence sets searched were both public and private (Dupont/Pioneer proprietary) sequences. A local implementation of NCBI Blast version 2.0 was used for the sequence searching. The initial starting protein sequence queries were the six publicly known Arabidopsis prototypes of this gene family, At_TFL1 (At5g03840), At_CEN (At2g27550), At_BFT (At5g62040), At_FT (At1g65480), At_TSF (At4g20370), and At_MFT (At1g18100). No other members of this gene family were found in Arabidopsis.

For maize the proprietary ESTs, EST assemblies, and genomic sequences, plus the public GSS and EST and other maize NCBI sequences were searched. All potential hits to conserved regions of the gene family were assembled and curated, and additional rounds of searching were done to extend the genomic and/or transcript sequences across the fullest possible coding region, but also across UTR and intron features of the genes. Successive rounds of back searching were done using the nucleotide and translation sequences until an exhaustive account of the maize gene family sequences was obtained. All gene and transcript sequences were curated to identify start and stop codons, intron-exon boundaries, UTRs, and the summary protein translation.

The approach for rice relied chiefly upon searching a combination of mostly public genome assemblies and cDNA contigs, with some proprietary cDNA supplemental information. The main genomic assemblies were the NCBI genomic contigs, but the BGI (Beijing Genomics Institute) dataset was also searched. The public rice sequence annotations were sometimes wrong, and improved ORF and translation determinations were made where needed. The approach for sorghum was similar, but relied upon the recently publicly released Sorghum GSS sequences. The GSS sequences overlapping this gene family were assembled and annotated for ORF and translation products. The barley, wheat, soybean, and Helianthus searches chiefly relied upon a dual search of proprietary ESTs and public cDNAs/ESTs. For onion, there was a large body of ESTs deposited in Genbank. They were retrieved and searched locally as a captive set. Any hits were assembled and annotated.

The resulting gene count from the various species, ignoring the known six from Arabidopsis, is as follows: Zea mays - 28, Oryza sativa - 21, Sorghum bicolor - 24, Triticum aestivum - 2, Allium cepa - 1, Glycine max - 7, Helianthus sp. - 3, for a total gene count of 86.

### EXAMPLE 4: Phylogenetic analysis of the maize PDR gene families and their tissue specific expression.

In the Arabidopsis genome, there are six genes comprising a *CETS* family including *FT* (*flowering locus T*) and *TFL1* (terminal *flower 1*) (Kardailsky *et al*., 1999; Kobayashi *et al.,* 1999), *ATC (Arabidopsis thaliana CENTRORADIALIS homologue)* (Mimida *et al*., 2001), *BFT (Brother of FT andTFL 1*), *MFT (Mother of FT andTFL1*), *TSF (Twin sister of FT*) (Kobayashi *et al.,* 1999). *FT* and *1TFL1* genes are the important regulators of flowering time with antagonistic action. The *FT* is an activator, whereas *TFL1*is a repressor of flowering (Kardailsky *et al*., 1999; Kobayashi *et al*., 1999). Constitutive expression of *FT* in transgenic Arabidopsis plants causes early flowering, and constitutive expression of *TFL1* causes late flowering. Other members of this gene family have been classified by their effect on flowering time. Over expression of *MFT* and *TSF* led to early flowering and over expression of *ACT* led to late flowering. No data are available for *BFT* (Yoo *et al*., 2004). However, the loss-of function mutants of *ATC* and *MFT* showed no obvious phenotypes indicating that these two genes rather have a role that is different from regulation of flowering time (Mimida *et al*., 2001; Yoo *et al*., 2004). No functions were assigned to them.

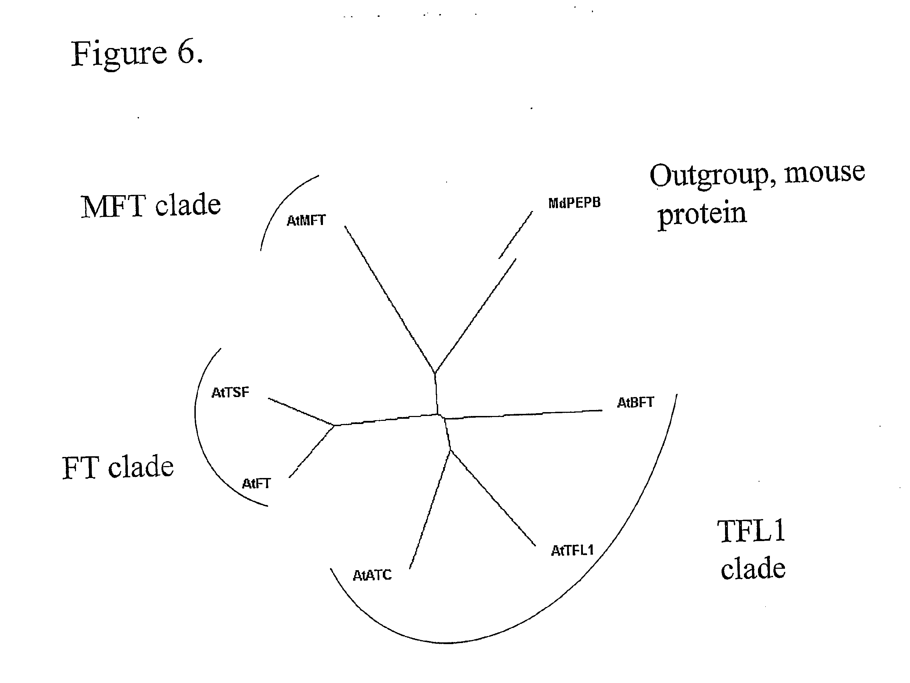

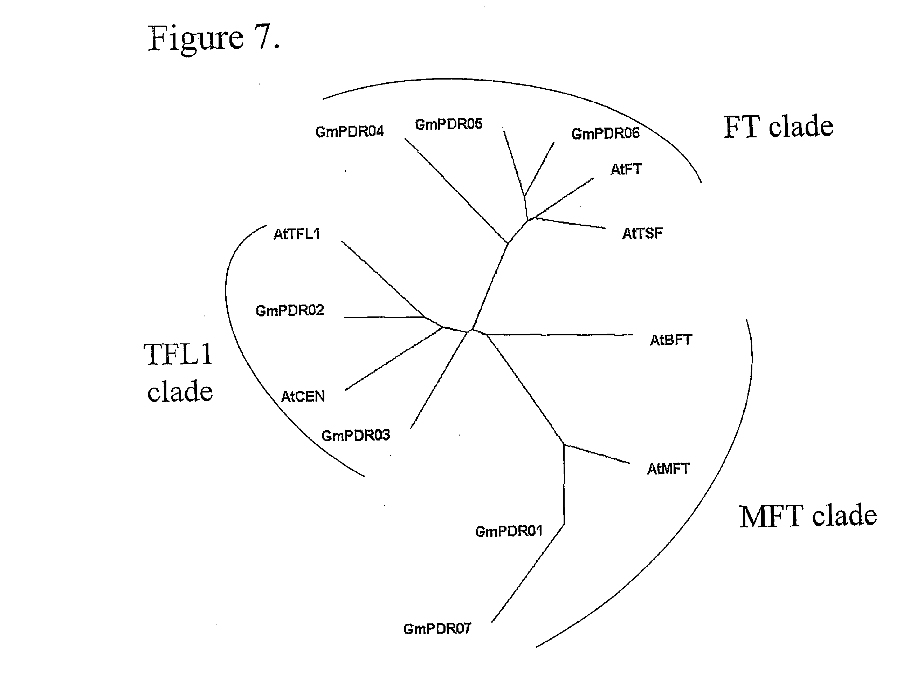

A phylogenetic tree constructed by neighbor-joining method (PAUP program), for Arabidopsis proteins including the mouse PEPB protein as an outgroup, delineated three clades which were named according their founders: the FT clade, the TFL1 clade, and the MFT clade (Figure 6). Extensive search of the soybean *(Glycin max)* EST database revealed seven *PDR* genes. The putative soybean proteins are grouped into the three clades on the phylogenetic tree similar to Arabidopsis (Figure 7).

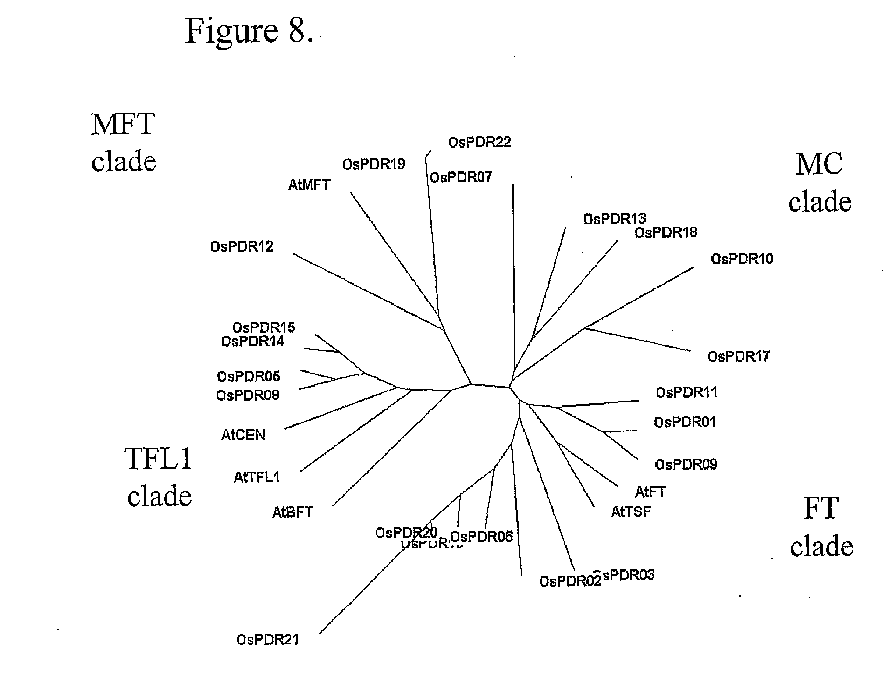

The rice genome contains 22 PDR genes. A phylogenetic tree of rice CETS proteins revealed four clades including three clades described for dicots (FT, TFL1, MST) and a new clade, which was named "the MC (monocot) clade" (Figure 8). Thus, the *PDR* gene family is larger and more complex in monocots than in dicots.

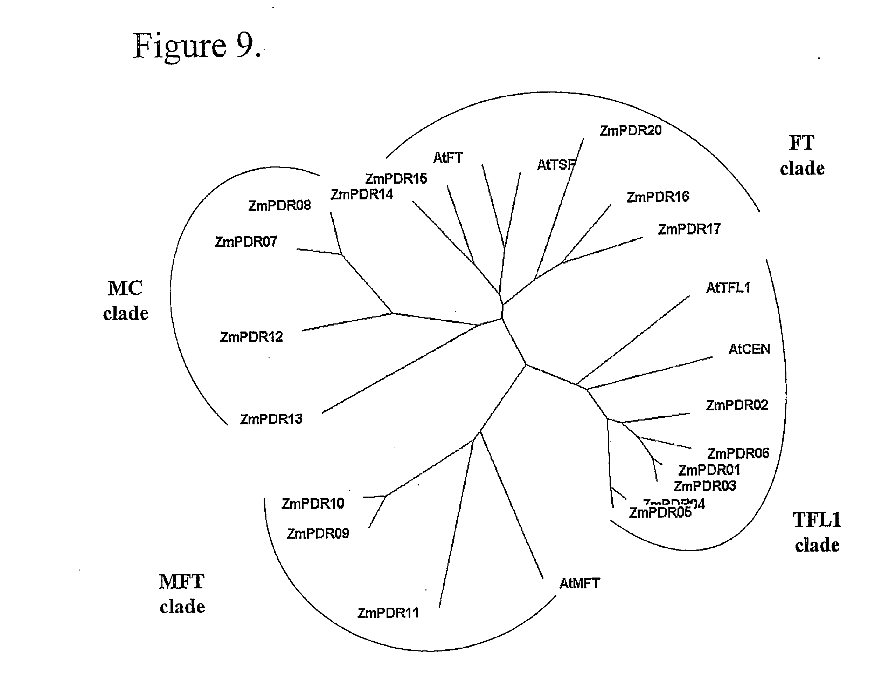

A Pioneer proprietary EST database, public Genomic Survey Sequences (GSS) and Maize assembled genomic sequences (TIGR and ISU-MAGl) were extensively searched, and 33 maize PDR genes were identified. Eighteen of the identified sequences are represented by their corresponding full-length proteins. Partial gene sequences are available for the other members. These 18 complete versions were chosen for the phylogenetic analysis. There are four clades of the CETS proteins in maize, as was the case for rice (Figure 9). It appears that monocots have the additional clade of the *CETS* genes (the MC clade) that is not found in dicots.

To predict function of maize *PDR* genes, we searched the RNA expression profiling MPSS (Massively Parallel Signature Sequencing) (Brenner *et al.,* 2000), proprietary database that represents more than 200 tissue samples under both normal and stressed conditions. MPSS technology generates 17-mer sequence tags that are unique identifiers of the cDNAs (Brenner *et al.,* 2000). The MPSS expression profiling revealed that genes from different clades showed tissue-specific patterns of expression, suggesting specific functions for each clade.

The maize `TFL1' clade is composed of 6 genes, which are closely related. Coding regions of ZmPDR01, ZmPDR03 and ZmPDR06 shared 85% homology at nucleotide sequence level, while introns shared 55% homology

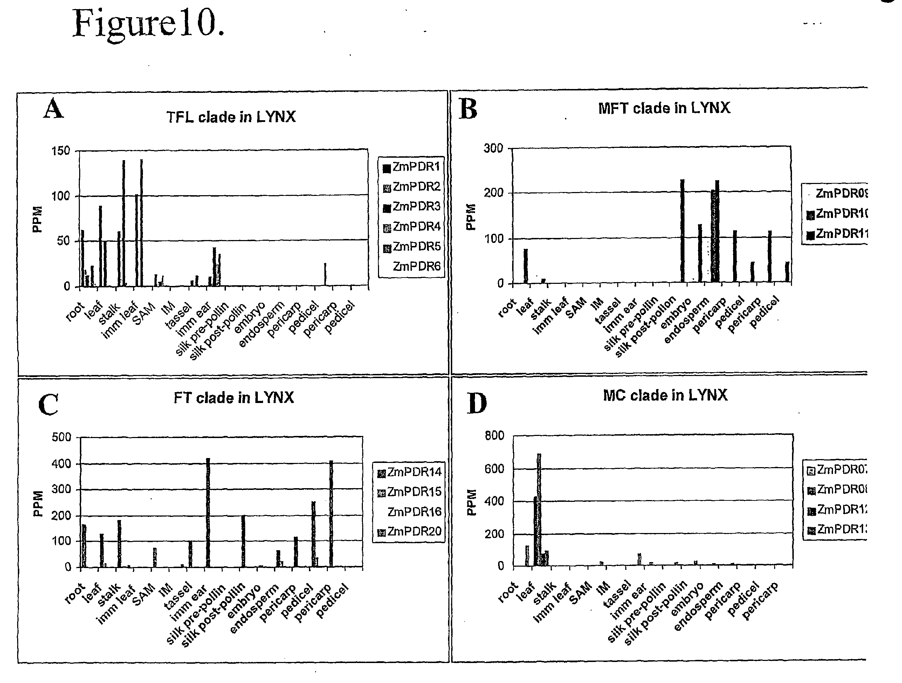

Coding regions of *ZmPDR04,* and *PDR05* shared 75% homology, but the introns showed only 28% homology. *ZMPDR01, ZmPDR02, ZmPDR04, ZmPDR05* and *ZmPDR06* are mapped to chromosomes 3, 4, 2 10 and 4, respectively. According MPSS profiling, *ZmPDR02* gene is expressed at low level in roots, stalks, immature ears, and silk. *ZmPDR04*/*05* showed expression predominantly in reproductive tissues. *ZmPDR04* is expressed in tassel, immature ears and pedicel, which is a maternal tissue connecting kernels with cob. *ZmPDR05* MPSS tags were only in the ear tips (Figure 10A).

The 'MFT' clade is composed of 3 genes *ZmPDR09, ZmPDR10, and ZmPDR11,* which are expressed mostly in kernels. *ZmPDR09* and *ZmPDR10* are highly related sharing 94% homology within the coding sequences and 50% homology within introns. *ZmPDR09, ZmPDR10* and *ZmPDR11* are mapped to chromosomes 8, 3 and 6, respectively. Figure 10B contains associated expression data. *ZmPDR09* is expressed in the embryo and the endosperm. *ZmPDR10* is more abundant in the aleurone layer. *ZmPDR11* has shown abundant expression in the embryo, endosperm and silk after pollination. A low level of expression may be detected in roots, leaves and tassel. Manipulation of the *ZmPDR09, ZmPDR10, and ZmPDR11* genes may result in modification of kernel traits in transgenic plants.

The 'FT' clade is composed of 4 genes *ZmPDR14, ZmPDR15, ZmPDR16* and *ZmPDRC06.* According to MPSS profiling, the *ZmPDR14* gene is expressed in both vegetative and reproductive tissues (Figure 10C). MPSS tags for *ZmPDR15, ZmPDR16* and *ZmFTC06* are detectable at low level in similar tissues. *ZmPDR14, ZmPDR15* and *ZmPDR16* are mapped to chromosomes 8, 6 and 5, respectively.

The 'MC' clade is specific to monocots. It is composed of 4 genes: *ZmPDR12, ZmPDR07, ZmPDR13,* and *ZmPDR08. ZmPDR12* is mapped to chromosome 3. Each of the genes are expressed preferentially in leaves (Figure 10D). *ZmPDR07* and *ZmPDR08* are duplicated genes, sharing 85% homology within the coding regions and 63% within introns. Transcription levels of these two genes are responsive to water availability. The level of their expression is 20 times higher in leaves under well-watered conditions than under the drought stress. Manipulation of the *ZmPDR07* and *ZmPDR08* in transgenics may result in modification of leaf traits and drought tolerance.

### EXAMPLE 5: RNA in situ hybridization of the maize PDR genes from the 'TFL1' Phylogenetic clade

MPSS expression profiling provided the tissue-specific pattern of expression of the *PDR* genes. Each tissue or organ is composed of many different cell types with specific functions. Gene expression is identified at the cellular level within a target organ with the help of *in situ* hybridization. The analysis provides the next level of cell-specific expression profiling on cellular level for prediction of possible gene function. Sense and anti-sense RNA were labeled with isotope S³⁵ using T3 or T7 RNA polymerases according to the manufacturer's protocols (Promega). Sense probes were used as controls, which indicated the background level while anti-sense probes produced true hybridization signals.

### ZmPDR01 gene is expressed in vascular bundles of developing leaves and stem.

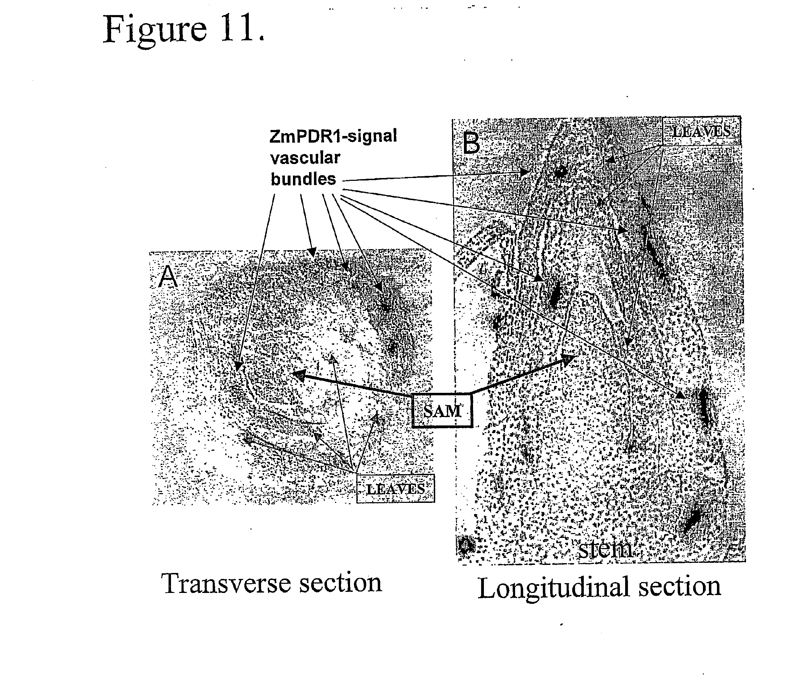

*ZmPDR01* anti-sense RNA showed a strong signal in vascular bundles. The hybridization signal was found in primordial provascular cells as well as in the cells which surround mature vascular bundles with differentiated phloem and xylem (Figure 11). On transverse sections the immature leaves appeared as concentric circles, which are wrapped around SAM (shoot apical meristem) (Figure 11A). The hybridization signal is concentrated around vascular bundles in a form of isolated islands on the transverse sections (Figure 11A), while on the longitudinal sections the hybridization signals concentrated in a form of elongated islands around vascular bundles (Figure 11B). At this stage the leaves are growing in a spiral mode wrapping the SAM.

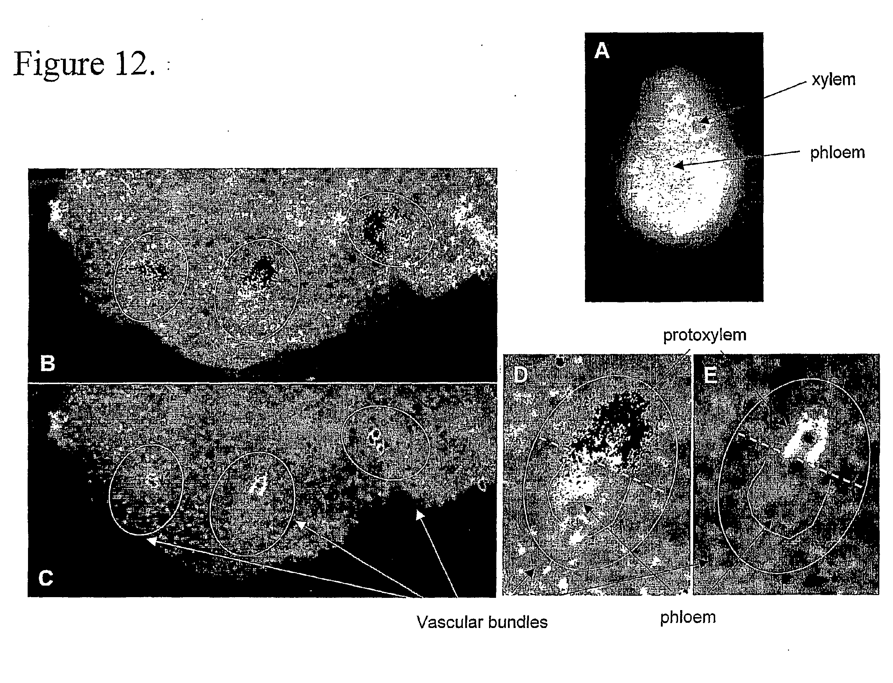

Vascular bundles appear as bright spots under UV illumination due to bright fluorescence of secondary cell walls in xylem (Figure 12A). Under higher magnification, strong signal can be seen in the primordial vascular bundle cells (in cambial cells) in young leaf (Figure 12B, C). Hybridization signal can be detected in vascular bundles with well developed xylem vessels, mostly from the adjacent cells which are presumably still in the process of differentiation into xylem, protoxylem and tracheids (figure12D, E). No obvious signal was detected from the phloem and companion cells. These observations show that *ZmPDR01* could be involved in the control of provascular cell identity and on later stages for protoxylem cell identity. *ZmPDR03* and *ZmPDR6* belong to the same sub-branch on the phylogenetic tree as *ZmPDR01* (Figure 9). The genes showed a similar pattern of expression (Figure 10A) suggesting similarity in function. The expression pattern for *ZmPDR01, ZmPDR03* and *ZmPDR6* is predicted to be associated with vascular bundles as well.

### ZmPDR02, ZmPDR04, ZmPDR05 genes are expressed in vascular bundles of developing ears.

*ZmPDR02, ZmPDR04* and *ZmPDR05* are members of the sub-group on the TFL1 clade which are expressed in immature ears (Figure 9, 10). RNA *in situ* hybridization showed strong signal from the *ZmPDR02*/*04*/*05* anti-sense RNAs in components of vascular bundies on the longitudinal sections of immature ears (Figure 13) as well as some specific differences. No apparent hybridization signals were found in the upper cell layers of the inflorescence meristem, spikelet pairs, or in spikelet meristems for the ZmPDR02/04/05 probes.

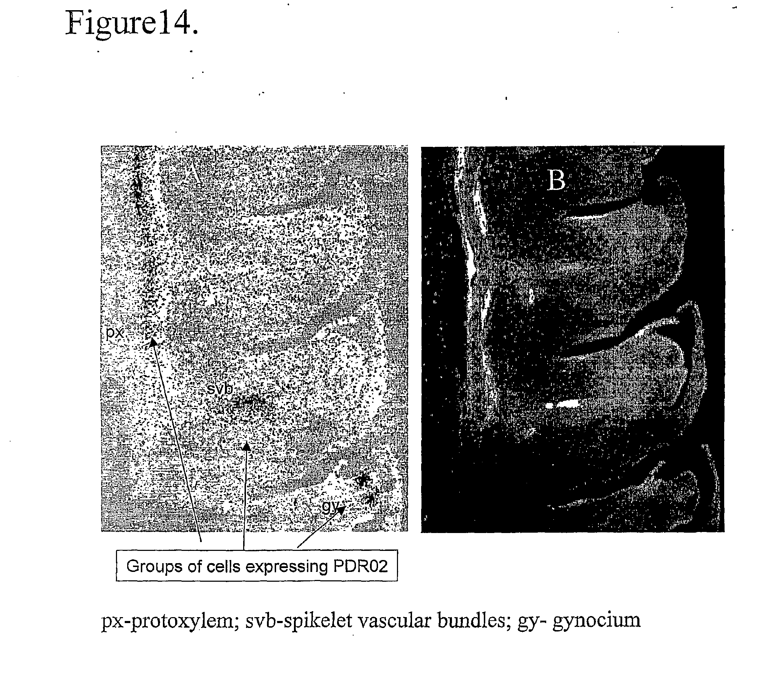

The *ZmPDR02* expression initially becomes evident in groups of cells underlying the foundation of each late spikelet pair meristem and each spikelet approximately two to four cell layers within the developing inflorescence stem. These cells have no specific morphological features at that stage except their location (Figure 13A). The older spikelets below the tenth to twelfth spikelet from the top of the inflorescence have no cells with detectable expression of the *ZmPDR02.* At the level of 15^{th} to 20^{th} spikelets from the top, expression of *ZmPDR02* can be detected within the vascular bundle cells, which are located on inner side of the vascular bundles (Figure 14A) closer to the axis of the inflorescence stem. These are most likely protoxylem cells. The *ZmPDR02* expression can be also seen in vascular bundles within spikelet stem (rachis) (Figure 14A). In addition the expression of *ZmPDR02* can be found in various components of female upper and lower florets (Figure 13A) such as stamens or at the basement of gynocium. These groups of cells have no specific morphology at that stage of development. *ZmPDR02* would be actively transcribed in protovascular cells as well as in proto-xylem or tracheids.ZmPDR04 showed the simplest pattern of expression, which is evident in cells of vascular bundles at the level of SPM (spikelet pair meristems) and below where the vascular bundles develop visible protoxylem and xylem (Figure 13B).

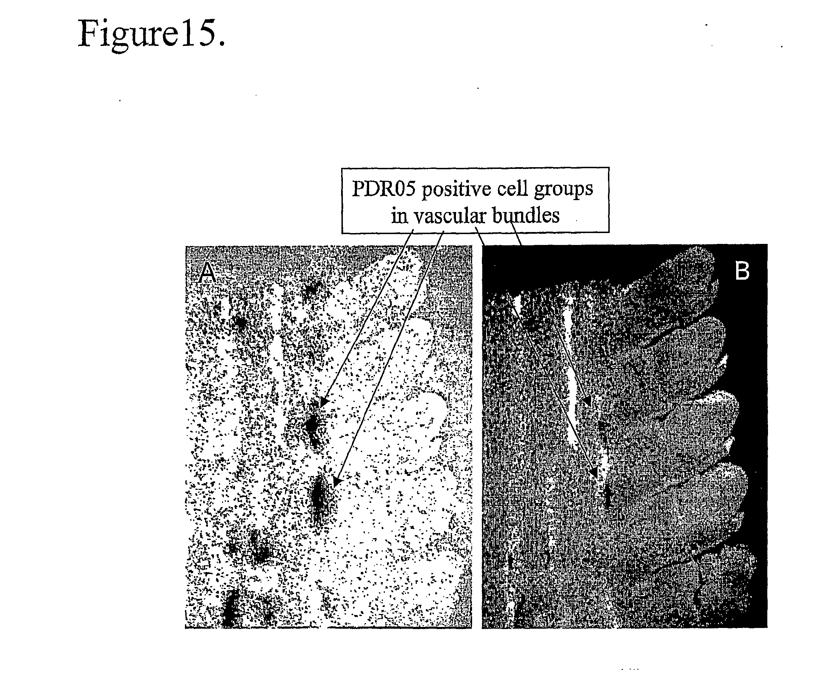

RNA *in situ* hybridization found strong signal from the *ZmPDR05* anti-sense RNA in various groups of cell in the longitudinal sections of the immature ear (Figure 13C). *ZmPDR05* expression is evident in groups of cells underlying inflorescence meristem (Figure 13C). The primary clusters are small and composed of 2 to 4 labeled cells, which are located inside the inflorescence meristem (IM) below the 6th or 8th layers of cells from the top surface. At the level of primary spikelet pair meristems the groups of labeled cells are located inside the growing ear approximately ¼ of the inflorescence stem diameter from the surface. The distribution of labeled cells has a characteristic segmental pattern with clusters of labeled cells at the base of each spikelet meristem. These cells have no specific morphological features except their location at this stage of development (Figure 13C). At the lower part of the ear inflorescence *ZmPDR05* expressing cells are located on the outer side of vascular bundles, which underly each spikelet (Figure 15A). This gene is expressed in phloem cells. In some places the expression of *ZmPDR05* can be traced to the individual cells apparent from the cross sectioning of the conducting cells. Comparative analysis of *ZmPDR02, ZmPDR04* and *ZmPDR05* expression in the developing ear showed that each of the three genes is expressed in specific groups of cells within a vascular bundle (Figure 13, 14, 15). The *ZmPDR05* is the first gene of this group to be expressed in progenitor cells produced by inflorescence meristem. The expression of *ZmPDR05* as well as *ZmPDR02* is induced in rhythmic fashion in small groups of cells if not in individual cells, which are entering the process of differentiation into the spikelet pair meristem. These genes are apparently activated in several other groups of cells in the developing florets. The position of the *ZmPDR02* and *ZmPDR05* expressing cells coincides with the position of the future bundle vessel plexuses between major vessel bundles and spikelet vessel bundles.

These observations are consistent with various studies that revealed a complex hierarchal organization of ear vascular system. The system is composed of multiple vessel bundles, which are interconnected in multiple plexuses underlying each spikelet. Major vessel bundles are running through the main stem of the cob each of which forms plexuses with the vessel bundles of each spikelet, which are in turn are branched into multiple micro bundles supplying water and nutrients to the developing kernel and various parts of the florets (Cheng, 1995).

### EXAMPLE 6: Vascular bundle specific promoters of ZmPDR01, 02, 03. 04, and 05 genes

The *ZmPDR01, ZmPDR02, ZmPDR04, ZmPDR05* genes are expressed within different zones of the vascular bundles as has been shown by *in situ* hybridization (Figures 11-15). These genes are the source of the vascular specific promoters used to target expression in the particular type cells of the vascular bundles.

*ZmPDR01* is expressed in vascular bundles of vegetative tissues (leaves and stems) with well developed xylem vessels, especially in the adjacent cells which are presumably still in the process of differentiation into xylem, protoxylem and tracheids. Thus its promoter may be used for gene expression in protoxylem.

*ZmPDR02* is actively transcribed in protovascular cells as well as in protoxylem or tracheids of developing ears. *ZmPDR04* is evident in cells of vascular bundles at the level of SPM (spikelet pair meristems) and below where the vascular bundles develop visible protoxylem and xylem. Both promoters may be used for gene expression in the ear specific protoxylem. *ZmPDR05* is apparently expressed in phloem cells of the developing ear and its promoter may be used for specific expression in phloem.

### EXAMPLE 7: Promoter optimization for maize PDR gene expression

Manipulation of the expression of different members of the *PDR* gene family in transgenic maize has been performed. Constitutive expression of *ZmPDR01* gene in maize resulted in overall enhanced growth effects in maize plants (see EXAMPLE 1). In order to create a desirable phenotype in a particular organ/tissue and optimize gene expression in tissue/organ specific manner, the *ZmPDR01* gene was linked to a number of tissue/organ-specific promoters. To modify ear traits, *ZmPDR01* was linked to the TB1 promoter, which is expressed in axillary branches (Doebley *et al.,* 1997; Hubbard *et al.,* 2002), to generate PHP24948. The *in situ* experiments show that *ZmPDR01* is expressed in vascular bundles and could be used to modify stalk traits. The S2A promoter, which is expressed in intervascular cambium around vascular bundles and inside vascular bundles in young stem (Abrahams *et al*., 1995) was employed to drive *ZmPDR01* in PHP24945. The expression of *ZmPDR01* in a root-specific manner was driven with the NAS2 promoter (Mizuno *et al.,* 2003) in PHP24943. *ZmFTM1* and *ZmFTM3* promoters, which are predominantly expressed in meristematic tissues, also manipulated the expression of *ZmPDR1* in the PHP24951 and PHP24952, respectively, to enhance ear and tassel sizes and avoid late flowering phenotype. The targeting of the tassel traits in transgenic maize, was accomplished using anther and pollen specific promoter SGB6 (U.S. patent numbers 5,470,359 and 5,837,850, Huffman) with both PDR overexpression and RNAi vectors designated as PHP24949 and PHP24950 respectively.

### EXAMPLE 8: Application of PDR gene for yield enhancement in soybeans and small grain crops.

High spikelet density induced by over expression of *ZmPDR1* gene is particularly valuable for crops with perfect flowers (male and female florets formed on the same spikelets) such as rice, wheat, sorghum, barley, rye that have spikes (head) equivalent to maize tassels. Over expression of *PDR* genes could increase the spikelet number per spike and grain yield of transgenic plants. The *PDR* genes ectopically expressed in transgenic soybean plants would be expected to increase the overall biomass, and the number of pods, that leading to higher yielding soybean varieties.

### EXAMPLE 9: Enhanced Agronomic Traits in ZmPDR03, ZmPDR04 and ZmPDR05 Transgenic Plants

Transgenic plants were produced for *ZmPDR03, ZmPDR04* and *ZmPDR05* genes that are closely related to *ZmPDR01* and *ZmPDR02.* Corresponding cDNAs were integrated into a transcriptional cassette between the Ubiquitin promoter and PINII terminator in a standard vector for Agrobacterium transformation. Twenty-five events were generated for each construct (PHP23176, *UBI::ZmPDR03,* PHP25992, *UBI::ZmPDR04;* and PHP26034 *UBI::ZmPDR05)).* Greenhouse-raised T0 plants exhibited an extended period of vegetative growth, produced more and larger leaves, and thicker stalks. Transgenic plants also produced tassels having an increased spikelet density and an increased amount of pollen. The *ZmPDR03*/*04*/*05* transgenics had phenotypic expression similar to that of *ZmPDR01* and *ZmPDR02* transgenic plants (see Example 1). This indicates that ZmPDR genes from the same phylogenetic clade are able to create similar transgenic traits in plants (see Figure 9).

All publications and patent applications in this specification are indicative of the level of ordinary skill in the art to which this invention pertains. All publications and patent applications are herein incorporated by reference to the same extent as if each individual publication or patent application was specifically and individually indicated by reference.

The invention has been described with reference to various specific and preferred embodiments and techniques. However, it should be understood that many variations and modifications may be made while remaining within the spirit and scope of the invention.

### FURTHER EMBODIMENTS OF THE INVENTION

1. A method for plant characteristics, the method comprising:
   a. introducing into a plant cell a recombinant expression cassette comprising a polynucleotide whose expression, alone or in combination with additional polynucleotides, functions as a plant developmental regulator polypeptide within the plant;
   b. culturing the plant cell under plant forming conditions to produce a plant; and,
   c. inducing expression of the polynucleotide for a time sufficient to alter the architecture of the plant.
2. The method of item 1 wherein the plant is a monocot.
3. The method of item 1 wherein the plant is a dicot.
4. The method of item 1 wherein the plant is maize, barley, wheat, rice, rye, oats, millet, sorghum, soybean, canola, or sunflower.
5. The method of item 2 wherein the plant is maize.
6. The method of item 1 wherein the polynucleotide is linked to a promoter.
7. The method of item 1 wherein the polynucleotide is selected from the group consisting of: SEQ ID NOS: 1, 3, 5, 7, 9, 11, 13, 15, 17, 19, 21, 23, 25, 27, 29, 31, 33, 35, 37, 39, 41, 43, 45, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183.
8. A transgenic plant produced by the method of item 1.
9. A transgenic seed produced by the transgenic plant of item 8.
10. The method of item 1, where expression of plant developmental regulator polypeptide within the plant is increased.
11. The method of item 1, where expression of plant developmental regulatory polypeptide within the plant is decreased.
12. The method of item 10, where said plant has increased kernel number per ear.
13. The method of item 10, where said plant has an increased tassel spikelet density.
14. The method of item 10, wherein said plant has an increased tassel branch number.
15. The method of item 10, where said plant has increased pollen production.
16. The method of item 10, where said plant has improved canopy shape.
17. The method of item 10, where said plant has increased photosynthetic capacity in leaf tissue.
18. The method of item 10, where said plant has improved stalk strength.
19. The method of item 10, where said plant has improved plant standability.
20. The method of item 10, where said plant has altered vascular bundle structure or number.
21. The method of item 10, where said plant has increased root biomass.
22. The method of item 10, where said plant has enhanced root growth.
23. The method of item 10, where said plant has modulated shoot development.
24. The method of item 10, where said plant has modulated leaf development.
25. The method of item 11, where said plant has shorter plant internodes.
26. The method of item 11, where said plant has stunted growth.
27. The method of item 11, where said plant is a dwarf plant.
28. A method for increasing plant harvestable yield, the method comprising:
   a. introducing into a plant cell a recombinant expression cassette comprising a polynucleotide whose expression, alone or in combination with additional polynucleotides, functions as a plant developmental regulator polypeptide within the plant;
   b. culturing the plant cell under plant forming conditions to produce a plant; and
   c. inducing expression of the polynucleotide for a time sufficient to increase the harvestable yield of the plant.
29. The method of item 28 wherein the plant is a monocot.
30. The method of item 28 wherein the plant is a dicot.
31. The method of item 28 wherein the plant is maize, barley, wheat, rice, rye, oats, millet, sorghum, canola, sunflower and soybean.
32. The method of item 29 wherein the plant is maize.
33. The method of item 28 wherein the polynucleotide is linked to a promoter.
34. The method of item 28 wherein the polynucleotide is selected from the group consisting of: SEQ ID NOS: 1, 3, 5, 7, 9, 11, 13, 15, 17, 19, 21, 23, 25, 27, 29, 31, 33, 35, 37, 39, 41, 43, 45, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183.
35. A transgenic plant of the method of item 28.
36. A transgenic seed of the transgenic plant of item 35.
37. An isolated nucleic acid comprising a polynucleotide sequence of the PDR coding region or a complement thereof.
38. An isolated nucleic acid wherein the polynucleotide has at least 75% sequence identity to the PDR coding region as determined by GAP 10 analysis using default parameters over the entire length of the sequence, or a complement thereof, wherein expression of the polynucleotide modulates the level of PDR expression in a plant.
39. An isolated nucleic acid wherein the polynucleotide hybridizes under high stringency conditions to the PDR coding region, or a complement thereof, wherein expression of the polynucleotide modulates the level of PDR expression in a plant.
40. An isolated nucleic acid wherein the polynucleotide comprises the PDR coding region, or a variant thereof, wherein the expression of the variant modulates the level of PDR expression in a plant.
41. An isolated nucleic acid wherein the polynucleotide comprises a fragment of the PDR coding region.
42. The isolated nucleic acid fragment of item 41 wherein the fragment is a functional fragment.
43. An expression cassette comprising the nucleic acid of item 37 operably linked to a promoter, wherein the nucleic acid is in sense or antisense orientation.
44. A non-human host cell stably transformed with the expression cassette of item 43.
45. The host cell of item 44 that is a plant cell.
46. The host cell of item 44 that is a bacterial cell.
47. A plant stably transformed with the expression cassette of item 43.
48. An isolated nucleic acid comprising a polynucleotide sequence of the PDR coding region, or a complement thereof.
49. An isolated polynucleotide selected from the group consisting of:
   a. a polynucleotide having at least 80% sequence identity, as determined by the GAP algorithm under default parameters, to the full length sequence of a polynucleotide selected from the group consisting of SEQ ID NOS: 51, 89, 91, 97, 119, 127, 139, 147, 165 and 171; wherein the polynucleotide encodes a polypeptide that has PDR functions; and
   b. a polynucleotide encoding a polypeptide selected from the group consisting of SEQ ID NO: 50, 90, 92, 98, 120, 128, 140, 148, 166, and 170; and
   c. a polynucleotide selected from the group consisting of SEQ ID NOS: 51, 89, 91, 97, 119, 127, 139, 147, 165 and 171; and
   d. a polynucleotide which is complementary to the polynucleotide of (a), (b), or (c).
50. A recombinant expression cassette, comprising the polynucleotide of Item 43, wherein the polynucleotide is operably linked, in sense or anti-sense orientation, to a promoter.
51. A host cell comprising the expression cassette of item 50.
52. A transgenic plant comprising the recombinant expression cassette of item 50.
53. The transgenic plant of item 52, wherein said plant is a monocot.
54. The transgenic plant of item 52, wherein said plant is a dicot.
55. The transgenic plant of item 52, wherein said plant is selected from the group consisting of: maize, soybean, sunflower, sorghum, canola, wheat, alfalfa, cotton, rice, barley, millet, peanut and cocoa.
56. A transgenic seed from the transgenic plant of item 52.
57. A method of increasing yield in plants, comprising:
   a. introducing into a plant cell a recombinant expression cassette comprising the polynucleotide of item operably linked to a promoter; and
   b. culturing the plant under plant cell growing conditions; wherein the plant architecture is improved.
58. The method of item 57, wherein the plant cell is from a plant selected from the group consisting of: maize, soybean, sunflower, sorghum, canola, wheat, alfalfa, cotton, rice, barley, millet, peanut and cocoa.
59. A method of increasing yield in a plant, comprising:
   a. introducing into a plant cell a recombinant expression cassette comprising the polynucleotide of item 49 operably linked to a promoter;
   b. culturing the plant cell under plant cell growing conditions; and
   c. regenerating a plant form said plant cell; wherein the plant architecture is improved.
60. The method of item 59, wherein the plant is selected from the group consisting of: maize, soybean, sorghum, canola, wheat, alfalfa, cotton, rice, barley, millet, peanut, and cocoa.

## Claims

1. A method for plant characteristics, the method comprising:
(a) introducing into a plant cell a recombinant expression cassette comprising a polynucleotide whose expression, alone or in combination with additional polynucleotides, functions as a plant developmental regulator polypeptide within the plant;
(b) culturing the plant cell under plant forming conditions to produce a plant; and,
(c) inducing expression of the polynucleotide for a time sufficient to alter the architecture of the plant.

2. A method for increasing plant harvestable yield, the method comprising:
(a) introducing into a plant cell a recombinant expression cassette comprising a polynucleotide whose expression, alone or in combination with additional polynucleotides, functions as a plant developmental regulator polypeptide within the plant;
(b) culturing the plant cell under plant forming conditions to produce a plant; and
(c) inducing expression of the polynucleotide for a time sufficient to increase the harvestable yield of the plant.

3. The method of claim 1 or 2 wherein:
(a) the plant is a monocot, and preferably said plant is maize;
(b) the plant is a dicot;
(c) the plant is maize, barley, wheat, rice, rye, oats, millet, sorghum, soybean, canola, or sunflower;
(d) the polynucleotide is linked to a promoter;
(e) the polynucleotide is selected from SEQ ID NOS: 5, 3, 7, 9, 11, 13, 17, 19, 21, 23, 25, 27, 29, 31, 33, 35, 37, 39, 41, 43, 45, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181,183,1,15.
(f) expression of plant developmental regulator polypeptide within the plant is increased, wherein optionally said plant has:
(i) increased kernel number per ear;
(ii) an increased tassel spikelet density;
(iii) an increased tassel branch number;
(iv) increased pollen production;
(v) improved canopy shape;
(vi) increased photosynthetic capacity in leaf tissue;
(vii) improved stalk strength;
(viii) improved plant standability;
(ix) altered vascular bundle structure or number;
(x) increased root biomass;
(xi) enhanced root growth;
(xii) modulated shoot development; or
(xiii) modulated leaf development; or
(g) expression of plant developmental regulatory polypeptide within the plant is decreased, wherein optionally said plant:
(i) has shorter plant internodes;
(ii) has stunted growth; or
(iii) is a dwarf plant.

4. An isolated nucleic acid selected from:
(a) a nucleic acid comprising a polynucleotide sequence of the PDR coding a region or a complement thereof;
(b) a nucleic acid wherein the polynucleotide has at least 75% sequence identity to the PDR coding region as determined by GAP 10 analysis using default parameters over the entire length of the sequence, or a complement thereof, wherein expression of the polynucleotide modulates the level of PDR expression in a plant;
(c) a nucleic acid wherein the polynucleotide hybridizes under high stringency conditions to the PDR coding region, or a complement thereof, wherein expression of the polynucleotide modulates the level of PDR expression in a plant;
(d) a nucleic acid wherein the polynucleotide comprises the PDR coding region, or a variant thereof, wherein the expression of the variant modulates the level of PDR expression in a plant; or
(e) a nucleic acid wherein the polynucleotide comprises a fragment of the PDR coding region, wherein preferably said fragment is a functional fragment.

5. An expression cassette comprising the nucleic acid of claim 4 operably linked to a promoter, wherein the nucleic acid is in sense or antisense orientation.

6. A non-human host cell stably transformed with the expression cassette of claim 5, wherein optionally said host cell is:
(a) a plant cell; or
(b) a bacterial cell.

7. A plant stably transformed with the expression cassette of claim 5.

8. An isolated polynucleotide selected from the group consisting of:
(a) a polynucleotide having at least 80% sequence identity, as determined by the GAP algorithm under default parameters, to the full length sequence of a polynucleotide selected from SEQ ID NOS: 51, 89, 91, 97, 119, 127, 139, 147, 165 and 171; wherein the polynucleotide encodes a polypeptide that has PDR functions; and
(b) a polynucleotide encoding a polypeptide selected from SEQ ID NOS: 50, 90, 92, 98, 120, 128, 140, 148, 166, and 170; and
(c) a polynucleotide selected from SEQ ID NOS: 51, 89, 91, 97, 119, 127, 139, 147, 165 and 171; and
(d) a polynucleotide which is complementary to the polynucleotide of (a), (b), or (c).

9. A recombinant expression cassette, comprising the polynucleotide of claim 8, wherein the polynucleotide is operably linked, in sense or anti-sense orientation, to a promoter.

10. A host cell comprising the expression cassette of claim 9.

11. A transgenic plant produced by the method of claim 1 or 2.

12. A transgenic plant comprising the recombinant expression cassette of claim 10 wherein optionally said plant is:
(a) a monocot;
(b) a dicot; or
(c) selected from maize, soybean, sunflower, sorghum, canola, wheat, alfalfa, cotton, rice, barley, millet, peanut and cocoa.

13. A transgenic seed produced by the transgenic plant of claim 11 or 12.

14. A method of increasing yield in plants, comprising:
(a) introducing into a plant cell a recombinant expression cassette comprising the polynucleotide of claim 8 operably linked to a promoter; and
(b) culturing the plant under plant cell growing conditions; wherein the plant architecture is improved,
wherein optionally the plant cell is from a plant selected from maize, soybean, sunflower, sorghum, canola, wheat, alfalfa, cotton, rice, barley, millet, peanut and cocoa.

15. A method of increasing yield in a plant, comprising:
(a) introducing into a plant cell a recombinant expression cassette comprising the polynucleotide of claim 8 operably linked to a promoter;
(b) culturing the plant cell under plant cell growing conditions; and
(c) regenerating a plant from said plant cell; wherein the plant architecture is improved,
wherein optionally the plant is selected from maize, soybean, sorghum, canola, wheat, alfalfa, cotton, rice, barley, millet, peanut, and cocoa.


SEQUENCE LISTING

<110> Pioneer Hi-Bred International, Inc.

<120> Methods for Improving Crop Plant
  Architecture and Yield

<130> 2040-PCT

<150> 60/684,617
<151> 2005-05-25

<160> 184

<170> FastSEQ for Windows Version 4.0

<210> 1
<211> 522
<212> DNA
<213> Zea mays

<400> 1
atgtctaggt ctgtggagcc tctcatagtc gggcgggtga ttggagaagt tctcgactcc 60
tttaacccat gtgtcaagat gatagtaacc tacaactcaa acaaacttgt attcaatggc 120
catgagatct acccatcagc aattgtatct aaacctaggg tagaggttca agggggtgat 180
ttgcggtctt tcttcacatt ggttatgaca gacccagatg ttccaggacc aagtgatcca 240
tatctaaggg agcaccttca ttggatcgtg actgatatac ctgggacaac agatgcctcc 300
tttgggcgag aggtcataag ctatgagagc ccaagaccta acatcggtat ccacaggttc 360
atttttgtgc tcttcaagca gaagggtagg caaactgtaa ccgtgccatc cttcagagat 420
catttcaaca cccggcagtt tgctgaggaa aatgaccttg gcctcccagt agctgctgtc 480
tacttcaatg cacagagaga aactgcagct aggagacgtt ga 522

<210> 2
<211> 173
<212> PRT
<213> Zea mays

<400> 2
Met Ser Arg Ser Val Glu Pro Leu Ile Val Gly Arg Val Ile Gly Glu
 1               5                   10                  15
Val Leu Asp Ser Phe Asn Pro Cys Val Lys Met Ile Val Thr Tyr Asn
            20                  25                  30
Ser Asn Lys Leu Val Phe Asn Gly His Glu Ile Tyr Pro Ser Ala Ile
        35                  40                  45
Val Ser Lys Pro Arg Val Glu Val Gln Gly Gly Asp Leu Arg Ser Phe
    50                  55                  60
Phe Thr Leu Val Met Thr Asp Pro Asp Val Pro Gly Pro Ser Asp Pro
65                  70                  75                  80
Tyr Leu Arg Glu His Leu His Trp Ile Val Thr Asp Ile Pro Gly Thr
                85                  90                  95
Thr Asp Ala Ser Phe Gly Arg Glu Val Ile Ser Tyr Glu Ser Pro Arg
            100                 105                 110
Pro Asn Ile Gly Ile His Arg Phe Ile Phe Val Leu Phe Lys Gln Lys
        115                 120                 125
Gly Arg Gln Thr Val Thr Val Pro Ser Phe Arg Asp His Phe Asn Thr
    130                 135                 140
Arg Gln Phe Ala Glu Glu Asn Asp Leu Gly Leu Pro Val Ala Ala Val
145                 150                 155                 160
Tyr Phe Asn Ala Gln Arg Glu Thr Ala Ala Arg Arg Arg
                165                 170

<210> 3
<211> 522
<212> DNA
<213> Zea mays

<400> 3
atgtcaaggg tgttggagcc tctcattgtg gggaaagtga ttggtgaggt cctggaccat 60
ttcaacccca cggtgaagat ggtggtcacc tacaactcca acaagcaggt gttcaacggg 120
cacgagttct tcccttcggc agtggccgcc aagccgcgtg ttgaggtcca agggggcgac 180
ctcaggtcct tcttcacgtt ggtgatgacc gaccccgatg ttcctggacc tagtgatcca 240
tacttgaggg agcaccttca ctggattgtc actgatattc ctgggactac cgatgcttct 300
tttgggaaag aggtggtgag ctacgagatc ccaaagccaa acattggcat ccacaggttc 360
atctttgtgc tgttccggca gaagagccgg caagcggtga acccgcygtc gtcgaaggac 420
cgcttcagca cccgccagtt cgctgaggag aacgacctcg gcctccccgt cgccgccgtc 480
tacttcaacg cgcagcgcga gaccgccgcc cgccgacgct aa 522

<210> 4
<211> 173
<212> PRT
<213> Zea mays

<220>
<221> UNSURE
<222> (136)...(136)
<223> Xaa = any amino acid

<400> 4
Met Ser Arg Val Leu Glu Pro Leu Ile Val Gly Lys Val Ile Gly Glu
1               5                   10                  15
Val Leu Asp His Phe Asn Pro Thr Val Lys Met Val Val Thr Tyr Asn
            20                  25                  30
Ser Asn Lys Gln Val Phe Asn Gly His Glu Phe Phe Pro Ser Ala Val
        35                  40                  45
Ala Ala Lys Pro Arg Val Glu Val Gln Gly Gly Asp Leu Arg Ser Phe
    50                  55                  60
Phe Thr Leu Val Met Thr Asp Pro Asp Val Pro Gly Pro Ser Asp Pro
65                  70                  75                  80
Tyr Leu Arg Glu His Leu His Trp Ile Val Thr Asp Ile Pro Gly Thr
                85                  90                  95
Thr Asp Ala Ser Phe Gly Lys Glu Val Val Ser Tyr Glu Ile Pro Lys
            100                 105                 110
Pro Asn Ile Gly Ile His Arg Phe Ile Phe Val Leu Phe Arg Gln Lys
        115                 120                 125
Ser Arg Gln Ala Val Asn Pro Xaa Ser Ser Lys Asp Arg Phe Ser Thr
    130                 135                 140
Arg Gln Phe Ala Glu Glu Asn Asp Leu Gly Leu Pro Val Ala Ala Val
145                 150                 155                 160
Tyr Phe Asn Ala Gln Arg Glu Thr Ala Ala Arg Arg Arg
                165                 170

<210> 5
<211> 522
<212> DNA
<213> Zea mays

<400> 5
atgtccaggt ctgtggagcc tctcatagtc gggcgggtga tcggagaagt cctcgactcc 60
ttcaacccgt gtgtgaagat gatagtgacc tacaactcca acaaactcgt gttcaatggc 120

catgagatct acccatcagc tgttgtgtcc aaaccaaggg tggcggttca agggggcgat 180
ttgcggtctt tcttcacatt ggttatgaca gacccagatg ttccaggacc aagtgatcca 240
tacctaaggg agcaccttca ttggatcgtg actgatatac ctgggacaac agatgcctcc 300
ttcgggcgac agatcataag ctacgagagc ccaagaccta gcattggtat ccacaggttc 360
atttttgtgc tcttcaagca gcagggtagg caaaatgtaa ctgtgccatc cttcagagat 420
catttcaaca cccggcagtt cgctgaggaa aatgaccttg gcctccctgt agctgccgtc 480
tacttcaatg cacagagaga aactgctgct aggagacgct ga 522

<210> 6
<211> 173
<212> PRT
<213> Zea mays

<400> 6
Met Ser Arg Ser Val Glu Pro Leu Ile Val Gly Arg Val Ile Gly Glu
1               5                   10                  15
Val Leu Asp Ser Phe Asn Pro Cys Val Lys Met Ile Val Thr Tyr Asn
            20                  25                  30
Ser Asn Lys Leu Val Phe Asn Gly His Glu Ile Tyr Pro Ser Ala Val
        35                  40                  45
Val Ser Lys Pro Arg Val Ala Val Gln Gly Gly Asp Leu Arg Ser Phe
    50                  55                  60
Phe Thr Leu Val Met Thr Asp Pro Asp Val Pro Gly Pro Ser Asp Pro
65                  70                  75                  80
Tyr Leu Arg Glu His Leu His Trp Ile Val Thr Asp Ile Pro Gly Thr
                85                  90                  95
Thr Asp Ala Ser Phe Gly Arg Gln Ile Ile Ser Tyr Glu Ser Pro Arg
            100                 105                 110
Pro Ser Ile Gly Ile His Arg Phe Ile Phe Val Leu Phe Lys Gln Gln
        115                 120                 125
Gly Arg Gln Asn Val Thr Val Pro Ser Phe Arg Asp His Phe Asn Thr
    130                 135                 140
Arg Gln Phe Ala Glu Glu Asn Asp Leu Gly Leu Pro Val Ala Ala Val
145                 150                 155                 160
Tyr Phe Asn Ala Gln Arg Glu Thr Ala Ala Arg Arg Arg
                165                 170

<210> 7
<211> 531
<212> DNA
<213> Zea mays

<400> 7
atgtctagag cgttggaacc tctggtcgtc ggcaaggtga tcggggaggt catcgacaac 60
ttcaacccca cggtgaagat gacggttacc tacggatcca acaagcaggt gttcaacggc 120
catgagttct ttccgtctgc ggttctgtcc aagccgcgcg tggaggttca gggcgacgac 180
atgaggtcct tcttcacgct ggtcatgact gacccagatg tgccagggcc tagtgatcca 240
tacctgagag agcacatcca ttggatcgtc accgacattc ctggaacaac tgatgcttct 300
ttcggaaggg agttggtgat gtacgagagc ccgaagccgt acatcggcat ccacaggttc 360
gtcttcgtgc tgttcaagca gagcagccgg cagtcggcgc gcccgccctc gtccggcggc 420
ggcagggact acttcaacac ccgccgcttt gccgccgaca acaatcttgg cctcccagtt 480
gccgcggtct acttcaacgc gcagcgggag actgccgcgc gccgccgctg a 531

<210> 8
<211> 176
<212> PRT
<213> Zea mays

<400> 8
Met Ser Arg Ala Leu Glu Pro Leu Val Val Gly Lys Val Ile Gly Glu
1               5                   10                  15
Val Ile Asp Asn Phe Asn Pro Thr Val Lys Met Thr Val Thr Tyr Gly
            20                  25                  30
Ser Asn Lys Gln Val Phe Asn Gly His Glu Phe Phe Pro Ser Ala Val
        35                  40                  45
Leu Ser Lys Pro Arg Val Glu Val Gln Gly Asp Asp Met Arg Ser Phe
    50                  55                  60
Phe Thr Leu Val Met Thr Asp Pro Asp Val Pro Gly Pro Ser Asp Pro
65                  70                  75                  80
Tyr Leu Arg Glu His Ile His Trp Ile Val Thr Asp Ile Pro Gly Thr
                85                  90                  95
Thr Asp Ala Ser Phe Gly Arg Glu Leu Val Met Tyr Glu Ser Pro Lys
            100                 105                 110
Pro Tyr Ile Gly Ile His Arg Phe Val Phe Val Leu Phe Lys Gln Ser
        115                 120                 125
Ser Arg Gln Ser Ala Arg Pro Pro Ser Ser Gly Gly Gly Arg Asp Tyr
    130                 135                 140
Phe Asn Thr Arg Arg Phe Ala Ala Asp Asn Asn Leu Gly Leu Pro Val
145                 150                 155                 160
Ala Ala Val Tyr Phe Asn Ala Gln Arg Glu Thr Ala Ala Arg Arg Arg
                165                 170                 175

<210> 9
<211> 522
<212> DNA
<213> Zea mays

<400> 9
atgtctaggg cgttggagcc tctagtcgtc ggcaaggtga tcggcgaagt catcgacaac 60
ttcaacccca cggtgaagat gacggtcacc tacggctccg acaagcaggt gttcaacggc 120
catgagttct ttccgtcggc ggttctgtcc aagccgcgag tgcaggttca gggcgacgac 180
atgaggtcct tcttcacact ggtcatgacg gacccagatg tgccagggcc tagtgatcca 240
tacctgagag agcacctcca ttggatggtc actgacattc ctggaacaac tgatgcttct 300
tttggaaggg agcaggtgat gtacgagagc cccaaaccct acatcggctt ccacaggttc 360
gtcttcgtgc tgttcaagca gagcagccgc cagtcggtgt gcccgccctc gtccagggac 420
tacttcaaca cccgccgctt tgccgccgac aacaatcttg gcctcccagt cgccgccgtc 480
tacttcaacg cgcagcggga gaccgccgcg cgccgccgct ga                    522

<210> 10
<211> 173
<212> PRT
<213> Zea mays

<400> 10
Met Ser Arg Ala Leu Glu Pro Leu Val Val Gly Lys Val Ile Gly Glu
1               5                   10                  15
Val Ile Asp Asn Phe Asn Pro Thr Val Lys Met Thr Val Thr Tyr Gly
            20                  25                  30
Ser Asp Lys Gln Val Phe Asn Gly His Glu Phe Phe Pro Ser Ala Val
        35                  40                  45
Leu Ser Lys Pro Arg Val Gln Val Gln Gly Asp Asp Met Arg Ser Phe
    50                  55                  60
Phe Thr Leu Val Met Thr Asp Pro Asp Val Pro Gly Pro Ser Asp Pro
65                  70                  75                  80
Tyr Leu Arg Glu His Leu His Trp Met Val Thr Asp Ile Pro Gly Thr
                85                  90                  95
Thr Asp Ala Ser Phe Gly Arg Glu Gln Val Met Tyr Glu Ser Pro Lys
            100                 105                 110

Pro Tyr Ile Gly Phe His Arg Phe Val Phe Val Leu Phe Lys Gln Ser
        115                 120                 125
Ser Arg Gln Ser Val Cys Pro Pro Ser Ser Arg Asp Tyr Phe Asn Thr
    130                 135                 140
Arg Arg Phe Ala Ala Asp Asn Asn Leu Gly Leu Pro Val Ala Ala Val
145                 150                 155                 160
Tyr Phe Asn Ala Gln Arg Glu Thr Ala Ala Arg Arg Arg
                165                 170

<210> 11
<211> 534
<212> DNA
<213> Zea mays

<400> 11
atgtctagat ctgtggagtc tctcgtagtc ggccgggtga tcggagaagt tctcgactgc 60
ttcagcccat gtgtgaagat ggtagtgacc tacaactcaa acaggctcgt cttcaatggc 120
cacgagatct acccgtcagc agtcgtgtct aaaccaagag tagaggttca agggggtgac 180
ttgcggtcgt tcttcacatt ggttatgaca gacccagacg tcccaggacc aagcgatcca 240
tatctaaggg agcaccttca ctggatcgtg actgatatac ctgggacaac tgatgcctca 300
ttcgggagag aagtcgtaag ctatgagagc ccgagacctg gcattggtat ccacaggttc 360
atctttgttc tcttcaagca gaagcgcagg cagcagcaga ctgtagcggc ggtgccatcc 420
tccagcaggg accatttcat cacgcgtcag ttcgctgcgg aaaacgatct tggccaccct 480
gtagccgctg tgtacttcaa cgcccagaga gaaactgctg ctaggaggcg ctga 534

<210> 12
<211> 177
<212> PRT
<213> Zea mays

<400> 12
Met Ser Arg Ser Val Glu Ser Leu Val Val Gly Arg Val Ile Gly Glu
 1               5                  10                  15
Val Leu Asp Cys Phe Ser Pro Cys Val Lys Met Val Val Thr Tyr Asn
            20                  25                  30
Ser Asn Arg Leu Val Phe Asn Gly His Glu Ile Tyr Pro Ser Ala Val
        35                  40                  45
Val Ser Lys Pro Arg Val Glu Val Gln Gly Gly Asp Leu Arg Ser Phe
    50                  55                  60
Phe Thr Leu Val Met Thr Asp Pro Asp Val Pro Gly Pro Ser Asp Pro
65                  70                  75                  80
Tyr Leu Arg Glu His Leu His Trp Ile Val Thr Asp Ile Pro Gly Thr
                85                  90                  95
Thr Asp Ala Ser Phe Gly Arg Glu Val Val Ser Tyr Glu Ser Pro Arg
            100                 105                 110
Pro Gly Ile Gly Ile His Arg Phe Ile Phe Val Leu Phe Lys Gln Lys
        115                 120                 125
Arg Arg Gln Gln Gln Thr Val Ala Ala Val Pro Ser Ser Ser Arg Asp
    130                 135                 140
His Phe Ile Thr Arg Gln Phe Ala Ala Glu Asn Asp Leu Gly His Pro
145                 150                 155                 160
Val Ala Ala Val Tyr Phe Asn Ala Gln Arg Glu Thr Ala Ala Arg Arg
                165                 170                 175
Arg

<210> 13
<211> 579
<212> DNA

<213> Zea mays

<400> 13
atgctcaggc tgcagcttcc tcagtcccat agggttattt ttctgcagta tttgtcagca 60
accgatcctt tggttatggc tcgtgtccta caggatgtgt tggatacctt tacaccaacc 120
attccactaa gaataacata caacaatagt caagttctgg caggtgctga gctaaagcca 180
tctgcggtta taaataaacc acgagtcgat atcggtggca atgacatgag gactttctac 240
accctggtac tgattgaccc ggacgcccca agtccaagcc atccatcact aagggagtac 300
ttgcactgga tgatgacaga tattcctgaa acaactagtg tcaacttcgg ccaagagcta 360
gtattttatg agagaccaga tccaagatct ggtatccaca ggctggtatt tgtgttgttc 420
cgccaacttg gcaggggtac ggtttttgca ccagaaatgc gccaaaactt caactgcaga 480
agctttgcac ggcaatatca cctcagcatt gccagtgcta cacatttcaa ctgtcaaagg 540
gaaggtggat cgggtggaag aaggtttagg gaagagtag 579

<210> 14
<211> 192
<212> PRT
<213> Zea mays

<400> 14
Met Leu Arg Leu Gln Leu Pro Gln Ser His Arg Val Ile Phe Leu Gln
1               5                   10                  15
Tyr Leu Ser Ala Thr Asp Pro Leu Val Met Ala Arg Val Leu Gln Asp
            20                  25                  30
Val Leu Asp Thr Phe Thr Pro Thr Ile Pro Leu Arg Ile Thr Tyr Asn
        35                  40                  45
Asn Ser Gln Val Leu Ala Gly Ala Glu Leu Lys Pro Ser Ala Val Ile
    50                  55                  60
Asn Lys Pro Arg Val Asp Ile Gly Gly Asn Asp Met Arg Thr Phe Tyr
65                  70                  75                  80
Thr Leu Val Leu Ile Asp Pro Asp Ala Pro Ser Pro Ser His Pro Ser
                85                  90                  95
Leu Arg Glu Tyr Leu His Trp Met Met Thr Asp Ile Pro Glu Thr Thr
            100                 105                 110
Ser Val Asn Phe Gly Gln Glu Leu Val Phe Tyr Glu Arg Pro Asp Pro
        115                 120                 125
Arg Ser Gly Ile His Arg Leu Val Phe Val Leu Phe Arg Gln Leu Gly
    130                 135                 140
Arg Gly Thr Val Phe Ala Pro Glu Met Arg Gln Asn Phe Asn Cys Arg
145                 150                 155                 160
Ser Phe Ala Arg Gln Tyr His Leu Ser Ile Ala Ser Ala Thr His Phe
                165                 170                 175
Asn Cys Gln Arg Glu Gly Gly Ser Gly Gly Arg Arg Phe Arg Glu Glu
            180                 185                 190

<210> 15
<211> 528
<212> DNA
<213> Zea mays

<400> 15
atgtcagcaa ccgatcattt ggttatggct cgtgtcatac aggatgtatt ggatcccttt 60
acaccaacca ttccactaag aataacgtac aacaataggc tacttctgcc aagtgctgag 120
ctaaagccat ccgcggttgt aagtaaacca cgagtcgata tcggtggcag tgacatgagg 180
gctttctaca ccctggtact gattgacccg gatgccccaa gtccaagcca tccatcacta 240
agggagtact tgcactggat ggtgacagat attccagaaa caactagtgt caactttggc 300
caagagctaa tattttatga gaggccggac ccaagatctg gcatccacag gctggtattt 360
gtgctgttcc gtcaacttgg cagggggaca gtttttgcac cagaaatgcg ccacaacttc 420
aactgcagaa gctttgcacg gcaatatcac ctcagcattg ccaccgctac acatttcaac 480
tgtcaaaggg aaggtggatc cggcggaaga aggtttaggg aagagtag 528

<210> 16
<211> 175
<212> PRT
<213> Zea mays

<400> 16
Met Ser Ala Thr Asp His Leu Val Met Ala Arg Val Ile Gln Asp Val
1               5                   10                  15
Leu Asp Pro Phe Thr Pro Thr Ile Pro Leu Arg Ile Thr Tyr Asn Asn
            20                  25                  30
Arg Leu Leu Leu Pro Ser Ala Glu Leu Lys Pro Ser Ala Val Val Ser
        35                  40                  45
Lys Pro Arg Val Asp Ile Gly Gly Ser Asp Met Arg Ala Phe Tyr Thr
    50                  55                  60
Leu Val Leu Ile Asp Pro Asp Ala Pro Ser Pro Ser His Pro Ser Leu
65                  70                  75                  80
Arg Glu Tyr Leu His Trp Met Val Thr Asp Ile Pro Glu Thr Thr Ser
                85                  90                  95
Val Asn Phe Gly Gln Glu Leu Ile Phe Tyr Glu Arg Pro Asp Pro Arg
            100                 105                 110
Ser Gly Ile His Arg Leu Val Phe Val Leu Phe Arg Gln Leu Gly Arg
        115                 120                 125
Gly Thr Val Phe Ala Pro Glu Met Arg His Asn Phe Asn Cys Arg Ser
    130                 135                 140
Phe Ala Arg Gln Tyr His Leu Ser Ile Ala Thr Ala Thr His Phe Asn
145                 150                 155                 160
Cys Gln Arg Glu Gly Gly Ser Gly Gly Arg Arg Phe Arg Glu Glu
                165                 170                 175

<210> 17
<211> 519
<212> DNA
<213> Zea mays

<400> 17
atggcgcgct tcgtggatcc gctggtggtg gggcgggtga tcggcgaggt ggtggacctg 60
ttcgtgcctt ccatctccat gaccgtcgcc tatgatggcc ccaaggacat cagcaacggc 120
tgcctcctca agccgtccgc caccgccgcg ccgccgctcg tccgcatctc cggccgccgc 180
aacgacctct acacgctgat catgacggac cccgatgcgc ctagccccag caacccgacc 240
atgagggagt acctccactg gatagtgatt aacataccag gaggaacaga tgctactaaa 300
ggtgaggagg tggtggagta catgggcccg cggccgccgg tgggtatcca ccgctacgtg 360
ctggtgctgt tcgagcagaa gacgcgcgtg cacgcggagg cccccggcga ccgcgccaac 420
ttcaagacgc gcgcgttcgc ggcggcgcac gagctcggcc tccccactgc cgtcgtctac 480
ttcaacgcgc agaaggagcc cgccagccgc cgccgctag                        519

<210> 18
<211> 172
<212> PRT
<213> Zea mays

<400> 18
Met Ala Arg Phe Val Asp Pro Leu Val Val Gly Arg Val Ile Gly Glu
1               5                   10                  15
Val Val Asp Leu Phe Val Pro Ser Ile Ser Met Thr Val Ala Tyr Asp
            20                  25                  30
Gly Pro Lys Asp Ile Ser Asn Gly Cys Leu Leu Lys Pro Ser Ala Thr
        35                  40                  45
Ala Ala Pro Pro Leu Val Arg Ile Ser Gly Arg Arg Asn Asp Leu Tyr
    50                  55                  60

Thr Leu Ile Met Thr Asp Pro Asp Ala Pro Ser Pro Ser Asn Pro Thr
65                  70                  75                  80
Met Arg Glu Tyr Leu His Trp Ile Val Ile Asn Ile Pro Gly Gly Thr
                85                  90                  95
Asp Ala Thr Lys Gly Glu Glu Val Val Glu Tyr Met Gly Pro Arg Pro
            100                 105                 110
Pro Val Gly Ile His Arg Tyr Val Leu Val Leu Phe Glu Gln Lys Thr
        115                 120                 125
Arg Val His Ala Glu Ala Pro Gly Asp Arg Ala Asn Phe Lys Thr Arg
    130                 135                 140
Ala Phe Ala Ala Ala His Glu Leu Gly Leu Pro Thr Ala Val Val Tyr
145                 150                 155                 160
Phe Asn Ala Gln Lys Glu Pro Ala Ser Arg Arg Arg
                165                 170

<210> 19
<211> 513
<212> DNA
<213> Zea mays

<400> 19
atggcgcggt tcgtggaccc gctggtggtg gggcgggtga tcggcgaggt ggtggacctg 60
ttcgtgccct ccgtctccat gaccgtcgcc tatggcccca aagacatcag caacggctgc 120
ctcctcaagc cgtccgccac cgccgcgccg ccgctcgtcc gcatctccgg ccgccgcgac 180
gacctctaca cgctgatcat gacggaccca gatgcgccta gccccagcga cccgaccatg 240
agggagtacc tccactggat agtgactaac ataccaggag gaacggatgc aaacaaagag 300
gtggtggagt acatgggccc gcggccgccg gtcggaatcc accgctacgt gctggtgctg 360
ttcgagcaga agacgcgtgt gcacgcggag ggtcccggtg agcgcgccaa cttcaacaca 420
cgcgcgttcg cggcggcgca cgagctcggc ctccccaccg ccgtcgtgta cttcaacgcg 480
cagaaagagc cggccaacca ccgccgccgc tag                               513

<210> 20
<211> 170
<212> PRT
<213> Zea mays

<400> 20
Met Ala Arg Phe Val Asp Pro Leu Val Val Gly Arg Val Ile Gly Glu
1               5                   10                  15
Val Val Asp Leu Phe Val Pro Ser Val Ser Met Thr Val Ala Tyr Gly
            20                  25                  30
Pro Lys Asp Ile Ser Asn Gly Cys Leu Leu Lys Pro Ser Ala Thr Ala
        35                  40                  45
Ala Pro Pro Leu Val Arg Ile Ser Gly Arg Arg Asp Asp Leu Tyr Thr
    50                  55                  60
Leu Ile Met Thr Asp Pro Asp Ala Pro Ser Pro Ser Asp Pro Thr Met
65                  70                  75                  80
Arg Glu Tyr Leu His Trp Ile Val Thr Asn Ile Pro Gly Gly Thr Asp
                85                  90                  95
Ala Asn Lys Glu Val Val Glu Tyr Met Gly Pro Arg Pro Pro Val Gly
            100                 105                 110
Ile His Arg Tyr Val Leu Val Leu Phe Glu Gln Lys Thr Arg Val His
        115                 120                 125
Ala Glu Gly Pro Gly Glu Arg Ala Asn Phe Asn Thr Arg Ala Phe Ala
    130                 135                 140
Ala Ala His Glu Leu Gly Leu Pro Thr Ala Val Val Tyr Phe Asn Ala
145                 150                 155                 160
Gln Lys Glu Pro Ala Asn His Arg Arg Arg
                165                 170

<210> 21
<211> 543
<212> DNA
<213> Zea mays

<400> 21
atggctgccc atgtggaccc gctggttgtg gggagggtga tcggcgacgt ggtggacttg 60
ttcgtgccga cggtggccgt gtcggcgcgc ttcggcgcca aggacctcac caacggctgc 120
gagatcaagc catccgtcgc cgcggccgct cccgccgtcc tcatcgccgg cagggccaac 180
gacctcttca ccctggttat gactgaccca gatgctccga gccctagcga gccaacgatg 240
agggagttgc tccactggct ggtggttaac ataccaggtg gagcagatgc ttctcaaggc 300
ggtgagacgg tggtgccgta cgtgggcccg cgcccgccgg tgggtatcca ccgctacgtg 360
ctggtggtgt accagcagaa ggcccgcgtc acggctccgc cgtcgctggc gccggcgacg 420
gaggcgacgc gcgcacggtt cagcaaccgc gccttcgccg accgccatga cctaggcctc 480
cctgtcgccg ccatgttctt caacgcgcag aaggagacag ctagtcgccg ccgccactac 540
tga 543

<210> 22
<211> 180
<212> PRT
<213> Zea mays

<400> 22
Met Ala Ala His Val Asp Pro Leu Val Val Gly Arg Val Ile Gly Asp
1               5                   10                  15
Val Val Asp Leu Phe Val Pro Thr Val Ala Val Ser Ala Arg Phe Gly
            20                  25                  30
Ala Lys Asp Leu Thr Asn Gly Cys Glu Ile Lys Pro Ser Val Ala Ala
        35                  40                  45
Ala Ala Pro Ala Val Leu Ile Ala Gly Arg Ala Asn Asp Leu Phe Thr
    50                  55                  60
Leu Val Met Thr Asp Pro Asp Ala Pro Ser Pro Ser Glu Pro Thr Met
65                  70                  75                  80
Arg Glu Leu Leu His Trp Leu Val Val Asn Ile Pro Gly Gly Ala Asp
                85                  90                  95
Ala Ser Gln Gly Gly Glu Thr Val Val Pro Tyr Val Gly Pro Arg Pro
            100                 105                 110
Pro Val Gly Ile His Arg Tyr Val Leu Val Val Tyr Gln Gln Lys Ala
        115                 120                 125
Arg Val Thr Ala Pro Pro Ser Leu Ala Pro Ala Thr Glu Ala Thr Arg
    130                 135                 140
Ala Arg Phe Ser Asn Arg Ala Phe Ala Asp Arg His Asp Leu Gly Leu
145                 150                 155                 160
Pro Val Ala Ala Met Phe Phe Asn Ala Gln Lys Glu Thr Ala Ser Arg
                165                 170                 175
Arg Arg His Tyr
            180

<210> 23
<211> 534
<212> DNA
<213> Zea mays

<400> 23
atgtctgatg tggagccgct ggttctggct catgtcatac gagatgtgtt ggattcattt 60
gcaccaagta tcgggctcag aataacctac aacagcaggt tacttctatc aggtgttgag 120
ctgaaaccat ccgcggttgt gaataagcca agagttgatg ttgggggcac cgacctcagg 180
gtgttctaca cattggtatt agtggatcca gatgccccaa gcccaagcaa tccatcactg 240
agggagtatc tgcactggat ggtgatagac attcctggaa caactggagc cagctttggt 300

caggagctca tgttttacga gaggccagag ccgaggtccg gcatacaccg catggtgttc 360
gtgctgttcc ggcagctcgg cagggggacg gtgtttgcac cagacatgcg gcacaacttc 420
aactgcaaga gcttcgcccg tcagtaccac ctggacgtcg tggctgccac gtatttcaac 480
tgccaaaggg aggcaggatc cgggggcaga aggttcaggc cggagagctc gtaa 534

<210> 24
<211> 177
<212> PRT
<213> Zea mays

<400> 24
Met Ser Asp Val Glu Pro Leu Val Leu Ala His Val Ile Arg Asp Val
1               5                   10                  15
Leu Asp Ser Phe Ala Pro Ser Ile Gly Leu Arg Ile Thr Tyr Asn Ser
            20                  25                  30
Arg Leu Leu Leu Ser Gly Val Glu Leu Lys Pro Ser Ala Val Val Asn
        35                  40                  45
Lys Pro Arg Val Asp Val Gly Gly Thr Asp Leu Arg Val Phe Tyr Thr
    50                  55                  60
Leu Val Leu Val Asp Pro Asp Ala Pro Ser Pro Ser Asn Pro Ser Leu
65                  70                  75                  80
Arg Glu Tyr Leu His Trp Met Val Ile Asp Ile Pro Gly Thr Thr Gly
                85                  90                  95
Ala Ser Phe Gly Gln Glu Leu Met Phe Tyr Glu Arg Pro Glu Pro Arg
            100                 105                 110
Ser Gly Ile His Arg Met Val Phe Val Leu Phe Arg Gln Leu Gly Arg
        115                 120                 125
Gly Thr Val Phe Ala Pro Asp Met Arg His Asn Phe Asn Cys Lys Ser
    130                 135                 140
Phe Ala Arg Gln Tyr His Leu Asp Val Val Ala Ala Thr Tyr Phe Asn
145                 150                 155                 160
Cys Gln Arg Glu Ala Gly Ser Gly Gly Arg Arg Phe Arg Pro Glu Ser
                165                 170                 175
Ser

<210> 25
<211> 555
<212> DNA
<213> Zea mays

<400> 25
atggccaacg attccttggt cacagctcgt gtcataggag atgtcctgga ccccttctac 60
agctccattg atctgatggt gctgttcaac ggtttgccta ttgttagtgg cgtggagctg 120
cgtcctcccg cggtctccga gagacccagg gtcgagatcg gaggagatga ttatcgcgtt 180
gcatgtactc tggtgatggt cgatccagat gccccgaacc caagcaaccc gaccctgagg 240
gagtacctgc actggatggt gactgacatc ccagcgtcca ccgatgatac acacggtcgg 300
gaggtgatgt gctacgaggc ccctaatccg acgacgggca tccaccgcat ggtgctggtg 360
ctgttccggc agctggggcg ggagacggtg tacgcgccat ccaggcgcca caacttcagc 420
acgcgcgcct tcgcccgccg ctacaacctc ggcgcgcccg tcgcagccat gtacttcaac 480
tgccagcgcc agaacggctc cggcggacgg aggttcaccg ggccctacac cggcggcaga 540
cgtggtggtg cttga 555

<210> 26
<211> 184
<212> PRT
<213> Zea mays

<400> 26
Met Ala Asn Asp Ser Leu Val Thr Ala Arg Val Ile Gly Asp Val Leu
1               5                   10                  15
Asp Pro Phe Tyr Ser Ser Ile Asp Leu Met Val Leu Phe Asn Gly Leu
            20                  25                  30
Pro Ile Val Ser Gly Val Glu Leu Arg Pro Pro Ala Val Ser Glu Arg
        35                  40                  45
Pro Arg Val Glu Ile Gly Gly Asp Asp Tyr Arg Val Ala Cys Thr Leu
    50                  55                  60
Val Met Val Asp Pro Asp Ala Pro Asn Pro Ser Asn Pro Thr Leu Arg
65                  70                  75                  80
Glu Tyr Leu His Trp Met Val Thr Asp Ile Pro Ala Ser Thr Asp Asp
                85                  90                  95
Thr His Gly Arg Glu Val Met Cys Tyr Glu Ala Pro Asn Pro Thr Thr
            100                 105                 110
Gly Ile His Arg Met Val Leu Val Leu Phe Arg Gln Leu Gly Arg Glu
        115                 120                 125
Thr Val Tyr Ala Pro Ser Arg Arg His Asn Phe Ser Thr Arg Ala Phe
    130                 135                 140
Ala Arg Arg Tyr Asn Leu Gly Ala Pro Val Ala Ala Met Tyr Phe Asn
145                 150                 155                 160
Cys Gln Arg Gln Asn Gly Ser Gly Gly Arg Arg Phe Thr Gly Pro Tyr
                165                 170                 175
Thr Gly Gly Arg Arg Gly Gly Ala
            180

<210> 27
<211> 522
<212> DNA
<213> Zea mays

<400> 27
atgcagcgtg gggatccgct ggtggtgggc cgcatcatcg gcgacgtggt ggaccccttc 60
gtgcgccggg tgccgctccg cgtcgcctac gccgcgcgcg aggtctccaa cggctgcgag 120
ctcaggccct ccgccatcgc cgaccagccg cgcgtcgagg tcggcggacc cgacatgcgc 180
accttctaca ccctcgtgat ggtagatcct gatgcgccga gccccagcga tcccaacctc 240
agggagtacc tgcactggct ggtcactgat attccggcga cgactggagt atcttttggg 300
accgaggtcg tgtgctacga gagcccacgg ccggtgctgg ggatccaccg ggtcgtgttt 360
ctgctcttcc agcagctcgg ccggcagacg gtgtacgccc cggggtggcg gcagaacttc 420
agcacccgcg acttcgccga gctctacaac ctcggcttgc cggtcgccgc cgtctacttc 480
aactgccaga gggagtccgg aaccggtggg agaagaatgt ga                   522

<210> 28
<211> 173
<212> PRT
<213> Zea mays

<400> 28
Met Gln Arg Gly Asp Pro Leu Val Val Gly Arg Ile Ile Gly Asp Val
1               5                   10                  15
Val Asp Pro Phe Val Arg Arg Val Pro Leu Arg Val Ala Tyr Ala Ala
            20                  25                  30
Arg Glu Val Ser Asn Gly Cys Glu Leu Arg Pro Ser Ala Ile Ala Asp
        35                  40                  45
Gln Pro Arg Val Glu Val Gly Gly Pro Asp Met Arg Thr Phe Tyr Thr
    50                  55                  60
Leu Val Met Val Asp Pro Asp Ala Pro Ser Pro Ser Asp Pro Asn Leu
65                  70                  75                  80
Arg Glu Tyr Leu His Trp Leu Val Thr Asp Ile Pro Ala Thr Thr Gly
                85                  90                  95

Val Ser Phe Gly Thr Glu Val Val Cys Tyr Glu Ser Pro Arg Pro Val
            100                 105                 110
Leu Gly Ile His Arg Val Val Phe Leu Leu Phe Gln Gln Leu Gly Arg
        115                 120                 125
Gln Thr Val Tyr Ala Pro Gly Trp Arg Gln Asn Phe Ser Thr Arg Asp
    130                 135                 140
Phe Ala Glu Leu Tyr Asn Leu Gly Leu Pro Val Ala Ala Val Tyr Phe
145                 150                 155                 160
Asn Cys Gln Arg Glu Ser Gly Thr Gly Gly Arg Arg Met
                165                 170

<210> 29
<211> 534
<212> DNA
<213> Zea mays

<400> 29
atggccggca gggacaggga gccgctggtg gttggtaggg tggtcggcga cgtgctggac 60
cccttcgtcc ggaccaccaa cctcagggtc agctacgggg ccaggaccgt gtccaacggc 120
tgcgagctca agccgtccat ggtggtgcac cagcccaggg tcgaggtcgg gggacctgac 180
atgaggacct tctacaccct cgtgatggtg gacccggatg ctccgagccc aagcgacccg 240
aaccttaggg agtacctaca ctggctggtg acggatattc cgggaactac tggggcagca 300
tttgggcaag aggtgatctg ctacgagagc cctcggccga ccatggggat ccaccgcttc 360
gtgctggtgc tgttccagca gctggggcgg cagacggtgt acgccccggg ctggcgccag 420
aacttcaaca ccagggactt cgccgagctc tacaacctgg gcccgcccgt cgccgccgtc 480
tacttcaact gccagcgtga ggccggctct gggggcagga ggatgtactc gtga 534

<210> 30
<211> 177
<212> PRT
<213> Zea mays

<400> 30
Met Ala Gly Arg Asp Arg Glu Pro Leu Val Val Gly Arg Val Val Gly
 1               5                  10                  15
Asp Val Leu Asp Pro Phe Val Arg Thr Thr Asn Leu Arg Val Ser Tyr
            20                  25                  30
Gly Ala Arg Thr Val Ser Asn Gly Cys Glu Leu Lys Pro Ser Met Val
        35                  40                  45
Val His Gln Pro Arg Val Glu Val Gly Gly Pro Asp Met Arg Thr Phe
    50                  55                  60
Tyr Thr Leu Val Met Val Asp Pro Asp Ala Pro Ser Pro Ser Asp Pro
65                  70                  75                  80
Asn Leu Arg Glu Tyr Leu His Trp Leu Val Thr Asp Ile Pro Gly Thr
                85                  90                  95
Thr Gly Ala Ala Phe Gly Gln Glu Val Ile Cys Tyr Glu Ser Pro Arg
            100                 105                 110
Pro Thr Met Gly Ile His Arg Phe Val Leu Val Leu Phe Gln Gln Leu
        115                 120                 125
Gly Arg Gln Thr Val Tyr Ala Pro Gly Trp Arg Gln Asn Phe Asn Thr
    130                 135                 140
Arg Asp Phe Ala Glu Leu Tyr Asn Leu Gly Pro Pro Val Ala Ala Val
145                 150                 155                 160
Tyr Phe Asn Cys Gln Arg Glu Ala Gly Ser Gly Gly Arg Arg Met Tyr
                165                 170                 175
Ser

<210> 31
<211> 525
<212> DNA
<213> Zea mays

<400> 31
atgtcaaggg acccacttgt cgtaggcaac gtagttggag atatcttgga cccatttatc 60
aaatcagcat cactcagagt cctatacaac aatagagaac tgactaatgg atctgagctc 120
aggccatcgc aagtagctta tgaaccaagg attgagattg ctggatatga catgaggacc 180
ctttacactt tggtaatggt ggatcctgac tcaccaagtc caagcaatcc aacaaaaaga 240
gagtaccttc actggttggt gacagatatt ccagaatcaa cagatgtgag ctttggaaat 300
gaggtagtaa gctatgaaag cccaaagcca agtgctggaa tacatcgctt cgtctttgtt 360
ctgttccgcc aatctgtcag gcaaactatt tatgcgccag gatggagaca aaatttcaac 420
acaagagact tctcagcact ctataatcta ggaccacctg tggcctcagt gttcttcaac 480
tgccaaaggg agaatgggtg cggtggcaga cgatatatta gatga 525

<210> 32
<211> 174
<212> PRT
<213> Zea mays

<400> 32
Met Ser Arg Asp Pro Leu Val Val Gly Asn Val Val Gly Asp Ile Leu
1               5                   10                  15
Asp Pro Phe Ile Lys Ser Ala Ser Leu Arg Val Leu Tyr Asn Asn Arg
            20                  25                  30
Glu Leu Thr Asn Gly Ser Glu Leu Arg Pro Ser Gln Val Ala Tyr Glu
        35                  40                  45
Pro Arg Ile Glu Ile Ala Gly Tyr Asp Met Arg Thr Leu Tyr Thr Leu
    50                  55                  60
Val Met Val Asp Pro Asp Ser Pro Ser Pro Ser Asn Pro Thr Lys Arg
65                  70                  75                  80
Glu Tyr Leu His Trp Leu Val Thr Asp Ile Pro Glu Ser Thr Asp Val
                85                  90                  95
Ser Phe Gly Asn Glu Val Val Ser Tyr Glu Ser Pro Lys Pro Ser Ala
            100                 105                 110
Gly Ile His Arg Phe Val Phe Val Leu Phe Arg Gln Ser Val Arg Gln
        115                 120                 125
Thr Ile Tyr Ala Pro Gly Trp Arg Gln Asn Phe Asn Thr Arg Asp Phe
    130                 135                 140
Ser Ala Leu Tyr Asn Leu Gly Pro Pro Val Ala Ser Val Phe Phe Asn
145                 150                 155                 160
Cys Gln Arg Glu Asn Gly Cys Gly Gly Arg Arg Tyr Ile Arg
                165                 170

<210> 33
<211> 540
<212> DNA
<213> Zea mays

<400> 33
atgttcaata tgtctaggga cccattggtc gtcggcaatg ttgtgggaga tattgtggat 60
cccttcatca caacggcgtc actgagagtg ttctacaaca ataaggagat gacaaatggt 120
tctgatctta agccatctca agtgatgaat gaaccaaggg tccacgtcgg tgggcgtgac 180
atgaggactc tttacacact tgtaagtgtc atggtggacc cagatgcacc aagccccagt 240
aaccctacaa aaagagagaa ccttcactgg ttggtgacag acattccaga gacaactgat 300
gccagtttcg ggaacgaaat agttccgtac gagagcccac gtccaatcgc cggaatccat 360
cgcttcgcat tcgtcctgtt caggcagtca gtgaggcaga ccacctatgc gccgggatgg 420
agatcaaact tcaacactag agacttcgca gccatctacg gccttggctc ccctgtcgct 480
gcagtgtact tcaactgcca gagagagaac ggatgtggtg gaagaaggta cataaggtga 540

<210> 34
<211> 179
<212> PRT
<213> Zea mays

<400> 34
Met Phe Asn Met Ser Arg Asp Pro Leu Val Val Gly Asn Val Val Gly
1               5                   10                  15
Asp Ile Val Asp Pro Phe Ile Thr Thr Ala Ser Leu Arg Val Phe Tyr
            20                  25                  30
Asn Asn Lys Glu Met Thr Asn Gly Ser Asp Leu Lys Pro Ser Gln Val
        35                  40                  45
Met Asn Glu Pro Arg Val His Val Gly Gly Arg Asp Met Arg Thr Leu
    50                  55                  60
Tyr Thr Leu Val Ser Val Met Val Asp Pro Asp Ala Pro Ser Pro Ser
65                  70                  75                  80
Asn Pro Thr Lys Arg Glu Asn Leu His Trp Leu Val Thr Asp Ile Pro
                85                  90                  95
Glu Thr Thr Asp Ala Ser Phe Gly Asn Glu Ile Val Pro Tyr Glu Ser
            100                 105                 110
Pro Arg Pro Ile Ala Gly Ile His Arg Phe Ala Phe Val Leu Phe Arg
        115                 120                 125
Gln Ser Val Arg Gln Thr Thr Tyr Ala Pro Gly Trp Arg Ser Asn Phe
    130                 135                 140
Asn Thr Arg Asp Phe Ala Ala Ile Tyr Gly Leu Gly Ser Pro Val Ala
145                 150                 155                 160
Ala Val Tyr Phe Asn Cys Gln Arg Glu Asn Gly Cys Gly Gly Arg Arg
                165                 170                 175
Tyr Ile Arg

<210> 35
<211> 222
<212> DNA
<213> Zea mays

<400> 35
ggcaatgaaa tagttcccta tgaaagccca aggccaccag ctggaattca tcgaattgtt 60
tttgtgctgt tcaaacagca aacaagacaa acagtttatg caccaggatg gcggcaaaat 120
ttcaacatca gagacttctc ggcaatttac aatcttggag caccagttgc tgcattatac 180
ttcaactgcc aaaaggaaag tggtgttggt ggcagaaggt ag                    222

<210> 36
<211> 73
<212> PRT
<213> Zea mays

<400> 36
Gly Asn Glu Ile Val Pro Tyr Glu Ser Pro Arg Pro Pro Ala Gly Ile
1               5                   10                  15
His Arg Ile Val Phe Val Leu Phe Lys Gln Gln Thr Arg Gln Thr Val
            20                  25                  30
Tyr Ala Pro Gly Trp Arg Gln Asn Phe Asn Ile Arg Asp Phe Ser Ala
        35                  40                  45
Ile Tyr Asn Leu Gly Ala Pro Val Ala Ala Leu Tyr Phe Asn Cys Gln
    50                  55                  60
Lys Glu Ser Gly Val Gly Gly Arg Arg
65                  70

<210> 37
<211> 195
<212> DNA
<213> Zea mays

<400> 37
atgtcaaggg atccactagt ggtaggacac gtggtgggtg acattttgga cccgtttact 60
aaagcagcct cgcttaaggt tctgtacaac aacaaggaac tgaccaatgg gtctgagctc 120
aagccatcgc aggtagcaaa tgaaccgagg gttgaaataa ttggtgggcg cgacatgagc 180
aacctttaca ctctg 195

<210> 38
<211> 65
<212> PRT
<213> Zea mays

<400> 38
Met Ser Arg Asp Pro Leu Val Val Gly His Val Val Gly Asp Ile Leu
1               5                   10                  15
Asp Pro Phe Thr Lys Ala Ala Ser Leu Lys Val Leu Tyr Asn Asn Lys
            20                  25                  30
Glu Leu Thr Asn Gly Ser Glu Leu Lys Pro Ser Gln Val Ala Asn Glu
        35                  40                  45
Pro Arg Val Glu Ile Ile Gly Gly Arg Asp Met Ser Asn Leu Tyr Thr
    50                  55                  60
Leu
65

<210> 39
<211> 528
<212> DNA
<213> Zea mays

<400> 39
atgtccaggg atccgcttgt ggtgggaagc atcgtgggcg acgtcgtgga ctacttctcg 60
gcgtcggcgc tgctccgagt gatgtacggc gggcgcgaga tgacctgcgg gtcggagctc 120
aggccgtcgc aggtggcgag cgagccgacg gtgcacatca cgggggggccg cgacgggagg 180
ccggtgctct acacactggt gatgctggac cccgatgcgc ccagcccaag caacccctcc 240
aagcgggagt atctccattg gttggtgact gacataccag aaggagctgg tgccaatcat 300
gggaacgagg tggtggcgta cgagagcccc cggccatcgg cggggatcca ccgattcgtg 360
ttcatcgtgt tccggcaggc ggtccggcag gcgatctacg cgcctgggtg gcgcgccaac 420
ttcaacacca gggacttcgc cgcctgctac agcctcggac cgcctgtcgc cgccacctac 480
ttcaactgcc agagggaggg cggctgcggt ggtcggaggt acaggtga 528

<210> 40
<211> 175
<212> PRT
<213> Zea mays

<400> 40
Met Ser Arg Asp Pro Leu Val Val Gly Ser Ile Val Gly Asp Val Val
1               5                   10                  15
Asp Tyr Phe Ser Ala Ser Ala Leu Leu Arg Val Met Tyr Gly Gly Arg
            20                  25                  30
Glu Met Thr Cys Gly Ser Glu Leu Arg Pro Ser Gln Val Ala Ser Glu
        35                  40                  45
Pro Thr Val His Ile Thr Gly Gly Arg Asp Gly Arg Pro Val Leu Tyr
    50                  55                  60

Thr Leu Val Met Leu Asp Pro Asp Ala Pro Ser Pro Ser Asn Pro Ser
65                  70                  75                  80
Lys Arg Glu Tyr Leu His Trp Leu Val Thr Asp Ile Pro Glu Gly Ala
                85                  90                  95
Gly Ala Asn His Gly Asn Glu Val Val Ala Tyr Glu Ser Pro Arg Pro
            100                 105                 110
Ser Ala Gly Ile His Arg Phe Val Phe Ile Val Phe Arg Gln Ala Val
        115                 120                 125
Arg Gln Ala Ile Tyr Ala Pro Gly Trp Arg Ala Asn Phe Asn Thr Arg
    130                 135                 140
Asp Phe Ala Ala Cys Tyr Ser Leu Gly Pro Pro Val Ala Ala Thr Tyr
145                 150                 155                 160
Phe Asn Cys Gln Arg Glu Gly Gly Cys Gly Gly Arg Arg Tyr Arg
                165                 170                 175

<210> 41
<211> 303
<212> DNA
<213> Zea mays

<400> 41
atggcgccgg cggctaacga ttccttggtc acagctcatg tgataggaga tgtcctggac 60
cccttctaca cagccgttga catgatgatc ctgttcggtg gtgctcccat catcagcggc 120
atggagctgc gcgctcaggc agtctctgat aggccaaggg ttgagatcgg aggagaagat 180
tatcgagatg catataccct ggtgatggtc gatcctgatg ctcctaaccc aagcaaccca 240
accttgaggg agtacttgca ctggatggtg actgacatcc ccgcatcaac tgacaataca 300
cac                                                               303

<210> 42
<211> 101
<212> PRT
<213> Zea mays

<400> 42
Met Ala Pro Ala Ala Asn Asp Ser Leu Val Thr Ala His Val Ile Gly
 1               5                  10                  15
Asp Val Leu Asp Pro Phe Tyr Thr Ala Val Asp Met Met Ile Leu Phe
            20                  25                  30
Gly Gly Ala Pro Ile Ile Ser Gly Met Glu Leu Arg Ala Gln Ala Val
        35                  40                  45
Ser Asp Arg Pro Arg Val Glu Ile Gly Gly Glu Asp Tyr Arg Asp Ala
    50                  55                  60
Tyr Thr Leu Val Met Val Asp Pro Asp Ala Pro Asn Pro Ser Asn Pro
65                  70                  75                  80
Thr Leu Arg Glu Tyr Leu His Trp Met Val Thr Asp Ile Pro Ala Ser
                85                  90                  95
Thr Asp Asn Thr His
            100

<210> 43
<211> 258
<212> DNA
<213> Zea mays

<400> 43
cgtgagatga tgtgctacga gcccccagcc ccgtcgacgg gcatccaccg gatggtgctg 60
gtgctgttcc agcagcttgg gcgggacacg gtgttcgcgg cgccgtcgag gcgccacaac 120
ttcagcaccc gtggcttcgc ccgccgctac aacctcggcg cgcccgtcgc cgccatgtac 180
ttcaactgcc agcgccagac cggctccggc ggccccaggt tcaccgggcc ctacaccagc 240

cgccgtcgtg cgggctga 258

<210> 44
<211> 85
<212> PRT
<213> Zea mays

<400> 44
Arg Glu Met Met Cys Tyr Glu Pro Pro Ala Pro Ser Thr Gly Ile His
1               5                   10                  15
Arg Met Val Leu Val Leu Phe Gln Gln Leu Gly Arg Asp Thr Val Phe
            20                  25                  30
Ala Ala Pro Ser Arg Arg His Asn Phe Ser Thr Arg Gly Phe Ala Arg
        35                  40                  45
Arg Tyr Asn Leu Gly Ala Pro Val Ala Ala Met Tyr Phe Asn Cys Gln
    50                  55                  60
Arg Gln Thr Gly Ser Gly Gly Pro Arg Phe Thr Gly Pro Tyr Thr Ser
65                  70                  75                  80
Arg Arg Arg Ala Gly
                85

<210> 45
<211> 315
<212> DNA
<213> Zea mays

<400> 45
cgcgaggtga tctgctacga gagccctcgg ccgccggcgg ggatccaccg cgtggtgttc 60
gtgctctacc agcagacggc gcgcggcgcc gtcgaccagc cgccgcttct ccgccacaac 120
ttctgcaccc gcagcttcgc cgtcgaccac gggctgggcg cccccgtcgc cgccgccttc 180
ttcacctgtc agcccgaggg tggcaccggc ggccgccgcc acgtcctccg ccagccagca 240
aggtcaccag cgcctataga tgtccaaaca gtacgggccg tccgtttggc ccgtgacccg 300
gcacgatttt ggccc 315

<210> 46
<211> 105
<212> PRT
<213> Zea mays

<400> 46
Arg Glu Val Ile Cys Tyr Glu Ser Pro Arg Pro Pro Ala Gly Ile His
1               5                   10                  15
Arg Val Val Phe Val Leu Tyr Gln Gln Thr Ala Arg Gly Ala Val Asp
            20                  25                  30
Gln Pro Pro Leu Leu Arg His Asn Phe Cys Thr Arg Ser Phe Ala Val
        35                  40                  45
Asp His Gly Leu Gly Ala Pro Val Ala Ala Ala Phe Phe Thr Cys Gln
    50                  55                  60
Pro Glu Gly Gly Thr Gly Gly Arg Arg His Val Leu Arg Gln Pro Ala
65                  70                  75                  80
Arg Ser Pro Ala Pro Ile Asp Val Gln Thr Val Arg Ala Val Arg Leu
                85                  90                  95
Ala Arg Asp Pro Ala Arg Phe Trp Pro
            100                 105

<210> 47
<211> 588
<212> DNA
<213> Zea mays

<400> 47
attttgagga gaccggtggt tgcaagatta tattcaactt taagcaaaag tatggaaaaa 60
cacggtgttg tgcccgatgt tatcgatgtt gcccccgaac aacaagtgga agtgtcgtat 120
cctagtggtg taaaggtaga ctttggtaac gaattaactc caacacaagt caaagatatc 180
ccggcagtaa aatggccggc cgataaagat tccctttaca cactttgcat gaccgatcct 240
gatgccccaa gtcgaaaaga acccaagttc cgtgaatggc accattggct cgttggaaat 300
atcccaggag gagaggtctc aaaaggcgaa gttctttctg aatatgttgg gtctggacca 360
ccaccaaata caggtcttca taggtatgtt ttcttggtgt acaaacagaa tggtaaattg 420
aattttgatg aaccaagatt gaccaatcga tccggggata atagaggtgg attttctatt 480
agaaagtttg cagcaaaata taatcttggg caacctgttg ctggcaattt gtaccaagct 540
gagtatgatg attatgttcc aattttgtac aagcaattgg gaggttaa 588

<210> 48
<211> 195
<212> PRT
<213> Zea mays

<400> 48
Ile Leu Arg Arg Pro Val Val Ala Arg Leu Tyr Ser Thr Leu Ser Lys
1               5                   10                  15
Ser Met Glu Lys His Gly Val Val Pro Asp Val Ile Asp Val Ala Pro
            20                  25                  30
Glu Gln Gln Val Glu Val Ser Tyr Pro Ser Gly Val Lys Val Asp Phe
        35                  40                  45
Gly Asn Glu Leu Thr Pro Thr Gln Val Lys Asp Ile Pro Ala Val Lys
    50                  55                  60
Trp Pro Ala Asp Lys Asp Ser Leu Tyr Thr Leu Cys Met Thr Asp Pro
65                  70                  75                  80
Asp Ala Pro Ser Arg Lys Glu Pro Lys Phe Arg Glu Trp His His Trp
                85                  90                  95
Leu Val Gly Asn Ile Pro Gly Gly Glu Val Ser Lys Gly Glu Val Leu
            100                 105                 110
Ser Glu Tyr Val Gly Ser Gly Pro Pro Pro Asn Thr Gly Leu His Arg
        115                 120                 125
Tyr Val Phe Leu Val Tyr Lys Gln Asn Gly Lys Leu Asn Phe Asp Glu
    130                 135                 140
Pro Arg Leu Thr Asn Arg Ser Gly Asp Asn Arg Gly Gly Phe Ser Ile
145                 150                 155                 160
Arg Lys Phe Ala Ala Lys Tyr Asn Leu Gly Gln Pro Val Ala Gly Asn
                165                 170                 175
Leu Tyr Gln Ala Glu Tyr Asp Asp Tyr Val Pro Ile Leu Tyr Lys Gln
            180                 185                 190
Leu Gly Gly
        195

<210> 49
<211> 333
<212> DNA
<213> Zea mays

<400> 49
gtgatggtgg atccagactc cccaagtcca agtaacccaa caaaaagaga ataccttcat 60
tggttggtga cagacatccc ggaatcagca aatgctagct atggaaacga aatcgtcagc 120
tatgaaaacc caaagccaac tgctggaata catcgctttg tctttgttct cttccgccag 180
tctgtccagc aaaccgttta tgcaccagga tggagacaaa atttcaacac gagagacttt 240
tctgcgttct ataatcttgg acctcctgtg gctgcagtgt tcttcaattg tcaaagggag 300
aatgggtgtg gaggcagacg atatattaga taa 333

<210> 50
<211> 110
<212> PRT
<213> Zea mays

<400> 50
Val Met Val Asp Pro Asp Ser Pro Ser Pro Ser Asn Pro Thr Lys Arg
1               5                   10                  15
Glu Tyr Leu His Trp Leu Val Thr Asp Ile Pro Glu Ser Ala Asn Ala
            20                  25                  30
Ser Tyr Gly Asn Glu Ile Val Ser Tyr Glu Asn Pro Lys Pro Thr Ala
        35                  40                  45
Gly Ile His Arg Phe Val Phe Val Leu Phe Arg Gln Ser Val Gln Gln
    50                  55                  60
Thr Val Tyr Ala Pro Gly Trp Arg Gln Asn Phe Asn Thr Arg Asp Phe
65                  70                  75                  80
Ser Ala Phe Tyr Asn Leu Gly Pro Pro Val Ala Ala Val Phe Phe Asn
                85                  90                  95
Cys Gln Arg Glu Asn Gly Cys Gly Gly Arg Arg Tyr Ile Arg
            100                 105                 110

<210> 51
<211> 267
<212> DNA
<213> Zea mays

<400> 51
cgagagctca taccatatga gagcccaagc cccaccatgg gcatccaccg tcttgtgttg 60
gtgctctacc agcaattggg gcggggcacg gtgtttgcgc cgcaagttcg tcagaacttc 120
aacttgcgta atttcgcacg ccgtttcaac ctcggcaagc ctgtggccgc gacgtacttc 180
aactgtcagc ggcaaacagg cacaggtggg agaaggttca cttgtgtttt tgatcatgtc 240
gttcaaggtg aaggccggca agcttga                                     267

<210> 52
<211> 88
<212> PRT
<213> Zea mays

<400> 52
Arg Glu Leu Ile Pro Tyr Glu Ser Pro Ser Pro Thr Met Gly Ile His
1               5                   10                  15
Arg Leu Val Leu Val Leu Tyr Gln Gln Leu Gly Arg Gly Thr Val Phe
            20                  25                  30
Ala Pro Gln Val Arg Gln Asn Phe Asn Leu Arg Asn Phe Ala Arg Arg
        35                  40                  45
Phe Asn Leu Gly Lys Pro Val Ala Ala Thr Tyr Phe Asn Cys Gln Arg
    50                  55                  60
Gln Thr Gly Thr Gly Gly Arg Arg Phe Thr Cys Val Phe Asp His Val
65                  70                  75                  80
Val Gln Gly Glu Gly Arg Gln Ala
                85

<210> 53
<211> 228
<212> DNA
<213> Zea mays

<400> 53
aatgaaattg tcagctatga aaacccaaag ccatctgctg gaatacatcg ctttgtcttt 60

gtactcttcc gccagtctgt acagcaaacc gtttatgcac caggatggag acaaaatttc 120
aacacgagag acttttctgc gctctataat cttggacctc cagtggctgc agttttcttc 180
aattgtcaaa gggagaatgg gtgtggagga agacgatata ttagataa 228

<210> 54
<211> 75
<212> PRT
<213> Zea mays

<400> 54
Asn Glu Ile Val Ser Tyr Glu Asn Pro Lys Pro Ser Ala Gly Ile His
1               5                   10                  15
Arg Phe Val Phe Val Leu Phe Arg Gln Ser Val Gln Gln Thr Val Tyr
            20                  25                  30
Ala Pro Gly Trp Arg Gln Asn Phe Asn Thr Arg Asp Phe Ser Ala Leu
        35                  40                  45
Tyr Asn Leu Gly Pro Pro Val Ala Ala Val Phe Phe Asn Cys Gln Arg
    50                  55                  60
Glu Asn Gly Cys Gly Gly Arg Arg Tyr Ile Arg
65                  70                  75

<210> 55
<211> 192
<212> DNA
<213> Zea mays

<400> 55
atggctaatg actccttgac gaggggccac ataatcgggg atgtcttaga cccgtttact 60
agctcagtgt ctctaagtgt cctgtatgat ggcagaccag tgtttgatgg gatggagttt 120
cgggcgtcgg cggtgtcggt gaaacctaga gttgagattg gaggtgatga ttttcgagtg 180
gcctataccc ta 192

<210> 56
<211> 64
<212> PRT
<213> Zea mays

<400> 56
Met Ala Asn Asp Ser Leu Thr Arg Gly His Ile Ile Gly Asp Val Leu
1               5                   10                  15
Asp Pro Phe Thr Ser Ser Val Ser Leu Ser Val Leu Tyr Asp Gly Arg
            20                  25                  30
Pro Val Phe Asp Gly Met Glu Phe Arg Ala Ser Ala Val Ser Val Lys
        35                  40                  45
Pro Arg Val Glu Ile Gly Gly Asp Asp Phe Arg Val Ala Tyr Thr Leu
    50                  55                  60

<210> 57
<211> 540
<212> DNA
<213> Oryza sativa

<400> 57
atggccggaa gtggcaggga cagggaccct cttgtggttg gtagggttgt gggtgatgtg 60
ctggacgcgt tcgtccggag caccaacctc aaggtcacct atggctccaa gaccgtgtcc 120
aatggctgcg agctcaagcc gtccatggtc acccaccagc ctagggtcga ggtcggcggc 180
aatgacatga ggacattcta caccettgtg atggtagacc cagatgcacc aagcccaagt 240
gaccctaacc ttagggagta tctacattgg ttggtcactg atattcctgg tactactgca 300
gcgtcatttg ggcaagaggt gatgtgctac gagagcccaa ggccaaccat ggggatccac 360

cggctggtgt tcgtgctgtt ccagcagctg gggcgtcaga cagtgtacgc gcccgggtgg 420
cgtcagaact tcaacaccaa ggacttcgcc gagctctaca acctcggctc gccggtcgcc 480
gccgtctact tcaactgcca gcgcgaggca ggctccggcg gcaggagggt ctacccctag 540

<210> 58
<211> 179
<212> PRT
<213> Oryza sativa

<400> 58
Met Ala Gly Ser Gly Arg Asp Arg Asp Pro Leu Val Val Gly Arg Val
1               5                   10                  15
Val Gly Asp Val Leu Asp Ala Phe Val Arg Ser Thr Asn Leu Lys Val
            20                  25                  30
Thr Tyr Gly Ser Lys Thr Val Ser Asn Gly Cys Glu Leu Lys Pro Ser
        35                  40                  45
Met Val Thr His Gln Pro Arg Val Glu Val Gly Gly Asn Asp Met Arg
    50                  55                  60
Thr Phe Tyr Thr Leu Val Met Val Asp Pro Asp Ala Pro Ser Pro Ser
65                  70                  75                  80
Asp Pro Asn Leu Arg Glu Tyr Leu His Trp Leu Val Thr Asp Ile Pro
                85                  90                  95
Gly Thr Thr Ala Ala Ser Phe Gly Gln Glu Val Met Cys Tyr Glu Ser
            100                 105                 110
Pro Arg Pro Thr Met Gly Ile His Arg Leu Val Phe Val Leu Phe Gln
        115                 120                 125
Gln Leu Gly Arg Gln Thr Val Tyr Ala Pro Gly Trp Arg Gln Asn Phe
    130                 135                 140
Asn Thr Lys Asp Phe Ala Glu Leu Tyr Asn Leu Gly Ser Pro Val Ala
145                 150                 155                 160
Ala Val Tyr Phe Asn Cys Gln Arg Glu Ala Gly Ser Gly Gly Arg Arg
                165                 170                 175
Val Tyr Pro

<210> 59
<211> 534
<212> DNA
<213> Oryza sativa

<400> 59
atgagcatgt cgagggaccc gctggtggtg gggagcatcg tcggcgacgt ggtggaccac 60
ttcggcgcgt cggcgctgct gaggctgttc tacaaccacc gcgagatgac gagcgggtcg 120
gagctcaggc cgtcgcaggt cgccggcgag ccggccgtcc agatcaccgg aggccgcgat 180
gggagggcgc tctacacgct cgtaatggtg gaccctgatg cacctagccc cagcaaccct 240
tccaaaaggg aataccttca ttggttggta actgacgtac cagaaggagg cgatacgagt 300
aaagggacgg aggtggtggc gtacgagagc ccgcggccga cagcggggat ccaccggttg 360
gtgttcatcg tgttccggca gacagtgcgg cagtccatct acgcgccggg gtggcgctcc 420
aacttcaaca ccagggactt cgccgcctgc tacagcctcg gctcccccgt cgccgccgcc 480
tacttcaact gccagaggga gggcggctgc ggcggccgga ggtacaggtc atga       534

<210> 60
<211> 177
<212> PRT
<213> Oryza sativa

<400> 60
Met Ser Met Ser Arg Asp Pro Leu Val Val Gly Ser Ile Val Gly Asp
1               5                   10                  15

Val Val Asp His Phe Gly Ala Ser Ala Leu Leu Arg Leu Phe Tyr Asn
            20                  25                  30
His Arg Glu Met Thr Ser Gly Ser Glu Leu Arg Pro Ser Gln Val Ala
        35                  40                  45
Gly Glu Pro Ala Val Gln Ile Thr Gly Gly Arg Asp Gly Arg Ala Leu
    50                  55                  60
Tyr Thr Leu Val Met Val Asp Pro Asp Ala Pro Ser Pro Ser Asn Pro
65                  70                  75                  80
Ser Lys Arg Glu Tyr Leu His Trp Leu Val Thr Asp Val Pro Glu Gly
                85                  90                  95
Gly Asp Thr Ser Lys Gly Thr Glu Val Val Ala Tyr Glu Ser Pro Arg
            100                 105                 110
Pro Thr Ala Gly Ile His Arg Leu Val Phe Ile Val Phe Arg Gln Thr
        115                 120                 125
Val Arg Gln Ser Ile Tyr Ala Pro Gly Trp Arg Ser Asn Phe Asn Thr
    130                 135                 140
Arg Asp Phe Ala Ala Cys Tyr Ser Leu Gly Ser Pro Val Ala Ala Ala
145                 150                 155                 160
Tyr Phe Asn Cys Gln Arg Glu Gly Gly Cys Gly Gly Arg Arg Tyr Arg
                165                 170                 175
Ser

<210> 61
<211> 522
<212> DNA
<213> Oryza sativa

<400> 61
atgtcacgag gtagggatcc tttggcattg agccaggtaa ttggcgatgt gttggatcct 60
ttcataaagt cagctgcaat gaggattaat tatggtgaga aggagattac aaatggaact 120
ggagtacgat catctgctgt tttcactgca ccacatgttg agattgaagg tcgtgaccaa 180
acgaagctct acacacttgt tatggtggat cctgatgcgc caagtccaag caaaccagaa 240
tacagggaat atttgcattg gttggtgaca gacatcccag aggcaataga tgcacgtttt 300
ggcaatgaaa tagttccgta cgaagctcca cggccaccgg ctggaattca tcggcttgtt 360
tttgtgctat tcaaacagga agcacgacaa acagtttatg ctccaggatg gcggcaaaat 420
ttcaacgtca gagatttctc tgcattttac aatcttggac cacctgttgc tgcattatac 480
ttcaactgcc agaaggagag tggtgttggt ggcagaaggt ag 522

<210> 62
<211> 173
<212> PRT
<213> Oryza sativa

<400> 62
Met Ser Arg Gly Arg Asp Pro Leu Ala Leu Ser Gln Val Ile Gly Asp
1               5                   10                  15
Val Leu Asp Pro Phe Ile Lys Ser Ala Ala Met Arg Ile Asn Tyr Gly
            20                  25                  30
Glu Lys Glu Ile Thr Asn Gly Thr Gly Val Arg Ser Ser Ala Val Phe
        35                  40                  45
Thr Ala Pro His Val Glu Ile Glu Gly Arg Asp Gln Thr Lys Leu Tyr
    50                  55                  60
Thr Leu Val Met Val Asp Pro Asp Ala Pro Ser Pro Ser Lys Pro Glu
65                  70                  75                  80
Tyr Arg Glu Tyr Leu His Trp Leu Val Thr Asp Ile Pro Glu Ala Ile
                85                  90                  95
Asp Ala Arg Phe Gly Asn Glu Ile Val Pro Tyr Glu Ala Pro Arg Pro
            100                 105                 110

Pro Ala Gly Ile His Arg Leu Val Phe Val Leu Phe Lys Gln Glu Ala
        115                 120                 125
Arg Gln Thr Val Tyr Ala Pro Gly Trp Arg Gln Asn Phe Asn Val Arg
    130                 135                 140
Asp Phe Ser Ala Phe Tyr Asn Leu Gly Pro Pro Val Ala Ala Leu Tyr
145                 150                 155                 160
Phe Asn Cys Gln Lys Glu Ser Gly Val Gly Gly Arg Arg
                165                 170

<210> 63
<211> 522
<212> DNA
<213> Oryza sativa

<400> 63
atgtctaggg tgctggagcc tctcgtcgtc gggaaggtga tcggagaggt catcgacaac 60
ttcaacccca cggtgaagat gacggcgacc tacagctcca acaagcaggt gttcaacggc 120
cacgagttat tcccgtcggc ggtcgtgtcc aagccgcgag tcgaggttca gggcggcgac 180
ctgaggtctt tcttcacact ggttatgaca gatccagacg tgccagggcc tagtgatccg 240
tacctgaggg agcacctcca ctggatcgtc actgatattc ctggcaccac tgatgcttcc 300
tttgggaggg aggtggtgag ctacgagagc ccgaagccca acattggcat ccacaggttc 360
gtcctcgtgc tgttcaagca gaagcgccgt caggcggtga ccccgccatc ctccagggac 420
tacttcagca cccgccgctt cgccgccgac aacgacctcg gcctccccgt cgccgccgtc 480
tacttcaacg cgcagcgaga gacggccgct cgccgccgct aa                    522

<210> 64
<211> 173
<212> PRT
<213> Oryza sativa

<400> 64
Met Ser Arg Val Leu Glu Pro Leu Val Val Gly Lys Val Ile Gly Glu
 1               5                  10                  15
Val Ile Asp Asn Phe Asn Pro Thr Val Lys Met Thr Ala Thr Tyr Ser
            20                  25                  30
Ser Asn Lys Gln Val Phe Asn Gly His Glu Leu Phe Pro Ser Ala Val
        35                  40                  45
Val Ser Lys Pro Arg Val Glu Val Gln Gly Gly Asp Leu Arg Ser Phe
    50                  55                  60
Phe Thr Leu Val Met Thr Asp Pro Asp Val Pro Gly Pro Ser Asp Pro
65                  70                  75                  80
Tyr Leu Arg Glu His Leu His Trp Ile Val Thr Asp Ile Pro Gly Thr
                85                  90                  95
Thr Asp Ala Ser Phe Gly Arg Glu Val Val Ser Tyr Glu Ser Pro Lys
            100                 105                 110
Pro Asn Ile Gly Ile His Arg Phe Val Leu Val Leu Phe Lys Gln Lys
        115                 120                 125
Arg Arg Gln Ala Val Thr Pro Pro Ser Ser Arg Asp Tyr Phe Ser Thr
    130                 135                 140
Arg Arg Phe Ala Ala Asp Asn Asp Leu Gly Leu Pro Val Ala Ala Val
145                 150                 155                 160
Tyr Phe Asn Ala Gln Arg Glu Thr Ala Ala Arg Arg Arg
                165                 170

<210> 65
<211> 522
<212> DNA
<213> Oryza sativa

<400> 65
atgtctaggg tgctggagcc tctcgtcgtc gggaaggtga tcggagaggt catcgacaac 60
ttcaacccca cggtgaagat gacggcgacc tacagctcca acaagcaggt gttcaacggc 120
cacgagttat tcccgtcggc ggtcgtgtcc aagccgcgag tcgaggttca gggcggcgac 180
ctgaggtctt tcttcacact ggttatgaca gatccagacg tgccagggcc tagtgatccg 240
tacctgaggg agcacctcca ctggatcgtc actgatattc ctggcaccac tgatgcttcc 300
tttgggaggg aggtggtgag ctacgagagc ccgaagccca acattggcat ccacaggttc 360
gtcctcgtgc tgttcaagca gaagcgccgt caggcggtga ccccgccatc ctccagggac 420
tacttcagca cccgccgctt cgccgccgac aacgacctcg gcctccccgt cgccgccgtc 480
tacttcaacg cgcagcgaga gacggccgct cgccgccgct aa 522

<210> 66
<211> 173
<212> PRT
<213> Oryza sativa

<400> 66
Met Ser Arg Val Leu Glu Pro Leu Val Val Gly Lys Val Ile Gly Glu
1               5                   10                  15
Val Ile Asp Asn Phe Asn Pro Thr Val Lys Met Thr Ala Thr Tyr Ser
            20                  25                  30
Ser Asn Lys Gln Val Phe Asn Gly His Glu Leu Phe Pro Ser Ala Val
        35                  40                  45
Val Ser Lys Pro Arg Val Glu Val Gln Gly Gly Asp Leu Arg Ser Phe
    50                  55                  60
Phe Thr Leu Val Met Thr Asp Pro Asp Val Pro Gly Pro Ser Asp Pro
65                  70                  75                  80
Tyr Leu Arg Glu His Leu His Trp Ile Val Thr Asp Ile Pro Gly Thr
                85                  90                  95
Thr Asp Ala Ser Phe Gly Arg Glu Val Val Ser Tyr Glu Ser Pro Lys
            100                 105                 110
Pro Asn Ile Gly Ile His Arg Phe Val Leu Val Leu Phe Lys Gln Lys
        115                 120                 125
Arg Arg Gln Ala Val Thr Pro Pro Ser Ser Arg Asp Tyr Phe Ser Thr
    130                 135                 140
Arg Arg Phe Ala Ala Asp Asn Asp Leu Gly Leu Pro Val Ala Ala Val
145                 150                 155                 160
Tyr Phe Asn Ala Gln Arg Glu Thr Ala Ala Arg Arg Arg
                165                 170

<210> 67
<211> 525
<212> DNA
<213> Oryza sativa

<400> 67
atgtcaaggg acccacttgt cgtaggacat gttgttgggg atatcttaga cccattcaac 60
aaatcagcat cactcaaggt cctatacaac aacaaggaat taacaaatgg gtctgagctc 120
aaaccgtcac aggtagcaaa tgaaccaagg attgaaattg ctggccgcga cataaggaac 180
ctttacactc tggtgatggt ggatcctgac tcgccaagtc caagcaaccc aacaaaaaga 240
gaataccttc attggttggt gacagacatt ccagaatcgg caaatgctag ttatggaaat 300
gaagttgtca gttatgaaag cccaaaacca actgcaggga tacatcgttt tgtctttata 360
ttatttcgcc aatatgtaca acagactatt tatgcaccag gatggagacc aaatttcaat 420
acaagagatt tttccgcact gtataatctt ggacctcctg tggcagcagt gttcttcaat 480
tgccagaggg agaacggatg tggaggcaga cggtacatta gataa 525

<210> 68
<211> 174
<212> PRT
<213> Oryza sativa

<400> 68
Met Ser Arg Asp Pro Leu Val Val Gly His Val Val Gly Asp Ile Leu
1               5                   10                  15
Asp Pro Phe Asn Lys Ser Ala Ser Leu Lys Val Leu Tyr Asn Asn Lys
            20                  25                  30
Glu Leu Thr Asn Gly Ser Glu Leu Lys Pro Ser Gln Val Ala Asn Glu
        35                  40                  45
Pro Arg Ile Glu Ile Ala Gly Arg Asp Ile Arg Asn Leu Tyr Thr Leu
    50                  55                  60
Val Met Val Asp Pro Asp Ser Pro Ser Pro Ser Asn Pro Thr Lys Arg
65                  70                  75                  80
Glu Tyr Leu His Trp Leu Val Thr Asp Ile Pro Glu Ser Ala Asn Ala
                85                  90                  95
Ser Tyr Gly Asn Glu Val Val Ser Tyr Glu Ser Pro Lys Pro Thr Ala
            100                 105                 110
Gly Ile His Arg Phe Val Phe Ile Leu Phe Arg Gln Tyr Val Gln Gln
        115                 120                 125
Thr Ile Tyr Ala Pro Gly Trp Arg Pro Asn Phe Asn Thr Arg Asp Phe
    130                 135                 140
Ser Ala Leu Tyr Asn Leu Gly Pro Pro Val Ala Ala Val Phe Phe Asn
145                 150                 155                 160
Cys Gln Arg Glu Asn Gly Cys Gly Gly Arg Arg Tyr Ile Arg
                165                 170

<210> 69
<211> 543
<212> DNA
<213> Oryza sativa

<400> 69
atgtcgtcgg cgaacagcct ggtgctgggg cgggtgatcg gcgacgtggt ggacctgttc 60
tcgccggagg tgacgctccg ggtgatgtac aacggcgtgc gggtcgtcaa cggcgaggac 120
ctccggccgt cggcggtgtc ggcgaggccc agcgtcgagg tcggagggga tctccaccag 180
ttctacacga tcgtgatggt ggatccagat gctccaaacc caagcaatcc gacgttgaga 240
gagtacttac actggttggt gacagatatt cctggaacaa ctgatgcgaa ctatgggcgc 300
gaggtggtgt gctacgagag cccccggcca gcggcgggga tccaccgggt ggcggtggtg 360
ctgttccggc agatggcgcg cggcggcgtg gaccagccgc cgctgctccg ccacaacttc 420
tccacccgcg gcttcgccga cgaccacgcc ctcggcgccc ccgtcgccgc cgccttcttc 480
acctgcaagc ccgagggcgg caccggcggc cgccgcttcc ggccaccgtc acggcatagc 540
tag                                                               543

<210> 70
<211> 180
<212> PRT
<213> Oryza sativa

<400> 70
Met Ser Ser Ala Asn Ser Leu Val Leu Gly Arg Val Ile Gly Asp Val
1               5                   10                  15
Val Asp Leu Phe Ser Pro Glu Val Thr Leu Arg Val Met Tyr Asn Gly
            20                  25                  30
Val Arg Val Val Asn Gly Glu Asp Leu Arg Pro Ser Ala Val Ser Ala
        35                  40                  45
Arg Pro Ser Val Glu Val Gly Gly Asp Leu His Gln Phe Tyr Thr Ile
    50                  55                  60
Val Met Val Asp Pro Asp Ala Pro Asn Pro Ser Asn Pro Thr Leu Arg
65                  70                  75                  80
Glu Tyr Leu His Trp Leu Val Thr Asp Ile Pro Gly Thr Thr Asp Ala
                85                  90                  95

Asn Tyr Gly Arg Glu Val Val Cys Tyr Glu Ser Pro Arg Pro Ala Ala
            100                 105                 110
Gly Ile His Arg Val Ala Val Val Leu Phe Arg Gln Met Ala Arg Gly
        115                 120                 125
Gly Val Asp Gln Pro Pro Leu Leu Arg His Asn Phe Ser Thr Arg Gly
    130                 135                 140
Phe Ala Asp Asp His Ala Leu Gly Ala Pro Val Ala Ala Ala Phe Phe
145                 150                 155                 160
Thr Cys Lys Pro Glu Gly Gly Thr Gly Gly Arg Arg Phe Arg Pro Pro
                165                 170                 175
Ser Arg His Ser
            180

<210> 71
<211> 522
<212> DNA
<213> Oryza sativa

<400> 71
atgtcgaggg tgctggagcc tctcattgtg gggaaggtga tcggcgaggt gctggacaac 60
ttcaacccca cggtgaagat gacggccacc tacggcgcca acaagcaggt gttcaacggc 120
cacgagttct tcccctccgc cgtcgccggc aagccgcgcg tcgaggtcca gggcggcgac 180
ctcaggtcct tcttcacatt ggtgatgact gaccctgatg tgccagggcc tagtgatcca 240
tacctgaggg agcatcttca ctggattgtt actgatattc ctgggactac tgatgcctct 300
tttgggaggg aggtggtgag ctacgagagc ccgcggccaa acatcggcat ccacaggttc 360
atcctggtgc tgttccggca gaagcgccgg caggcggtga gcccgccgcc gtcgagggac 420
cgcttcagca cccgccagtt cgccgaggac aacgacctcg gcctccccgt cgccgccgtc 480
tacttcaacg cgcagcgcga gaccgccgct cgccgccgct aa                    522

<210> 72
<211> 173
<212> PRT
<213> Oryza sativa

<400> 72
Met Ser Arg Val Leu Glu Pro Leu Ile Val Gly Lys Val Ile Gly Glu
 1               5                  10                  15
Val Leu Asp Asn Phe Asn Pro Thr Val Lys Met Thr Ala Thr Tyr Gly
            20                  25                  30
Ala Asn Lys Gln Val Phe Asn Gly His Glu Phe Phe Pro Ser Ala Val
        35                  40                  45
Ala Gly Lys Pro Arg Val Glu Val Gln Gly Gly Asp Leu Arg Ser Phe
    50                  55                  60
Phe Thr Leu Val Met Thr Asp Pro Asp Val Pro Gly Pro Ser Asp Pro
65                  70                  75                  80
Tyr Leu Arg Glu His Leu His Trp Ile Val Thr Asp Ile Pro Gly Thr
                85                  90                  95
Thr Asp Ala Ser Phe Gly Arg Glu Val Val Ser Tyr Glu Ser Pro Arg
            100                 105                 110
Pro Asn Ile Gly Ile His Arg Phe Ile Leu Val Leu Phe Arg Gln Lys
        115                 120                 125
Arg Arg Gln Ala Val Ser Pro Pro Pro Ser Arg Asp Arg Phe Ser Thr
    130                 135                 140
Arg Gln Phe Ala Glu Asp Asn Asp Leu Gly Leu Pro Val Ala Ala Val
145                 150                 155                 160
Tyr Phe Asn Ala Gln Arg Glu Thr Ala Ala Arg Arg Arg
                165                 170

<210> 73

<211> 537
<212> DNA
<213> Oryza sativa

<400> 73
atggccggca gcggcaggga cgatcctctt gtggttggca ggattgtggg tgatgtgctg 60
gatccattcg tccggatcac taacctcagt gtcagctatg gtgcaaggat cgtctccaat 120
ggctgcgagc tcaagccgtc catggtgacc caacagccca gggtcgtggt cggtggcaat 180
gacatgagga cgttctacac actcgtgatg gtagacccgg atgctccgag cccaagcaac 240
cctaacctta gggagtatct acactggctg gtcaccgata ttcctggtac cactggagca 300
acatttggcc aagaggtgat gtgctacgag agcccaaggc caaccatggg gatccaccgg 360
ctggtgttcg tgctgttcca gcagctgggg cgtcagacgg tgtacgcacc ggggtggcgc 420
cagaacttca gcaccaggaa cttcgccgag ctctacaacc tcggctcgcc ggtcgccacc 480
gtctacttca actgccagcg cgaggccggc tccggcggca ggagggtcta cccctag 537

<210> 74
<211> 178
<212> PRT
<213> Oryza sativa

<400> 74
Met Ala Gly Ser Gly Arg Asp Asp Pro Leu Val Val Gly Arg Ile Val
1               5                   10                  15
Gly Asp Val Leu Asp Pro Phe Val Arg Ile Thr Asn Leu Ser Val Ser
            20                  25                  30
Tyr Gly Ala Arg Ile Val Ser Asn Gly Cys Glu Leu Lys Pro Ser Met
        35                  40                  45
Val Thr Gln Gln Pro Arg Val Val Val Gly Gly Asn Asp Met Arg Thr
    50                  55                  60
Phe Tyr Thr Leu Val Met Val Asp Pro Asp Ala Pro Ser Pro Ser Asn
65                  70                  75                  80
Pro Asn Leu Arg Glu Tyr Leu His Trp Leu Val Thr Asp Ile Pro Gly
                85                  90                  95
Thr Thr Gly Ala Thr Phe Gly Gln Glu Val Met Cys Tyr Glu Ser Pro
            100                 105                 110
Arg Pro Thr Met Gly Ile His Arg Leu Val Phe Val Leu Phe Gln Gln
        115                 120                 125
Leu Gly Arg Gln Thr Val Tyr Ala Pro Gly Trp Arg Gln Asn Phe Ser
    130                 135                 140
Thr Arg Asn Phe Ala Glu Leu Tyr Asn Leu Gly Ser Pro Val Ala Thr
145                 150                 155                 160
Val Tyr Phe Asn Cys Gln Arg Glu Ala Gly Ser Gly Gly Arg Arg Val
                165                 170                 175
Tyr Pro

<210> 75
<211> 552
<212> DNA
<213> Oryza sativa

<400> 75
atgtctggtg tgccaactgt ggagcccttg gttttggctc atgtcataca tgacgtgtta 60
gatccattta gaccaactat gccccttaga ataacataca acgataggtt acttctggca 120
ggtgctgagc tgaaaccatc tgcaactgtg cataaaccaa gagtagatat tggtggcacc 180
gacctgaggg tgttctacac attggtactg gtggatccag atgctccaag cccaagcaac 240
ccatcactag gggagtattt gcactatctc cactggatgg tgatagatat cccaggaaca 300
actgagtcaa ctttatccca agacctcatg ctttatgaaa gaccggaact gagatatggt 360
atccaccgga tggtatttgt gttattccga caacttggca ggggaaccgt ttttgcacca 420
gagatgcgac acaacttcca ttgtagaagc tttgcgcaac aataccatct ggacattgtg 480

gccgctacat atttcaactg ccaaagggaa gccggctctg gtggaagaag gttcaggtcc 540
gagagttctt aa 552

<210> 76
<211> 183
<212> PRT
<213> Oryza sativa

<400> 76
Met Ser Gly Val Pro Thr Val Glu Pro Leu Val Leu Ala His Val Ile
1               5                   10                  15
His Asp Val Leu Asp Pro Phe Arg Pro Thr Met Pro Leu Arg Ile Thr
            20                  25                  30
Tyr Asn Asp Arg Leu Leu Leu Ala Gly Ala Glu Leu Lys Pro Ser Ala
        35                  40                  45
Thr Val His Lys Pro Arg Val Asp Ile Gly Gly Thr Asp Leu Arg Val
    50                  55                  60
Phe Tyr Thr Leu Val Leu Val Asp Pro Asp Ala Pro Ser Pro Ser Asn
65                  70                  75                  80
Pro Ser Leu Gly Glu Tyr Leu His Tyr Leu His Trp Met Val Ile Asp
                85                  90                  95
Ile Pro Gly Thr Thr Glu Ser Thr Leu Ser Gln Asp Leu Met Leu Tyr
            100                 105                 110
Glu Arg Pro Glu Leu Arg Tyr Gly Ile His Arg Met Val Phe Val Leu
        115                 120                 125
Phe Arg Gln Leu Gly Arg Gly Thr Val Phe Ala Pro Glu Met Arg His
    130                 135                 140
Asn Phe His Cys Arg Ser Phe Ala Gln Gln Tyr His Leu Asp Ile Val
145                 150                 155                 160
Ala Ala Thr Tyr Phe Asn Cys Gln Arg Glu Ala Gly Ser Gly Gly Arg
                165                 170                 175
Arg Phe Arg Ser Glu Ser Ser
            180

<210> 77
<211> 531
<212> DNA
<213> Oryza sativa

<400> 77
atgagcgggc gggggagggg ggacccgctg gtgctgggga gggtggtggg ggacgtggtg 60
gacccgttcg tgaggagggt ggcgctgcgg gtggcgtacg gagcgcggga ggtggccaac 120
ggctgcgagc tccgcccctc cgccgtcgcc gaccagcccc gcgtcgccgt cggcggcccc 180
gacatgcgca ccttctacac cctggtgatg gtggatccgg acgcgccgag cccgagcgat 240
ccaaacctca gggagtacct gcactggctg gtcaccgaca tcccggctac cacaggagtc 300
tcttttggga cagaggtggt gtgctacgag agcccgcggc cggtgctggg gatccacagg 360
ctggtgttcc tgctgttcga gcagctgggg cggcagacgg tgtacgcacc ggggtggcgc 420
cagaacttca gcacccgcga cttcgccgag ctctacaacc tcggcctccc tgtcgccgcc 480
gtctacttca actgccagag ggagtctgga accggaggaa gaagaatgtg a 531

<210> 78
<211> 176
<212> PRT
<213> Oryza sativa

<400> 78
Met Ser Gly Arg Gly Arg Gly Asp Pro Leu Val Leu Gly Arg Val Val
1               5                   10                  15
Gly Asp Val Val Asp Pro Phe Val Arg Arg Val Ala Leu Arg Val Ala
            20                  25                  30

Tyr Gly Ala Arg Glu Val Ala Asn Gly Cys Glu Leu Arg Pro Ser Ala
        35                  40                  45
Val Ala Asp Gln Pro Arg Val Ala Val Gly Gly Pro Asp Met Arg Thr
    50                  55                  60
Phe Tyr Thr Leu Val Met Val Asp Pro Asp Ala Pro Ser Pro Ser Asp
65                  70                  75                  80
Pro Asn Leu Arg Glu Tyr Leu His Trp Leu Val Thr Asp Ile Pro Ala
                85                  90                  95
Thr Thr Gly Val Ser Phe Gly Thr Glu Val Val Cys Tyr Glu Ser Pro
            100                 105                 110
Arg Pro Val Leu Gly Ile His Arg Leu Val Phe Leu Leu Phe Glu Gln
        115                 120                 125
Leu Gly Arg Gln Thr Val Tyr Ala Pro Gly Trp Arg Gln Asn Phe Ser
    130                 135                 140
Thr Arg Asp Phe Ala Glu Leu Tyr Asn Leu Gly Leu Pro Val Ala Ala
145                 150                 155                 160
Val Tyr Phe Asn Cys Gln Arg Glu Ser Gly Thr Gly Gly Arg Arg Met
                165                 170                 175

<210> 79
<211> 525
<212> DNA
<213> Oryza sativa

<400> 79
atggcccgtt tcgtggatcc gctggtggtg ggacgggtga tcggggaggt ggtggatttg 60
ttcgttccat ccatctccat gaccgccgcc tacggcgaca gggacatcag caacggctgc 120
ctcgtccgcc catccgccgc cgactaccct cccctcgtcc gcatctccgg ccgccgcaac 180
gacctctaca ccctgatcat gacggacccg gacgcaccta gccctagcga cccatccatg 240
agggagtttc tccactggat cgtggttaac ataccggggg gaacagatgc atctaaaggt 300
gaggagatgg tggagtacat ggggccacgg ccgacggtgg ggatacacag gtacgtgctg 360
gtgctgtacg agcagaaggc gcgcttcgtg gacggcgcgc tgatgccgcc ggcggacagg 420
cccaacttca acacaagagc attcgcggcg taccatcagc tcggcctccc caccgccgtc 480
gtccacttca actcccagag ggagcccgcc aaccgccgcc gctaa                 525

<210> 80
<211> 174
<212> PRT
<213> Oryza sativa

<400> 80
Met Ala Arg Phe Val Asp Pro Leu Val Val Gly Arg Val Ile Gly Glu
 1               5                  10                  15
Val Val Asp Leu Phe Val Pro Ser Ile Ser Met Thr Ala Ala Tyr Gly
            20                  25                  30
Asp Arg Asp Ile Ser Asn Gly Cys Leu Val Arg Pro Ser Ala Ala Asp
        35                  40                  45
Tyr Pro Pro Leu Val Arg Ile Ser Gly Arg Arg Asn Asp Leu Tyr Thr
    50                  55                  60
Leu Ile Met Thr Asp Pro Asp Ala Pro Ser Pro Ser Asp Pro Ser Met
65                  70                  75                  80
Arg Glu Phe Leu His Trp Ile Val Val Asn Ile Pro Gly Gly Thr Asp
                85                  90                  95
Ala Ser Lys Gly Glu Glu Met Val Glu Tyr Met Gly Pro Arg Pro Thr
            100                 105                 110
Val Gly Ile His Arg Tyr Val Leu Val Leu Tyr Glu Gln Lys Ala Arg
        115                 120                 125
Phe Val Asp Gly Ala Leu Met Pro Pro Ala Asp Arg Pro Asn Phe Asn
    130                 135                 140

Thr Arg Ala Phe Ala Ala Tyr His Gln Leu Gly Leu Pro Thr Ala Val
145                 150                 155                 160
Val His Phe Asn Ser Gln Arg Glu Pro Ala Asn Arg Arg Arg
                165                 170

<210> 81
<211> 558
<212> DNA
<213> Oryza sativa

<400> 81
atggccaacg attcattggc tacagggcgt gtgatcggag atgtcctgga tcccttcatc 60
agcaccgtcg atctcaccgt catgtatggt gatgatggca tgccggtcat aagcggcgtg 120
gagcttcgcg caccggcggt cgcggagaaa ccggtggtcg aagtcggggg agacgatctt 180
cgcgtcgcat atactctggt gatggttgat cctgatgcac ctaaccctag caatccaact 240
ctgagggaat acctccactg gatggtgact gacatcccgg cttcaaccga tgctacatat 300
gggagggagg tggtgtgcta cgagagcccg aacccgacga cggggatcca caggatggtg 360
ctggtgctgt tccggcagct ggggagggag acggtgtacg cgccggcggt gcgccacaac 420
ttcaccaccc gcgccttcgc ccgccgctac aacctcggcg cgcccgtcgc cgccgtctac 480
ttcaactgcc agcgccaggc cggctccggc ggccggaggt tcaccggacc ttacacctcc 540
cgccgccgcc aagcctaa                                                558

<210> 82
<211> 185
<212> PRT
<213> Oryza sativa

<400> 82
Met Ala Asn Asp Ser Leu Ala Thr Gly Arg Val Ile Gly Asp Val Leu
1               5                   10                  15
Asp Pro Phe Ile Ser Thr Val Asp Leu Thr Val Met Tyr Gly Asp Asp
            20                  25                  30
Gly Met Pro Val Ile Ser Gly Val Glu Leu Arg Ala Pro Ala Val Ala
        35                  40                  45
Glu Lys Pro Val Val Glu Val Gly Gly Asp Asp Leu Arg Val Ala Tyr
    50                  55                  60
Thr Leu Val Met Val Asp Pro Asp Ala Pro Asn Pro Ser Asn Pro Thr
65                  70                  75                  80
Leu Arg Glu Tyr Leu His Trp Met Val Thr Asp Ile Pro Ala Ser Thr
                85                  90                  95
Asp Ala Thr Tyr Gly Arg Glu Val Val Cys Tyr Glu Ser Pro Asn Pro
            100                 105                 110
Thr Thr Gly Ile His Arg Met Val Leu Val Leu Phe Arg Gln Leu Gly
        115                 120                 125
Arg Glu Thr Val Tyr Ala Pro Ala Val Arg His Asn Phe Thr Thr Arg
    130                 135                 140
Ala Phe Ala Arg Arg Tyr Asn Leu Gly Ala Pro Val Ala Ala Val Tyr
145                 150                 155                 160
Phe Asn Cys Gln Arg Gln Ala Gly Ser Gly Gly Arg Arg Phe Thr Gly
                165                 170                 175
Pro Tyr Thr Ser Arg Arg Arg Gln Ala
            180                 185

<210> 83
<211> 522
<212> DNA
<213> Oryza sativa

<400> 83

atgtctaggt ctgtggagcc tcttgttgtt gggcgggtga tcggagaagt tattgattca 60
ttcaacccat gtacgaagat gatagtaacc tacaattcaa acaagcttgt ctttaatggc 120
catgagttct acccatcagc agttgtatct aaaccaagag tcgaggtcca agggggtgat 180
atgcgttctt tcttcacatt ggttatgaca gacccagatg tgccaggacc aagtgatcca 240
tatctaaggg aacacctaca ttggattgta actgatatac ctggaacaac ggatgcctct 300
tttggacggg aaatcataag ctatgagagc ccaaagccca gcattggtat ccacaggttc 360
gtttttgtgc tcttcaagca gaagcgtagg caggctgtag ttgtgccatc ctctagggat 420
catttcaata cacgccagtt tgctgaggag aacgaacttg gccttcctgt cgctgctgtc 480
tacttcaatg ctcagagaga gactgctgcc aggagacgct aa 522

<210> 84
<211> 173
<212> PRT
<213> Oryza sativa

<400> 84
Met Ser Arg Ser Val Glu Pro Leu Val Val Gly Arg Val Ile Gly Glu
1               5                   10                  15
Val Ile Asp Ser Phe Asn Pro Cys Thr Lys Met Ile Val Thr Tyr Asn
            20                  25                  30
Ser Asn Lys Leu Val Phe Asn Gly His Glu Phe Tyr Pro Ser Ala Val
        35                  40                  45
Val Ser Lys Pro Arg Val Glu Val Gln Gly Gly Asp Met Arg Ser Phe
    50                  55                  60
Phe Thr Leu Val Met Thr Asp Pro Asp Val Pro Gly Pro Ser Asp Pro
65                  70                  75                  80
Tyr Leu Arg Glu His Leu His Trp Ile Val Thr Asp Ile Pro Gly Thr
                85                  90                  95
Thr Asp Ala Ser Phe Gly Arg Glu Ile Ile Ser Tyr Glu Ser Pro Lys
            100                 105                 110
Pro Ser Ile Gly Ile His Arg Phe Val Phe Val Leu Phe Lys Gln Lys
        115                 120                 125
Arg Arg Gln Ala Val Val Val Pro Ser Ser Arg Asp His Phe Asn Thr
    130                 135                 140
Arg Gln Phe Ala Glu Glu Asn Glu Leu Gly Leu Pro Val Ala Ala Val
145                 150                 155                 160
Tyr Phe Asn Ala Gln Arg Glu Thr Ala Ala Arg Arg Arg
                165                 170

<210> 85
<211> 522
<212> DNA
<213> Oryza sativa

<400> 85
atgtctaggt ctgtggagcc tcttgttgta gggcgcgtga ttggggaagt tcttgatacc 60
tttaacccat gcatgaagat gatagtgacc tataactcca acaagcttgt atttaatggt 120
catgagctct acccatcagc agttgtgtct aaaccaagag ttgaggtcca agggggtgac 180
ctgcgatctt tcttcacatt ggttatgaca gacccagatg tgccaggacc aagtgatcct 240
tatctaaggg agcaccttca ttggattgtt actgatatac ctgggacaac ggatgcttct 300
tttgggcgcg aggtcataag ctatgagagt ccaaagccga acattggcat ccataggttc 360
atttttgtgc tcttcaagca gaagcgcagg caaactgtaa ttgtgccatc cttcagggac 420
catttcaaca cccgccggtt cgccgaggag aatgatcttg gccttcctgt ggctgctgtc 480
tacttcaatg cccagagaga gactgcagcc aggaggcgct ga 522

<210> 86
<211> 173
<212> PRT
<213> Oryza sativa

<400> 86
Met Ser Arg Ser Val Glu Pro Leu Val Val Gly Arg Val Ile Gly Glu
1               5                   10                  15
Val Leu Asp Thr Phe Asn Pro Cys Met Lys Met Ile Val Thr Tyr Asn
            20                  25                  30
Ser Asn Lys Leu Val Phe Asn Gly His Glu Leu Tyr Pro Ser Ala Val
        35                  40                  45
Val Ser Lys Pro Arg Val Glu Val Gln Gly Gly Asp Leu Arg Ser Phe
    50                  55                  60
Phe Thr Leu Val Met Thr Asp Pro Asp Val Pro Gly Pro Ser Asp Pro
65                  70                  75                  80
Tyr Leu Arg Glu His Leu His Trp Ile Val Thr Asp Ile Pro Gly Thr
                85                  90                  95
Thr Asp Ala Ser Phe Gly Arg Glu Val Ile Ser Tyr Glu Ser Pro Lys
            100                 105                 110
Pro Asn Ile Gly Ile His Arg Phe Ile Phe Val Leu Phe Lys Gln Lys
        115                 120                 125
Arg Arg Gln Thr Val Ile Val Pro Ser Phe Arg Asp His Phe Asn Thr
    130                 135                 140
Arg Arg Phe Ala Glu Glu Asn Asp Leu Gly Leu Pro Val Ala Ala Val
145                 150                 155                 160
Tyr Phe Asn Ala Gln Arg Glu Thr Ala Ala Arg Arg Arg
                165                 170

<210> 87
<211> 525
<212> DNA
<213> Oryza sativa

<400> 87
atgtctaggg acccattggt tgtcggtcat gtcgtcggcg atatcgtgga cccgttcgtc 60
accaccgctt cgcttagggt cttctacaac agcaaggaga tgacaaatgg gtctgagctc 120
aagccatctc aggtgttgaa ccaaccaagg atttatatcg aaggtcgcga catgaggacg 180
ctctacacgc ttgtaatggt ggaccctgat gcaccaagcc ccagcaaccc tactaaaaga 240
gagtaccttc attggatggt gacagacatt ccagagacca ctgatgccag atttggtaat 300
gagattgtcc cctatgagag cccacgccca actgcaggca tccatcgctt cgtgttcatc 360
ctattcaggc agtcagtcag gcagaccacc tatgcaccag ggtggcgcca aaacttcaat 420
acaagggact ttgctgagct ctacaacctc ggttcgccgg tcgccgcgct cttcttcaac 480
tgccagaggg agaacggctg tggaggaaga aggtgtgtta gatga                 525

<210> 88
<211> 174
<212> PRT
<213> Oryza sativa

<400> 88
Met Ser Arg Asp Pro Leu Val Val Gly His Val Val Gly Asp Ile Val
1               5                   10                  15
Asp Pro Phe Val Thr Thr Ala Ser Leu Arg Val Phe Tyr Asn Ser Lys
            20                  25                  30
Glu Met Thr Asn Gly Ser Glu Leu Lys Pro Ser Gln Val Leu Asn Gln
        35                  40                  45
Pro Arg Ile Tyr Ile Glu Gly Arg Asp Met Arg Thr Leu Tyr Thr Leu
    50                  55                  60
Val Met Val Asp Pro Asp Ala Pro Ser Pro Ser Asn Pro Thr Lys Arg
65                  70                  75                  80
Glu Tyr Leu His Trp Met Val Thr Asp Ile Pro Glu Thr Thr Asp Ala
                85                  90                  95
Arg Phe Gly Asn Glu Ile Val Pro Tyr Glu Ser Pro Arg Pro Thr Ala
            100                 105                 110

Gly Ile His Arg Phe Val Phe Ile Leu Phe Arg Gln Ser Val Arg Gln
        115                 120                 125
Thr Thr Tyr Ala Pro Gly Trp Arg Gln Asn Phe Asn Thr Arg Asp Phe
    130                 135                 140
Ala Glu Leu Tyr Asn Leu Gly Ser Pro Val Ala Ala Leu Phe Phe Asn
145                 150                 155                 160
Cys Gln Arg Glu Asn Gly Cys Gly Gly Arg Arg Cys Val Arg
                165                 170

<210> 89
<211> 525
<212> DNA
<213> Oryza sativa

<400> 89
atggatcctt tgtacctatc tcagatcata ccggatgtgt tggatccatt tatttcaacc 60
atttcactca gagtaaccta caacagcagg ctacttctgg caggagcagc gcttaaacca 120
tctgcagttg taagcaagcc acaggttgat gttggtggca atgacatgag ggtttcctac 180
acactggtat tggtggatcc agatgcccca agcccaagtg acccatcgct gagggagtac 240
ttgcactgga tggtaacaga tatccctgaa acaacttcca tcagctttgg cgaagagtta 300
atattatatg agaagccaga gccaagatca ggcatccatc ggatggtatt tgtgctgttc 360
cgccaacttg gcaggcggac agtctttgca ccggaaaaac gacataactt caactgcaga 420
atttttgcac gccaacacca cctcaacatc gtggctgcca catacttcaa ctgtcaaagg 480
gaggcaggat ggggtggaag aaagtttgcg cctgaaggcc cttaa 525

<210> 90
<211> 174
<212> PRT
<213> Oryza sativa

<400> 90
Met Asp Pro Leu Tyr Leu Ser Gln Ile Ile Pro Asp Val Leu Asp Pro
1               5                   10                  15
Phe Ile Ser Thr Ile Ser Leu Arg Val Thr Tyr Asn Ser Arg Leu Leu
            20                  25                  30
Leu Ala Gly Ala Ala Leu Lys Pro Ser Ala Val Val Ser Lys Pro Gln
        35                  40                  45
Val Asp Val Gly Gly Asn Asp Met Arg Val Ser Tyr Thr Leu Val Leu
    50                  55                  60
Val Asp Pro Asp Ala Pro Ser Pro Ser Asp Pro Ser Leu Arg Glu Tyr
65                  70                  75                  80
Leu His Trp Met Val Thr Asp Ile Pro Glu Thr Thr Ser Ile Ser Phe
                85                  90                  95
Gly Glu Glu Leu Ile Leu Tyr Glu Lys Pro Glu Pro Arg Ser Gly Ile
            100                 105                 110
His Arg Met Val Phe Val Leu Phe Arg Gln Leu Gly Arg Arg Thr Val
        115                 120                 125
Phe Ala Pro Glu Lys Arg His Asn Phe Asn Cys Arg Ile Phe Ala Arg
    130                 135                 140
Gln His His Leu Asn Ile Val Ala Ala Thr Tyr Phe Asn Cys Gln Arg
145                 150                 155                 160
Glu Ala Gly Trp Gly Gly Arg Lys Phe Ala Pro Glu Gly Pro
                165                 170

<210> 91
<211> 522
<212> DNA
<213> Oryza sativa

<400> 91
atggcaaatg actcattgac aaggagccat atagttggag atgtgttaga ccaattttca 60
aactcagtgc ctctaactgt gatgtatgat gggaggcctg tgtttaatgg caaggagttc 120
cgttcctcgg cagtctcgat gaaacctaga gttgagattg gtggcgatga ttttcgattt 180
gcctataccc tagttatggt ggatcctgat gctcctaatc ccagcaaccc aaccttgagg 240
gaatacctgc actggatggt gactgatatc ccatcatcga cggacgatag ctttgggcgg 300
gagatcgtaa catacgaaag cccaagcccc accatgggca tccaccgcat cgtgatggtg 360
ttgtatcagc agcttgggcg cggcacggtg ttcgcgccgc aggtgcgtca gaacttcaac 420
ctgcgcagct tcgcgcgccg tttcaacctc ggcaagccgg tggccgccat gtacttcaac 480
tgccagcgcc cgacaggcac aggtgggagg aggccaacct ga 522

<210> 92
<211> 173
<212> PRT
<213> Oryza sativa

<400> 92
Met Ala Asn Asp Ser Leu Thr Arg Ser His Ile Val Gly Asp Val Leu
1               5                   10                  15
Asp Gln Phe Ser Asn Ser Val Pro Leu Thr Val Met Tyr Asp Gly Arg
            20                  25                  30
Pro Val Phe Asn Gly Lys Glu Phe Arg Ser Ser Ala Val Ser Met Lys
        35                  40                  45
Pro Arg Val Glu Ile Gly Gly Asp Asp Phe Arg Phe Ala Tyr Thr Leu
    50                  55                  60
Val Met Val Asp Pro Asp Ala Pro Asn Pro Ser Asn Pro Thr Leu Arg
65                  70                  75                  80
Glu Tyr Leu His Trp Met Val Thr Asp Ile Pro Ser Ser Thr Asp Asp
                85                  90                  95
Ser Phe Gly Arg Glu Ile Val Thr Tyr Glu Ser Pro Ser Pro Thr Met
            100                 105                 110
Gly Ile His Arg Ile Val Met Val Leu Tyr Gln Gln Leu Gly Arg Gly
        115                 120                 125
Thr Val Phe Ala Pro Gln Val Arg Gln Asn Phe Asn Leu Arg Ser Phe
    130                 135                 140
Ala Arg Arg Phe Asn Leu Gly Lys Pro Val Ala Ala Met Tyr Phe Asn
145                 150                 155                 160
Cys Gln Arg Pro Thr Gly Thr Gly Gly Arg Arg Pro Thr
                165                 170

<210> 93
<211> 531
<212> DNA
<213> Oryza sativa

<400> 93
atggcatcgc atgtggaccc gctggtggtg gggagggtga tcggcgacgt ggtggacctg 60
ttcgtgccga cgacggccat gtcggtgcgg ttcgggacca aggacctcac caacggctgc 120
gagatcaagc cgtccgtcgc cgccgcgccg cccgccgtgc agatcgccgg cagggtcaac 180
gagctcttcg ctctggtcat gactgatcca gatgctccta gccccagcga gccgactatg 240
agagagtggc ttcactggct ggtggttaac ataccaggtg gaacagatcc ttctcaaggg 300
gatgtggtgg tgccgtacat ggggccacgg ccgccggtgg ggatccaccg ctacgtgatg 360
gtgctgttcc agcagaaggc gcgcgtggcg gcgccgccgc ccgacgagga cgccgcgcgc 420
gccaggttca gcacgcgcgc cttcgccgac cgccacgacc tcggcctccc cgtcgccgcc 480
ctctacttca acgcccagaa ggagcccgcc aaccgccgcc gccgctacta g 531

<210> 94
<211> 176
<212> PRT
<213> Oryza sativa

<400> 94
Met Ala Ser His Val Asp Pro Leu Val Val Gly Arg Val Ile Gly Asp
1               5                   10                  15
Val Val Asp Leu Phe Val Pro Thr Thr Ala Met Ser Val Arg Phe Gly
            20                  25                  30
Thr Lys Asp Leu Thr Asn Gly Cys Glu Ile Lys Pro Ser Val Ala Ala
        35                  40                  45
Ala Pro Pro Ala Val Gln Ile Ala Gly Arg Val Asn Glu Leu Phe Ala
    50                  55                  60
Leu Val Met Thr Asp Pro Asp Ala Pro Ser Pro Ser Glu Pro Thr Met
65                  70                  75                  80
Arg Glu Trp Leu His Trp Leu Val Val Asn Ile Pro Gly Gly Thr Asp
                85                  90                  95
Pro Ser Gln Gly Asp Val Val Val Pro Tyr Met Gly Pro Arg Pro Pro
            100                 105                 110
Val Gly Ile His Arg Tyr Val Met Val Leu Phe Gln Gln Lys Ala Arg
        115                 120                 125
Val Ala Ala Pro Pro Pro Asp Glu Asp Ala Ala Arg Ala Arg Phe Ser
    130                 135                 140
Thr Arg Ala Phe Ala Asp Arg His Asp Leu Gly Leu Pro Val Ala Ala
145                 150                 155                 160
Leu Tyr Phe Asn Ala Gln Lys Glu Pro Ala Asn Arg Arg Arg Arg Tyr
                165                 170                 175

<210> 95
<211> 525
<212> DNA
<213> Oryza sativa

<400> 95
atgtcaaggg atccacttgt tgtaggcaat gtggttgggg atatcttgga cccatttatc 60
aaatcagcat cactcagagt gctttacagc aatagggaac tgactaatgg atctgagctc 120
aagccttcac aagtagcgaa cgagccaagg attgagattg ctggtcgtga catgaggaca 180
ctttacactt tggtgatggt ggatcctgac tcaccaagtc caagcaatcc aaccaaaaga 240
gaataccttc attggttggt gacggacatt ccagaaacaa caaatgcgag ctttggaaat 300
gagatagtca gctatgaaag tccaaagcca acagcgggaa tacatcgctt tgtctttgtg 360
cttttccgtc aatctgtcca acagaccatt tatgcacctg gatggcgaca aaattttaac 420
acaagggatt tctcggcact ttacaaccta ggaccaccgg tggctgccgt gttcttcaac 480
tgccaaagag agaatggttg tggtggcaga cgatacatta gatga                 525

<210> 96
<211> 174
<212> PRT
<213> Oryza sativa

<400> 96
Met Ser Arg Asp Pro Leu Val Val Gly Asn Val Val Gly Asp Ile Leu
1               5                   10                  15
Asp Pro Phe Ile Lys Ser Ala Ser Leu Arg Val Leu Tyr Ser Asn Arg
            20                  25                  30
Glu Leu Thr Asn Gly Ser Glu Leu Lys Pro Ser Gln Val Ala Asn Glu
        35                  40                  45
Pro Arg Ile Glu Ile Ala Gly Arg Asp Met Arg Thr Leu Tyr Thr Leu
    50                  55                  60
Val Met Val Asp Pro Asp Ser Pro Ser Pro Ser Asn Pro Thr Lys Arg
65                  70                  75                  80
Glu Tyr Leu His Trp Leu Val Thr Asp Ile Pro Glu Thr Thr Asn Ala
                85                  90                  95
Ser Phe Gly Asn Glu Ile Val Ser Tyr Glu Ser Pro Lys Pro Thr Ala

                100                 105                 110
Gly Ile His Arg Phe Val Phe Val Leu Phe Arg Gln Ser Val Gln Gln
        115                 120                 125
Thr Ile Tyr Ala Pro Gly Trp Arg Gln Asn Phe Asn Thr Arg Asp Phe
    130                 135                 140
Ser Ala Leu Tyr Asn Leu Gly Pro Pro Val Ala Ala Val Phe Phe Asn
145                 150                 155                 160
Cys Gln Arg Glu Asn Gly Cys Gly Gly Arg Arg Tyr Ile Arg
                165                 170

<210> 97
<211> 231
<212> DNA
<213> Oryza sativa

<400> 97
gatgtggtgg tgccgtacat ggggccacgg ccgccggtgg ggatccaccg ctacgtgatg 60
gtgctgttcc agcagaaggc gcgcgtggcg gcgccgccgc ccgacgagga cgccgcgcgc 120
gccaggttca gcacgcgcgc cttcgccgac cgccacgacc tcggcctccc cgtcgccgcc 180
ctctacttca acgcccagaa ggagcccgcc aaccgccgcc gccgctacta g          231

<210> 98
<211> 76
<212> PRT
<213> Oryza sativa

<400> 98
Asp Val Val Val Pro Tyr Met Gly Pro Arg Pro Pro Val Gly Ile His
 1               5                  10                  15
Arg Tyr Val Met Val Leu Phe Gln Gln Lys Ala Arg Val Ala Ala Pro
            20                  25                  30
Pro Pro Asp Glu Asp Ala Ala Arg Ala Arg Phe Ser Thr Arg Ala Phe
        35                  40                  45
Ala Asp Arg His Asp Leu Gly Leu Pro Val Ala Ala Leu Tyr Phe Asn
    50                  55                  60
Ala Gln Lys Glu Pro Ala Asn Arg Arg Arg Arg Tyr
65                  70                  75

<210> 99
<211> 516
<212> DNA
<213> Sorghum bicolor

<400> 99
atggcgcggt tcgtggatcc gctggtggtg gggcgggtga tcggcgaggt ggtggacctg 60
ttcgtgccct ccatctccat gaccgtcgcc tatggcccca aggacatcag caacggctgc 120
ctcctcaagc cgtccgccac cgccgcgccg ccgctcgtcc gcatctccgg ccgccgcaac 180
gacctctaca cgctgatcat gacggaccct gatgcgccta gccccagcga cccgaccatg 240
agggagtacc tccactggat agtgaccaac ataccaggag gaacggatgc aagcaaaggt 300
gaggaggtgg tggagtacat gggcccgcgg ccgccggtgg gcatccaccg ctacgtgctg 360
gtgctgttcg agcagaagac gcgcgtgcac gcggaggcgc cccgcgagcg cgccaacttc 420
aacacgcgcg cgttcgcggc ggcgcacgag ctcggcctcc ccaccgccgt cgtctacttc 480
aacgcgcaga aggagcccgc caaccgccgc cgctag                           516

<210> 100
<211> 171
<212> PRT
<213> Sorghum bicolor

<400> 100
Met Ala Arg Phe Val Asp Pro Leu Val Val Gly Arg Val Ile Gly Glu
1               5                   10                  15
Val Val Asp Leu Phe Val Pro Ser Ile Ser Met Thr Val Ala Tyr Gly
            20                  25                  30
Pro Lys Asp Ile Ser Asn Gly Cys Leu Leu Lys Pro Ser Ala Thr Ala
        35                  40                  45
Ala Pro Pro Leu Val Arg Ile Ser Gly Arg Arg Asn Asp Leu Tyr Thr
    50                  55                  60
Leu Ile Met Thr Asp Pro Asp Ala Pro Ser Pro Ser Asp Pro Thr Met
65                  70                  75                  80
Arg Glu Tyr Leu His Trp Ile Val Thr Asn Ile Pro Gly Gly Thr Asp
                85                  90                  95
Ala Ser Lys Gly Glu Glu Val Val Glu Tyr Met Gly Pro Arg Pro Pro
            100                 105                 110
Val Gly Ile His Arg Tyr Val Leu Val Leu Phe Glu Gln Lys Thr Arg
        115                 120                 125
Val His Ala Glu Ala Pro Arg Glu Arg Ala Asn Phe Asn Thr Arg Ala
    130                 135                 140
Phe Ala Ala Ala His Glu Leu Gly Leu Pro Thr Ala Val Val Tyr Phe
145                 150                 155                 160
Asn Ala Gln Lys Glu Pro Ala Asn Arg Arg Arg
                165                 170

<210> 101
<211> 522
<212> DNA
<213> Sorghum bicolor

<400> 101
atgtctagat ctgtggagtc tctcatagtt ggtcgggtga ttggagaagt tctcgactcc 60
tttagcccat gtgtgaagat ggtagtgacc tacaactcaa acaagcttgt cttcaatggc 120
catgagatct acccatcagc agttgtatcc aaaccaagag tagaggttca agggggtgac 180
ttgcggtctt tcttcacatt ggttatgaca gacccagatg ttccagggcc aagtgatcca 240
tatctaaggg agcaccttca ctggatcgtg actgatatac ctgggacaac agatgcctca 300
ttcgggcgag aagttataag ctatgagagc ccaagaccta gcattggtat ccacaggttc 360
atttttgttc tcttcaagca gaagcgcagg caaactgtag ctatgccatc ctccagggac 420
catttcatca cacgacagtt tgctgaggaa aatgatcttg gactccctgt agctgctgtc 480
tacttcaacg ctcagagaga aactgctgct aggaggcgct ga                    522

<210> 102
<211> 173
<212> PRT
<213> Sorghum bicolor

<400> 102
Met Ser Arg Ser Val Glu Ser Leu Ile Val Gly Arg Val Ile Gly Glu
1               5                   10                  15
Val Leu Asp Ser Phe Ser Pro Cys Val Lys Met Val Val Thr Tyr Asn
            20                  25                  30
Ser Asn Lys Leu Val Phe Asn Gly His Glu Ile Tyr Pro Ser Ala Val
        35                  40                  45
Val Ser Lys Pro Arg Val Glu Val Gln Gly Gly Asp Leu Arg Ser Phe
    50                  55                  60
Phe Thr Leu Val Met Thr Asp Pro Asp Val Pro Gly Pro Ser Asp Pro
65                  70                  75                  80
Tyr Leu Arg Glu His Leu His Trp Ile Val Thr Asp Ile Pro Gly Thr
                85                  90                  95
Thr Asp Ala Ser Phe Gly Arg Glu Val Ile Ser Tyr Glu Ser Pro Arg
            100                 105                 110

Pro Ser Ile Gly Ile His Arg Phe Ile Phe Val Leu Phe Lys Gln Lys
        115                 120                 125
Arg Arg Gln Thr Val Ala Met Pro Ser Ser Arg Asp His Phe Ile Thr
    130                 135                 140
Arg Gln Phe Ala Glu Glu Asn Asp Leu Gly Leu Pro Val Ala Ala Val
145                 150                 155                 160
Tyr Phe Asn Ala Gln Arg Glu Thr Ala Ala Arg Arg Arg
                165                 170

<210> 103
<211> 522
<212> DNA
<213> Sorghum bicolor

<400> 103
atgtctaggg tgttggaacc tctagtcgtc ggcaaggtga ttggggaagt catcgacaac 60
ttcaacccca cggtgaagat gacggttacc tacggctcca acaaccaggt gttcaacggc 120
catgagttct tcccgtctgc ggttctgtcc aagccgcgcg tggaggttca gggcgacgac 180
atgaggtcct tcttcacgct ggtcatgact gacccagatg tgccagggcc tagtgatcca 240
tacctgagag agcatctcca ttggatcgtc actgacattc ctggaacaac tgatgcttct 300
tttggaacgg agttggcgat gtacgagagc cccaaaccct acatcggcat ccacaggttc 360
gtcttcgtgc tgttcaagca gaagagccgc cagtcggtgc gcccgccctc gtccagggac 420
tacttcagca cccgccgctt tgccgccgac aacgatctcg gcctcccagt cgctgccgtc 480
tacttcaacg cgcagcggga gaccgccgcg cgccgccgct ga                    522

<210> 104
<211> 173
<212> PRT
<213> Sorghum bicolor

<400> 104
Met Ser Arg Val Leu Glu Pro Leu Val Val Gly Lys Val Ile Gly Glu
1               5                   10                  15
Val Ile Asp Asn Phe Asn Pro Thr Val Lys Met Thr Val Thr Tyr Gly
            20                  25                  30
Ser Asn Asn Gln Val Phe Asn Gly His Glu Phe Phe Pro Ser Ala Val
        35                  40                  45
Leu Ser Lys Pro Arg Val Glu Val Gln Gly Asp Asp Met Arg Ser Phe
    50                  55                  60
Phe Thr Leu Val Met Thr Asp Pro Asp Val Pro Gly Pro Ser Asp Pro
65                  70                  75                  80
Tyr Leu Arg Glu His Leu His Trp Ile Val Thr Asp Ile Pro Gly Thr
                85                  90                  95
Thr Asp Ala Ser Phe Gly Thr Glu Leu Ala Met Tyr Glu Ser Pro Lys
            100                 105                 110
Pro Tyr Ile Gly Ile His Arg Phe Val Phe Val Leu Phe Lys Gln Lys
        115                 120                 125
Ser Arg Gln Ser Val Arg Pro Pro Ser Ser Arg Asp Tyr Phe Ser Thr
    130                 135                 140
Arg Arg Phe Ala Ala Asp Asn Asp Leu Gly Leu Pro Val Ala Ala Val
145                 150                 155                 160
Tyr Phe Asn Ala Gln Arg Glu Thr Ala Ala Arg Arg Arg
                165                 170

<210> 105
<211> 534
<212> DNA
<213> Sorghum bicolor

<400> 105
atgtcgtcga gggatccgct agtggttgga agcatcgtgg gcgacatcgt ggactacttc 60
tcagcgtcgg cgctgctccg agttatgtac ggcgggcgcg agatcacctg cgggtcggag 120
ctcaggccgt cccaggtcgc cggcgagccg acggtgcaca tcaccggagg ccgccgcgac 180
gggacgccgg cgttctacac actgctgatg ctggaccctg atgcgcccag cccaagcaac 240
ccgaccaaac gggagtatct ccattggttg gtgactgata taccagaagg agctggtgcc 300
aatcatggga acgaggtggt ggcgtacgag agcccccggc catcggcggg gatccaccgg 360
ttcgtgttca tcgtgttccg gcaggagatc cggcagttga tatacacgcc ggggtggcgc 420
gccaacttca catccaggga cttcgccgcc agctacagcc tcggaccgcc tgtcgccgcc 480
acttacttca acttccagag ggaggtaggc tgcggtggct ggaggtacag gtga 534

<210> 106
<211> 177
<212> PRT
<213> Sorghum bicolor

<400> 106
Met Ser Ser Arg Asp Pro Leu Val Val Gly Ser Ile Val Gly Asp Ile
1               5                   10                  15
Val Asp Tyr Phe Ser Ala Ser Ala Leu Leu Arg Val Met Tyr Gly Gly
            20                  25                  30
Arg Glu Ile Thr Cys Gly Ser Glu Leu Arg Pro Ser Gln Val Ala Gly
        35                  40                  45
Glu Pro Thr Val His Ile Thr Gly Gly Arg Arg Asp Gly Thr Pro Ala
    50                  55                  60
Phe Tyr Thr Leu Leu Met Leu Asp Pro Asp Ala Pro Ser Pro Ser Asn
65                  70                  75                  80
Pro Thr Lys Arg Glu Tyr Leu His Trp Leu Val Thr Asp Ile Pro Glu
                85                  90                  95
Gly Ala Gly Ala Asn His Gly Asn Glu Val Val Ala Tyr Glu Ser Pro
            100                 105                 110
Arg Pro Ser Ala Gly Ile His Arg Phe Val Phe Ile Val Phe Arg Gln
        115                 120                 125
Glu Ile Arg Gln Leu Ile Tyr Thr Pro Gly Trp Arg Ala Asn Phe Thr
    130                 135                 140
Ser Arg Asp Phe Ala Ala Ser Tyr Ser Leu Gly Pro Pro Val Ala Ala
145                 150                 155                 160
Thr Tyr Phe Asn Phe Gln Arg Glu Val Gly Cys Gly Gly Trp Arg Tyr
                165                 170                 175
Arg

<210> 107
<211> 549
<212> DNA
<213> Sorghum bicolor

<400> 107
atggccaacg attccttggt tacagctcgt gtcataggag atgtcctgga ccccttctac 60
agctccattg atctgatggt gctcttcaat ggtatgccca ttgtcagcgg catggagttg 120
cgtgctccga cggtctctga gaggccaagg gttgagatcg gaggagatga ctatcgtgtt 180
gcttataccc tggtgatggt tgatcctgat gctccaaacc caagcaaccc aaccctaagg 240
gagtacctgc actggatggt cactgacatt ccagcgtcaa ctgatgacac ctacggcgcg 300
gaggtgatgt gctacgaggc cccaaacccg acgacgggga tccaccgcat ggtgctggtg 360
ctgttccggc agctggggcg ggagacggtg tacgcgccgt cctggcgcca caacttcagc 420
acgcgcggct tcgcccgccg ctacaacctc ggcgcgcccg tcgccgccat gtacttcaac 480
tgccagcgcc agaacggctc cggcggacgg aggttcaccg gggcctacac cggcggcaga 540
catggttag 549

<210> 108

<211> 182
<212> PRT
<213> Sorghum bicolor

<400> 108
Met Ala Asn Asp Ser Leu Val Thr Ala Arg Val Ile Gly Asp Val Leu
1               5                   10                  15
Asp Pro Phe Tyr Ser Ser Ile Asp Leu Met Val Leu Phe Asn Gly Met
            20                  25                  30
Pro Ile Val Ser Gly Met Glu Leu Arg Ala Pro Thr Val Ser Glu Arg
        35                  40                  45
Pro Arg Val Glu Ile Gly Gly Asp Asp Tyr Arg Val Ala Tyr Thr Leu
    50                  55                  60
Val Met Val Asp Pro Asp Ala Pro Asn Pro Ser Asn Pro Thr Leu Arg
65                  70                  75                  80
Glu Tyr Leu His Trp Met Val Thr Asp Ile Pro Ala Ser Thr Asp Asp
                85                  90                  95
Thr Tyr Gly Arg Glu Val Met Cys Tyr Glu Ala Pro Asn Pro Thr Thr
            100                 105                 110
Gly Ile His Arg Met Val Leu Val Leu Phe Arg Gln Leu Gly Arg Glu
        115                 120                 125
Thr Val Tyr Ala Pro Ser Trp Arg His Asn Phe Ser Thr Arg Gly Phe
    130                 135                 140
Ala Arg Arg Tyr Asn Leu Gly Ala Pro Val Ala Ala Met Tyr Phe Asn
145                 150                 155                 160
Cys Gln Arg Gln Asn Gly Ser Gly Gly Arg Arg Phe Thr Gly Ala Tyr
                165                 170                 175
Thr Gly Gly Arg His Gly
            180


<210> 109
<211> 522
<212> DNA
<213> Sorghum bicolor

<400> 109
atgtcaaggg tgttggagcc tctcattgtg gggaaagtga ttggtgaggt gctggaccat 60
ttcaacccca cggtgaagat ggtggtcacc tacaactcca acaagcaggt cttcaacgga 120
catgagttct tcccttccgc agtcaccgcc aagccgcgtg ttgaggtcca agggggtgac 180
ctcaggtcct tcttcacatt ggtgatgact gaccctgatg ttccaggacc tagtgatccc 240
tacctgaggg agcaccttca ctggattgtt actgatattc ctgggactac tgatgcttct 300
tttgggagag aggtggtgag ctacgagacc ccaaagccaa acattggcat ccacaggttc 360
atctttgtgc tgttccggca gaagcgccgg caggcggtga acccgccgtc gtccaaggac 420
cgcttcagca cccgccagtt cgctgaggac aacgacctcg gcctccccgt cgccgccgtc 480
tacttcaacg cacagcgcga gaccgccgcc cgccggcgct aa                    522

<210> 110
<211> 173
<212> PRT
<213> Sorghum bicolor

<400> 110
Met Ser Arg Val Leu Glu Pro Leu Ile Val Gly Lys Val Ile Gly Glu
1               5                   10                  15
Val Leu Asp His Phe Asn Pro Thr Val Lys Met Val Val Thr Tyr Asn
            20                  25                  30
Ser Asn Lys Gln Val Phe Asn Gly His Glu Phe Phe Pro Ser Ala Val
        35                  40                  45
Thr Ala Lys Pro Arg Val Glu Val Gln Gly Gly Asp Leu Arg Ser Phe
    50                  55                  60

Phe Thr Leu Val Met Thr Asp Pro Asp Val Pro Gly Pro Ser Asp Pro
65                  70                  75                  80
Tyr Leu Arg Glu His Leu His Trp Ile Val Thr Asp Ile Pro Gly Thr
                85                  90                  95
Thr Asp Ala Ser Phe Gly Arg Glu Val Val Ser Tyr Glu Thr Pro Lys
            100                 105                 110
Pro Asn Ile Gly Ile His Arg Phe Ile Phe Val Leu Phe Arg Gln Lys
        115                 120                 125
Arg Arg Gln Ala Val Asn Pro Pro Ser Ser Lys Asp Arg Phe Ser Thr
    130                 135                 140
Arg Gln Phe Ala Glu Asp Asn Asp Leu Gly Leu Pro Val Ala Ala Val
145                 150                 155                 160
Tyr Phe Asn Ala Gln Arg Glu Thr Ala Ala Arg Arg Arg
                165                 170

<210> 111
<211> 531
<212> DNA
<213> Sorghum bicolor

<400> 111
atgtcacgag gaagggatcc tttggcattg agccaggtaa ttggtgatgt gttggatcct 60
ttcataaagt cagcaacaat gaggattaat tatggtgaca aggagatcac aaatggcact 120
ggactacgag catctgctgt gttcaatgca ccacatgttg agattgaagg ccacgaccaa 180
acaaagctct acacacttgt tatggtggat cctgatgcac caagtccgag caaaccagag 240
tacagggaat atctgcattg gttggtgaca gatacaccag aggcaagaga catacgtttt 300
ggcaatgaaa tagtccccta tgaaagccca agaccaccag ctggaattca tcgaattgtt 360
tttgtgctat tcaaacagca agcaagacaa acagtttatg caccaggatg gcggcaaaat 420
ttcaacatca gagacttctc agcaatttac aatcttggag caccagttgc tgcattatac 480
ttcaactgcc aaaaggaaag cggtgttggt ggcagaaggt tcctgggatc a          531

<210> 112
<211> 177
<212> PRT
<213> Sorghum bicolor

<400> 112
Met Ser Arg Gly Arg Asp Pro Leu Ala Leu Ser Gln Val Ile Gly Asp
 1               5                  10                  15
Val Leu Asp Pro Phe Ile Lys Ser Ala Thr Met Arg Ile Asn Tyr Gly
            20                  25                  30
Asp Lys Glu Ile Thr Asn Gly Thr Gly Leu Arg Ala Ser Ala Val Phe
        35                  40                  45
Asn Ala Pro His Val Glu Ile Glu Gly His Asp Gln Thr Lys Leu Tyr
    50                  55                  60
Thr Leu Val Met Val Asp Pro Asp Ala Pro Ser Pro Ser Lys Pro Glu
65                  70                  75                  80
Tyr Arg Glu Tyr Leu His Trp Leu Val Thr Asp Thr Pro Glu Ala Arg
                85                  90                  95
Asp Ile Arg Phe Gly Asn Glu Ile Val Pro Tyr Glu Ser Pro Arg Pro
            100                 105                 110
Pro Ala Gly Ile His Arg Ile Val Phe Val Leu Phe Lys Gln Gln Ala
        115                 120                 125
Arg Gln Thr Val Tyr Ala Pro Gly Trp Arg Gln Asn Phe Asn Ile Arg
    130                 135                 140
Asp Phe Ser Ala Ile Tyr Asn Leu Gly Ala Pro Val Ala Ala Leu Tyr
145                 150                 155                 160
Phe Asn Cys Gln Lys Glu Ser Gly Val Gly Gly Arg Arg Phe Leu Gly
                165                 170                 175
Ser

<210> 113
<211> 264
<212> DNA
<213> Sorghum bicolor

<400> 113
ttggtcactg atattccggc gacgactgga gtttcttttg ggactgaggt tgtgtgctac 60
gagagcccac ggccggtgct gggaatccac cggatggtgt ttctgctctt ccaacagctc 120
ggccggcaga cggtgtacgc cccagggtgg cggcagaact tcagcacccg cgacttcgcc 180
gagctctaca acctcggctt gccagtggcc gccgtttact tcaactgcca aagggagtcc 240
ggaactggtg ggagaagaat gtga 264

<210> 114
<211> 87
<212> PRT
<213> Sorghum bicolor

<400> 114
Leu Val Thr Asp Ile Pro Ala Thr Thr Gly Val Ser Phe Gly Thr Glu
1               5                   10                  15
Val Val Cys Tyr Glu Ser Pro Arg Pro Val Leu Gly Ile His Arg Met
            20                  25                  30
Val Phe Leu Leu Phe Gln Gln Leu Gly Arg Gln Thr Val Tyr Ala Pro
        35                  40                  45
Gly Trp Arg Gln Asn Phe Ser Thr Arg Asp Phe Ala Glu Leu Tyr Asn
    50                  55                  60
Leu Gly Leu Pro Val Ala Ala Val Tyr Phe Asn Cys Gln Arg Glu Ser
65                  70                  75                  80
Gly Thr Gly Gly Arg Arg Met
                85

<210> 115
<211> 258
<212> DNA
<213> Sorghum bicolor

<400> 115
cgcgaggtga tatgctacga gagccctcgg ccgccggcgg ggatccaccg cgtggtgttc 60
gtgctcttcc agcagatggc gcgtggctcc gtcgaccagc cgccggttct ccgccacaac 120
ttctgcaccc gcaacttcgc cgtcgaccac ggcctgggcg cccccgtcgc cgccgccttc 180
ttcacctgcc agcccgaggg tggcaccggc ggccgccgcc acgacctccg ccagccacgg 240
agaccgccgg cgtcctag 258

<210> 116
<211> 85
<212> PRT
<213> Sorghum bicolor

<400> 116
Arg Glu Val Ile Cys Tyr Glu Ser Pro Arg Pro Pro Ala Gly Ile His
1               5                   10                  15
Arg Val Val Phe Val Leu Phe Gln Gln Met Ala Arg Gly Ser Val Asp
            20                  25                  30
Gln Pro Pro Val Leu Arg His Asn Phe Cys Thr Arg Asn Phe Ala Val
        35                  40                  45
Asp His Gly Leu Gly Ala Pro Val Ala Ala Ala Phe Phe Thr Cys Gln
    50                  55                  60

Pro Glu Gly Gly Thr Gly Gly Arg Arg His Asp Leu Arg Gln Pro Arg
65                  70                  75                  80
Arg Pro Pro Ala Ser
                85

<210> 117
<211> 258
<212> DNA
<213> Sorghum bicolor

<400> 117
cgtgagatga tgtgctacga gccccctgcc ccgtccacgg gcatccaccg gatggtgctg 60
gtgctattcc agcagcttgg ccgtgacacg gtgttcgcgg cgccgtccag gcgccacaac 120
ttcaacaccc gtgccttcgc ccgccgctac aacctcggcg cgcccgtcgc cgccatgttc 180
ttcaactgcc agcgccagac cggctccggt ggccccaggt tcaccgggcc ctacaccagc 240
cgccgtcgtg cgggctga                                                258

<210> 118
<211> 85
<212> PRT
<213> Sorghum bicolor

<400> 118
Arg Glu Met Met Cys Tyr Glu Pro Pro Ala Pro Ser Thr Gly Ile His
 1               5                  10                  15
Arg Met Val Leu Val Leu Phe Gln Gln Leu Gly Arg Asp Thr Val Phe
            20                  25                  30
Ala Ala Pro Ser Arg Arg His Asn Phe Asn Thr Arg Ala Phe Ala Arg
        35                  40                  45
Arg Tyr Asn Leu Gly Ala Pro Val Ala Ala Met Phe Phe Asn Cys Gln
    50                  55                  60
Arg Gln Thr Gly Ser Gly Gly Pro Arg Phe Thr Gly Pro Tyr Thr Ser
65                  70                  75                  80
Arg Arg Arg Ala Gly
                85

<210> 119
<211> 246
<212> DNA
<213> Sorghum bicolor

<400> 119
gagacggtga tgccatacct gggcccttgc ccgccggtgg gcatccaccg ctacgttctg 60
gtggtgtacc agcagaaggc ccgcttcagg gctccgccgg tgctagcacc gggggcggag 120
gtggaggcgt cgcgcgcacg gttcaggaac cgcgccttcg ccgaccgcca tgacctaggc 180
ctcccagtcg ccgccatgta cttcaacgcg cagaaggagc cagcaaaccg ccaccgccac 240
tactga                                                             246

<210> 120
<211> 81
<212> PRT
<213> Sorghum bicolor

<400> 120
Glu Thr Val Met Pro Tyr Leu Gly Pro Cys Pro Pro Val Gly Ile His
 1               5                  10                  15
Arg Tyr Val Leu Val Val Tyr Gln Gln Lys Ala Arg Phe Arg Ala Pro
            20                  25                  30

Pro Val Leu Ala Pro Gly Ala Glu Val Glu Ala Ser Arg Ala Arg Phe
        35                  40                  45
Arg Asn Arg Ala Phe Ala Asp Arg His Asp Leu Gly Leu Pro Val Ala
    50                  55                  60
Ala Met Tyr Phe Asn Ala Gln Lys Glu Pro Ala Asn Arg His Arg His
65                  70                  75                  80
Tyr

<210> 121
<211> 228
<212> DNA
<213> Sorghum bicolor

<400> 121
caagaggtga tctgctacga gagccctcgg ccgaccatgg ggatccaccg cttcgtgctg 60
gtgctgttcc agcagctggg gcgtcagacg gtgtacgccc cggggtggcg ccagaacttc 120
aacaccaggg acttcgccga gctctacaac ctgggccctc ccgtcgccgc cgtctacttc 180
aactgccagc gtgaggccgg atctggggga aggaggatgt actcatga             228

<210> 122
<211> 75
<212> PRT
<213> Sorghum bicolor

<400> 122
Gln Glu Val Ile Cys Tyr Glu Ser Pro Arg Pro Thr Met Gly Ile His
 1               5                  10                  15
Arg Phe Val Leu Val Leu Phe Gln Gln Leu Gly Arg Gln Thr Val Tyr
            20                  25                  30
Ala Pro Gly Trp Arg Gln Asn Phe Asn Thr Arg Asp Phe Ala Glu Leu
        35                  40                  45
Tyr Asn Leu Gly Pro Pro Val Ala Ala Val Tyr Phe Asn Cys Gln Arg
    50                  55                  60
Glu Ala Gly Ser Gly Gly Arg Arg Met Tyr Ser
65                  70                  75

<210> 123
<211> 228
<212> DNA
<213> Sorghum bicolor

<400> 123
aatgaggtag taagctatga aagtccaaag ccaagtgctg gaatacatcg cttcgtcttt 60
gtgctcttcc gccaatctgt ccggcaaact atttatgcgc caggatggag gcaaaatttc 120
aacacaagag acttctcagc attctacaat ctaggaccac ctgtggcctc agtgttcttc 180
aactgccaaa gggagaatgg gtgtggtggc agacgatata ttagatga             228

<210> 124
<211> 75
<212> PRT
<213> Sorghum bicolor

<400> 124
Asn Glu Val Val Ser Tyr Glu Ser Pro Lys Pro Ser Ala Gly Ile His
 1               5                  10                  15
Arg Phe Val Phe Val Leu Phe Arg Gln Ser Val Arg Gln Thr Ile Tyr
            20                  25                  30

Ala Pro Gly Trp Arg Gln Asn Phe Asn Thr Arg Asp Phe Ser Ala Phe
        35                  40                  45
Tyr Asn Leu Gly Pro Pro Val Ala Ser Val Phe Phe Asn Cys Gln Arg
    50                  55                  60
Glu Asn Gly Cys Gly Gly Arg Arg Tyr Ile Arg
65                  70                  75

<210> 125
<211> 228
<212> DNA
<213> Sorghum bicolor

<400> 125
aatgaaatag ttccatatga gagcccacgt ccaactgccg gaatccatcg ctttgcattc 60
gtcttgttca ggcagtcagt caggcagacc acctatgcgc cggggtggag atcaaacttt 120
aacacaaggg acttcgcagc catctacaac cttggctccc ctgtcgctgc agtgtacttc 180
aactgccaga gagagaacgg ctgtggtgga agaaggtaca taaggtga             228

<210> 126
<211> 75
<212> PRT
<213> Sorghum bicolor

<400> 126
Asn Glu Ile Val Pro Tyr Glu Ser Pro Arg Pro Thr Ala Gly Ile His
 1               5                  10                  15
Arg Phe Ala Phe Val Leu Phe Arg Gln Ser Val Arg Gln Thr Thr Tyr
            20                  25                  30
Ala Pro Gly Trp Arg Ser Asn Phe Asn Thr Arg Asp Phe Ala Ala Ile
        35                  40                  45
Tyr Asn Leu Gly Ser Pro Val Ala Ala Val Tyr Phe Asn Cys Gln Arg
    50                  55                  60
Glu Asn Gly Cys Gly Gly Arg Arg Tyr Ile Arg
65                  70                  75

<210> 127
<211> 225
<212> DNA
<213> Sorghum bicolor

<400> 127
cgagagctca taccatatga gaacccaagc cccaccatgg gcatccaccg tattgtcttg 60
gtgctctacc agcaactggg gcggggcacg gtgtttgcac cgcaagtgcg tcaaaacttc 120
aacttgcgca attttgcacg ccgtttcaac ctcggcaagc ctgtggctgc gatgtacttc 180
aactgccagc ggcaaacagg cacaggtggg agaaggttca cttga                225

<210> 128
<211> 74
<212> PRT
<213> Sorghum bicolor

<400> 128
Arg Glu Leu Ile Pro Tyr Glu Asn Pro Ser Pro Thr Met Gly Ile His
 1               5                  10                  15
Arg Ile Val Leu Val Leu Tyr Gln Gln Leu Gly Arg Gly Thr Val Phe
            20                  25                  30
Ala Pro Gln Val Arg Gln Asn Phe Asn Leu Arg Asn Phe Ala Arg Arg
        35                  40                  45

Phe Asn Leu Gly Lys Pro Val Ala Ala Met Tyr Phe Asn Cys Gln Arg
    50                  55                  60
Gln Thr Gly Thr Gly Gly Arg Arg Phe Thr
65                  70

<210> 129
<211> 234
<212> DNA
<213> Sorghum bicolor

<400> 129
caggagctca tgttttacga aaggccagaa ccgagatctg gtatacaccg catggtattt 60
gtgctgttcc ggcaacttgg tagggggaca gtttttgcac cagacatgcg acataacttc 120
aactgcaaga actttgcacg tcaataccac ctagacattg tggctgccac atatttcaac 180
tgtcaaaggg aagcaggatc tggagggaga aggttcaggc ccgaaagttc gtaa       234

<210> 130
<211> 77
<212> PRT
<213> Sorghum bicolor

<400> 130
Gln Glu Leu Met Phe Tyr Glu Arg Pro Glu Pro Arg Ser Gly Ile His
 1               5                  10                  15
Arg Met Val Phe Val Leu Phe Arg Gln Leu Gly Arg Gly Thr Val Phe
            20                  25                  30
Ala Pro Asp Met Arg His Asn Phe Asn Cys Lys Asn Phe Ala Arg Gln
        35                  40                  45
Tyr His Leu Asp Ile Val Ala Ala Thr Tyr Phe Asn Cys Gln Arg Glu
    50                  55                  60
Ala Gly Ser Gly Gly Arg Arg Phe Arg Pro Glu Ser Ser
65                  70                  75

<210> 131
<211> 192
<212> DNA
<213> Sorghum bicolor

<400> 131
atgtcaaggg acccacttgt agtaggcaac gtagttggag atatcttgga tccatttatc 60
aaatcagcat cactcagagt cctatacaac aatagggaac tgactaatgg atctgagctc 120
aagccatcgc aagtagccaa tgaaccaagg attgagattg ctggacatga catgaggacc 180
ctttacactt tg                                                      192

<210> 132
<211> 64
<212> PRT
<213> Sorghum bicolor

<400> 132
Met Ser Arg Asp Pro Leu Val Val Gly Asn Val Val Gly Asp Ile Leu
 1               5                  10                  15
Asp Pro Phe Ile Lys Ser Ala Ser Leu Arg Val Leu Tyr Asn Asn Arg
            20                  25                  30
Glu Leu Thr Asn Gly Ser Glu Leu Lys Pro Ser Gln Val Ala Asn Glu
        35                  40                  45
Pro Arg Ile Glu Ile Ala Gly His Asp Met Arg Thr Leu Tyr Thr Leu
    50                  55                  60

<210> 133
<211> 201
<212> DNA
<213> Sorghum bicolor

<400> 133
atgttcaata tgtctaggga cccattggtc gtcgggcatg tcgtggggga tattgtggat 60
ccattcatca caactgcatc actgagggtg ttctacaaca ataaggagat gacaaatggt 120
tctgacctta agccatctca agtgatgaat gagccaaggg tccacatcag tgggcgtgac 180
atgaggactc tctacacact t 201

<210> 134
<211> 67
<212> PRT
<213> Sorghum bicolor

<400> 134
Met Phe Asn Met Ser Arg Asp Pro Leu Val Val Gly His Val Val Gly
1               5                   10                  15
Asp Ile Val Asp Pro Phe Ile Thr Thr Ala Ser Leu Arg Val Phe Tyr
            20                  25                  30
Asn Asn Lys Glu Met Thr Asn Gly Ser Asp Leu Lys Pro Ser Gln Val
        35                  40                  45
Met Asn Glu Pro Arg Val His Ile Ser Gly Arg Asp Met Arg Thr Leu
    50                  55                  60
Tyr Thr Leu
65

<210> 135
<211> 255
<212> DNA
<213> Sorghum bicolor

<400> 135
atgcagcgcg gggacccgct ggtggtgggg cgcatcatcg gcgacgtggt cgacccgttc 60
gtgcgccggg tgccgctccg cgtcgcctac gccgcgcgcg agatctccaa cggctgcgag 120
ctcaggccct ccgccatcgc cgaccagccg cgcgtcgagg tcggcggacc cgacatgcgc 180
accttctaca ccctcgtgat ggtagatcct gatgcgccaa gccccagcga tcccaacctc 240
agggagtacc tgcac 255

<210> 136
<211> 85
<212> PRT
<213> Sorghum bicolor

<400> 136
Met Gln Arg Gly Asp Pro Leu Val Val Gly Arg Ile Ile Gly Asp Val
1               5                   10                  15
Val Asp Pro Phe Val Arg Arg Val Pro Leu Arg Val Ala Tyr Ala Ala
            20                  25                  30
Arg Glu Ile Ser Asn Gly Cys Glu Leu Arg Pro Ser Ala Ile Ala Asp
        35                  40                  45
Gln Pro Arg Val Glu Val Gly Gly Pro Asp Met Arg Thr Phe Tyr Thr
    50                  55                  60
Leu Val Met Val Asp Pro Asp Ala Pro Ser Pro Ser Asp Pro Asn Leu
65                  70                  75                  80
Arg Glu Tyr Leu His
                85

<210> 137
<211> 255
<212> DNA
<213> Sorghum bicolor

<400> 137
atggcggcta acgattcctt ggttactgct catgtgatag gagatgtctt ggaccccttc 60
tatacaaccg ttgatatgat gatcctattc gatggtactc ctattatcag cggcatggag 120
ttgcgtgctc cggcggtttc tgacaggcca agggttgaga ttggaggaga tgattatcga 180
gttgcatata ctctggtgat ggtcgatcct gatgctccta acccaagcaa cccaaccttg 240
agggagtact tgcac 255

<210> 138
<211> 85
<212> PRT
<213> Sorghum bicolor

<400> 138
Met Ala Ala Asn Asp Ser Leu Val Thr Ala His Val Ile Gly Asp Val
1               5                   10                  15
Leu Asp Pro Phe Tyr Thr Thr Val Asp Met Met Ile Leu Phe Asp Gly
            20                  25                  30
Thr Pro Ile Ile Ser Gly Met Glu Leu Arg Ala Pro Ala Val Ser Asp
        35                  40                  45
Arg Pro Arg Val Glu Ile Gly Gly Asp Asp Tyr Arg Val Ala Tyr Thr
    50                  55                  60
Leu Val Met Val Asp Pro Asp Ala Pro Asn Pro Ser Asn Pro Thr Leu
65                  70                  75                  80
Arg Glu Tyr Leu His
                85

<210> 139
<211> 261
<212> DNA
<213> Sorghum bicolor

<400> 139
atgtcgacga cgtcaaggga cagcctggtg ctggggcggg tggtcggcga cgtggtggac 60
cagttctccg cgacggcggc gctccgggtc tcctataacg gccggcgcgt catcaacggc 120
tccgacctcc ggccgtcggc ggtggcagca aggcctcgca tcgagatcgg gggcaccgat 180
ttcaggcagt cctacacgct tgttatggtg gatcctgacg ctcccaaccc gagcaatccg 240
acgttgaggg agtatttgca t 261

<210> 140
<211> 87
<212> PRT
<213> Sorghum bicolor

<400> 140
Met Ser Thr Thr Ser Arg Asp Ser Leu Val Leu Gly Arg Val Val Gly
1               5                   10                  15
Asp Val Val Asp Gln Phe Ser Ala Thr Ala Ala Leu Arg Val Ser Tyr
            20                  25                  30
Asn Gly Arg Arg Val Ile Asn Gly Ser Asp Leu Arg Pro Ser Ala Val
        35                  40                  45
Ala Ala Arg Pro Arg Ile Glu Ile Gly Gly Thr Asp Phe Arg Gln Ser
    50                  55                  60
Tyr Thr Leu Val Met Val Asp Pro Asp Ala Pro Asn Pro Ser Asn Pro
65                  70                  75                  80

Thr Leu Arg Glu Tyr Leu His
                85

<210> 141
<211> 255
<212> DNA
<213> Sorghum bicolor

<400> 141
atggctgccc atgtggaccc gctggtggtg gggagggtga tcggcgatgt ggtggacctg 60
ttcgtgccga cggtggccat gtcggtgcgc ttcggcacca aggacgtaac caacggctgc 120
gagatcaagc catccctcac cgccgctgct ccggtcgtcc agattgccgg cagggccaac 180
gacctcttca ccctggttat gactgaccca gatgctccga gccccagcga gccaacgatg 240
agggagttga tccac                                                  255

<210> 142
<211> 85
<212> PRT
<213> Sorghum bicolor

<400> 142
Met Ala Ala His Val Asp Pro Leu Val Val Gly Arg Val Ile Gly Asp
 1               5                  10                  15
Val Val Asp Leu Phe Val Pro Thr Val Ala Met Ser Val Arg Phe Gly
            20                  25                  30
Thr Lys Asp Val Thr Asn Gly Cys Glu Ile Lys Pro Ser Leu Thr Ala
        35                  40                  45
Ala Ala Pro Val Val Gln Ile Ala Gly Arg Ala Asn Asp Leu Phe Thr
    50                  55                  60
Leu Val Met Thr Asp Pro Asp Ala Pro Ser Pro Ser Glu Pro Thr Met
65                  70                  75                  80
Arg Glu Leu Ile His
                85

<210> 143
<211> 252
<212> DNA
<213> Sorghum bicolor

<400> 143
atggctaatg actctctgac gaggggacac ataatcgggg atgtcttaga cccgtttact 60
agctcagtgc ctctaactgk catgtatgat ggcagaccgg tgtttgatgg gatggagttt 120
cgggcgtcgg cggtgtcggt gaaacctaga gttgagattg gaggtgatga ttttcgagtg 180
gcctataccc tagttatggt ggatcctgat gcgcctaatc ccagcaaccc taccctacgg 240
gaatacttgc at                                                     252

<210> 144
<211> 84
<212> PRT
<213> Sorghum bicolor

<220>
<221> UNSURE
<222> (27)...(27)
<223> Xaa = any amino acid

<400> 144
Met Ala Asn Asp Ser Leu Thr Arg Gly His Ile Ile Gly Asp Val Leu
 1               5                  10                  15

Asp Pro Phe Thr Ser Ser Val Pro Leu Thr Xaa Met Tyr Asp Gly Arg
            20                  25                  30
Pro Val Phe Asp Gly Met Glu Phe Arg Ala Ser Ala Val Ser Val Lys
        35                  40                  45
Pro Arg Val Glu Ile Gly Gly Asp Asp Phe Arg Val Ala Tyr Thr Leu
    50                  55                  60
Val Met Val Asp Pro Asp Ala Pro Asn Pro Ser Asn Pro Thr Leu Arg
65                  70                  75                  80
Glu Tyr Leu His

<210> 145
<211> 267
<212> DNA
<213> Sorghum bicolor

<400> 145
atggccggca gcggcaggga aagggagacg ctggtggttg gtagggtggt gggcgacgtg 60
ctggacccct tcgtccggac caccaacctc agggtcagct acggcaccag gaccgtatcc 120
aacggctgcg agctcaagcc gtccatggtg gtgaaccagc ccagggtcga ggtcggggga 180
cccgacatga ggaccttcta caccctcgtg atggtcgacc cggatgctcc gagcccaagc 240
gacccaaatc ttagggagta tctgcac                                     267

<210> 146
<211> 89
<212> PRT
<213> Sorghum bicolor

<400> 146
Met Ala Gly Ser Gly Arg Glu Arg Glu Thr Leu Val Val Gly Arg Val
1               5                   10                  15
Val Gly Asp Val Leu Asp Pro Phe Val Arg Thr Thr Asn Leu Arg Val
            20                  25                  30
Ser Tyr Gly Thr Arg Thr Val Ser Asn Gly Cys Glu Leu Lys Pro Ser
        35                  40                  45
Met Val Val Asn Gln Pro Arg Val Glu Val Gly Gly Pro Asp Met Arg
    50                  55                  60
Thr Phe Tyr Thr Leu Val Met Val Asp Pro Asp Ala Pro Ser Pro Ser
65                  70                  75                  80
Asp Pro Asn Leu Arg Glu Tyr Leu His
                85

<210> 147
<211> 411
<212> DNA
<213> Allium cepa

<400> 147
atgttgcgag agagagtagc aagggatcct ctagtcttgg gacagataat tggagatgtt 60
gtggatccgt ttaccaaatc cgtgaatctc aaagtagttt atggagataa ggaagtgagt 120
aatggcacaa gacttcgtca atcgatggtt ataaatcaac cacgtgttac cattgaagga 180
cgtgactcaa ggactcttta tagccttgtt atgataaacc ctgatgcacc aagcccaact 240
aatccaactc atagagaata cttacactgg ttggtgacgg acataccaga aacagtcgat 300
gcaagttatg gaaatgagat agtacaatat gagagtccat ggacgccaac tgggattcat 360
cgaattgtat ttgtactatt ccagcagcaa attcaacaaa cggtgtatgc a          411

<210> 148
<211> 137
<212> PRT

<213> Allium cepa

<400> 148
Met Leu Arg Glu Arg Val Ala Arg Asp Pro Leu Val Leu Gly Gln Ile
1               5                   10                  15
Ile Gly Asp Val Val Asp Pro Phe Thr Lys Ser Val Asn Leu Lys Val
            20                  25                  30
Val Tyr Gly Asp Lys Glu Val Ser Asn Gly Thr Arg Leu Arg Gln Ser
        35                  40                  45
Met Val Ile Asn Gln Pro Arg Val Thr Ile Glu Gly Arg Asp Ser Arg
    50                  55                  60
Thr Leu Tyr Ser Leu Val Met Ile Asn Pro Asp Ala Pro Ser Pro Thr
65                  70                  75                  80
Asn Pro Thr His Arg Glu Tyr Leu His Trp Leu Val Thr Asp Ile Pro
                85                  90                  95
Glu Thr Val Asp Ala Ser Tyr Gly Asn Glu Ile Val Gln Tyr Glu Ser
            100                 105                 110
Pro Trp Thr Pro Thr Gly Ile His Arg Ile Val Phe Val Leu Phe Gln
        115                 120                 125
Gln Gln Ile Gln Gln Thr Val Tyr Ala
    130                 135


<210> 149
<211> 528
<212> DNA
<213> Triticum aestivum

<400> 149
atgcatgccc agcgcgggga cccgctggtg gtggggcgcg tgatcggcga cgtggtggac 60
ccgttcgtgc ggcgggtggc gctgcgggtc ggctacgcgt ccagggacgt ggccaacggc 120
tgcgagctga ggccgtccgc catcgccgac ccgccgcgcg tcgaggtcgg cggcccggac 180
atgcgcacct tctacacgct ggtgatggtg gatccggatg ctccaagtcc cagcgatccc 240
agccttaggg agtacttgca ctggctggtc accgacatcc cggcgacgac aggagtgtct 300
tttgggaccg aggtggtgtg ctacgagggc ccgcggccgg tgctcgggat ccaccggctg 360
gtgttcctgc tcttccagca gctgggccgc cagacggtgt acgccccggg gtggcggcag 420
aacttcagca cccgcgactt cgccgagctc tacaacctcg gcctgcccgt cgccgccgtc 480
tacttcaact gccagaggga gaccggaacc ggcgggagaa ggatgtga              528

<210> 150
<211> 175
<212> PRT
<213> Triticum aestivum

<400> 150
Met His Ala Gln Arg Gly Asp Pro Leu Val Val Gly Arg Val Ile Gly
1               5                   10                  15
Asp Val Val Asp Pro Phe Val Arg Arg Val Ala Leu Arg Val Gly Tyr
            20                  25                  30
Ala Ser Arg Asp Val Ala Asn Gly Cys Glu Leu Arg Pro Ser Ala Ile
        35                  40                  45
Ala Asp Pro Pro Arg Val Glu Val Gly Gly Pro Asp Met Arg Thr Phe
    50                  55                  60
Tyr Thr Leu Val Met Val Asp Pro Asp Ala Pro Ser Pro Ser Asp Pro
65                  70                  75                  80
Ser Leu Arg Glu Tyr Leu His Trp Leu Val Thr Asp Ile Pro Ala Thr
                85                  90                  95
Thr Gly Val Ser Phe Gly Thr Glu Val Val Cys Tyr Glu Gly Pro Arg
            100                 105                 110
Pro Val Leu Gly Ile His Arg Leu Val Phe Leu Leu Phe Gln Gln Leu
        115                 120                 125

Gly Arg Gln Thr Val Tyr Ala Pro Gly Trp Arg Gln Asn Phe Ser Thr
    130                 135                 140
Arg Asp Phe Ala Glu Leu Tyr Asn Leu Gly Leu Pro Val Ala Ala Val
145                 150                 155                 160
Tyr Phe Asn Cys Gln Arg Glu Thr Gly Thr Gly Gly Arg Arg Met
                165                 170                 175

<210> 151
<211> 543
<212> DNA
<213> Triticum aestivum

<400> 151
atggcagccc atgtggatcc ccttgtggtt gggagggtga tcggtgacgt ggtggacatg 60
ttcgtgccca ccatgccggt gaccgtgcgc ttcgggacga aggacctgac gaacggctgc 120
gagatcaagc cgtccatcgc cgacgcggcg ccctcgatcc agatagccgg ccgggccggc 180
gatctcttca ccctggttat gactgatccg gacgcaccga gccccagcga gccaaccatg 240
aaggagtggc ttcactggct ggtggttaac atacctggtg gatcagatcc ttctcaaggg 300
gaggaggtgg tgccctacat gggtccgaag ccgccgttgg gcatccaccg ctacgtgctg 360
gtgctgttcc agcagaaggc gcgtgtgctg gcgccggctc ccggcggcga cacagcagcg 420
tcggccatgc gcgcgcggtt cagcacccgt gccttcgcag agcgccatga cctggggctc 480
cccgtcgccg ccatgtactt caacgcgcag aaggagccgg ccaaccgccg ccgccgctac 540
tag                                                               543

<210> 152
<211> 180
<212> PRT
<213> Triticum aestivum

<400> 152
Met Ala Ala His Val Asp Pro Leu Val Val Gly Arg Val Ile Gly Asp
 1               5                  10                  15
Val Val Asp Met Phe Val Pro Thr Met Pro Val Thr Val Arg Phe Gly
            20                  25                  30
Thr Lys Asp Leu Thr Asn Gly Cys Glu Ile Lys Pro Ser Ile Ala Asp
        35                  40                  45
Ala Ala Pro Ser Ile Gln Ile Ala Gly Arg Ala Gly Asp Leu Phe Thr
    50                  55                  60
Leu Val Met Thr Asp Pro Asp Ala Pro Ser Pro Ser Glu Pro Thr Met
65                  70                  75                  80
Lys Glu Trp Leu His Trp Leu Val Val Asn Ile Pro Gly Gly Ser Asp
                85                  90                  95
Pro Ser Gln Gly Glu Glu Val Val Pro Tyr Met Gly Pro Lys Pro Pro
            100                 105                 110
Leu Gly Ile His Arg Tyr Val Leu Val Leu Phe Gln Gln Lys Ala Arg
        115                 120                 125
Val Leu Ala Pro Ala Pro Gly Gly Asp Thr Ala Ala Ser Ala Met Arg
    130                 135                 140
Ala Arg Phe Ser Thr Arg Ala Phe Ala Glu Arg His Asp Leu Gly Leu
145                 150                 155                 160
Pro Val Ala Ala Met Tyr Phe Asn Ala Gln Lys Glu Pro Ala Asn Arg
                165                 170                 175
Arg Arg Arg Tyr
            180

<210> 153
<211> 519
<212> DNA
<213> Glycine max

<400> 153
atggcagcct ccgtggatcc cctagtggtt ggtcgcgtga tcggcgatgt ggtagacatg 60
ttcattcctt cagtcaacat gtccgtttac tttgggtcga agcacgtcac aaatggctgt 120
gacatcaagc catccattgc catcagccct cctaagctca ccctcaccgg caacatggat 180
aacctctaca cactggttat gactgatcct gacgcaccta gccccagtga accaagcatg 240
cgcgagtgga tacattggat cttagttgac atacctggag gaacaaaccc atttcgcgga 300
aaagagattg tttcatatgt gggaccaaga ccacctattg gaatacatcg ctatatcttt 360
gtgttgtttc aacagaaagg acctttaggt cttgtggagc aaccaccaac tcgagcaagc 420
ttcaacactc gttattttgc caggcaattg gacttgggac ttccagtggc cactgtctac 480
ttcaactctc aaaaagaacc tgctgttaag aggcgctga 519

<210> 154
<211> 172
<212> PRT
<213> Glycine max

<400> 154
Met Ala Ala Ser Val Asp Pro Leu Val Val Gly Arg Val Ile Gly Asp
1               5                   10                  15
Val Val Asp Met Phe Ile Pro Ser Val Asn Met Ser Val Tyr Phe Gly
            20                  25                  30
Ser Lys His Val Thr Asn Gly Cys Asp Ile Lys Pro Ser Ile Ala Ile
        35                  40                  45
Ser Pro Pro Lys Leu Thr Leu Thr Gly Asn Met Asp Asn Leu Tyr Thr
    50                  55                  60
Leu Val Met Thr Asp Pro Asp Ala Pro Ser Pro Ser Glu Pro Ser Met
65                  70                  75                  80
Arg Glu Trp Ile His Trp Ile Leu Val Asp Ile Pro Gly Gly Thr Asn
                85                  90                  95
Pro Phe Arg Gly Lys Glu Ile Val Ser Tyr Val Gly Pro Arg Pro Pro
            100                 105                 110
Ile Gly Ile His Arg Tyr Ile Phe Val Leu Phe Gln Gln Lys Gly Pro
        115                 120                 125
Leu Gly Leu Val Glu Gln Pro Pro Thr Arg Ala Ser Phe Asn Thr Arg
    130                 135                 140
Tyr Phe Ala Arg Gln Leu Asp Leu Gly Leu Pro Val Ala Thr Val Tyr
145                 150                 155                 160
Phe Asn Ser Gln Lys Glu Pro Ala Val Lys Arg Arg
                165                 170

<210> 155
<211> 522
<212> DNA
<213> Glycine max

<400> 155
atggcaagaa tgcctttaga gcctctaata gtggggagag tcataggaga agttcttgat 60
tcttttacca caagcacaaa aatgattgtg agttacaaca agaatcaagt ctacaatggc 120
catgaactct tcccttccac tgtcaacacc aagcccaagg ttgagattga gggtggtgat 180
atgaggtcct tctttacact gatcatgact gaccctgatg ttcctggccc tagtgaccct 240
tatctgagag agcacttgca ctggatagtg acagatattc caggcacaac agatgccaca 300
tttgggaaag agttggtgag ctatgagatc ccaaagccta atattgggat ccataggttt 360
gtgtttgtcc tgttcaagca aaagcgtaga cagtgtgtta ctccacccac ttcaaggdac 420
cacttcaaca cacgcaaatt cgcagcagag aacgaccttg ccctccctgt ggctgctgtc 480
tacttcaatg cacagaggga aacggctgca agaagacgct ag 522

<210> 156
<211> 173
<212> PRT

<213> Glycine max

<400> 156
Met Ala Arg Met Pro Leu Glu Pro Leu Ile Val Gly Arg Val Ile Gly
1               5                   10                  15
Glu Val Leu Asp Ser Phe Thr Thr Ser Thr Lys Met Ile Val Ser Tyr
            20                  25                  30
Asn Lys Asn Gln Val Tyr Asn Gly His Glu Leu Phe Pro Ser Thr Val
        35                  40                  45
Asn Thr Lys Pro Lys Val Glu Ile Glu Gly Gly Asp Met Arg Ser Phe
    50                  55                  60
Phe Thr Leu Ile Met Thr Asp Pro Asp Val Pro Gly Pro Ser Asp Pro
65                  70                  75                  80
Tyr Leu Arg Glu His Leu His Trp Ile Val Thr Asp Ile Pro Gly Thr
                85                  90                  95
Thr Asp Ala Thr Phe Gly Lys Glu Leu Val Ser Tyr Glu Ile Pro Lys
            100                 105                 110
Pro Asn Ile Gly Ile His Arg Phe Val Phe Val Leu Phe Lys Gln Lys
        115                 120                 125
Arg Arg Gln Cys Val Thr Pro Pro Thr Ser Arg Asp His Phe Asn Thr
    130                 135                 140
Arg Lys Phe Ala Ala Glu Asn Asp Leu Ala Leu Pro Val Ala Ala Val
145                 150                 155                 160
Tyr Phe Asn Ala Gln Arg Glu Thr Ala Ala Arg Arg Arg
                165                 170


<210> 157
<211> 522
<212> DNA
<213> Glycine max

<400> 157
atgtctaggc taatggaaca accacttgtt gtgggaagag tgataggaga agtggttgac 60
attttcagcc caagtgtaag aatgaatgtt acatattcca ctaagcaagt tgctaatggt 120
catgagttaa tgccttctac tattatggcc aagccacgcg ttgagattgg tggtgatgac 180
atgaggactg cttatacctt gatcatgaca gacccagatg ctccaagtcc tagtgatcca 240
catctgaggg aacatctcca ctggacggtt acagatatcc ctggcaccac agatgtctct 300
tttggaaaag agattgtagg ctatgagagt ccaaagccag taataggaat ccacaggtat 360
gtgttcatct tgttcaagca gagaggaaga caaacagtga ggcctccatc ttcaagagac 420
cacttcaaca caaggaggtt ctcagaagag aatggccttg gcctaccagt tgctgcagtt 480
tacttcaatg ctcaaagaga gactgctgca agaaggaggt ga                    522

<210> 158
<211> 173
<212> PRT
<213> Glycine max

<400> 158
Met Ser Arg Leu Met Glu Gln Pro Leu Val Val Gly Arg Val Ile Gly
1               5                   10                  15
Glu Val Val Asp Ile Phe Ser Pro Ser Val Arg Met Asn Val Thr Tyr
            20                  25                  30
Ser Thr Lys Gln Val Ala Asn Gly His Glu Leu Met Pro Ser Thr Ile
        35                  40                  45
Met Ala Lys Pro Arg Val Glu Ile Gly Gly Asp Asp Met Arg Thr Ala
    50                  55                  60
Tyr Thr Leu Ile Met Thr Asp Pro Asp Ala Pro Ser Pro Ser Asp Pro
65                  70                  75                  80
His Leu Arg Glu His Leu His Trp Thr Val Thr Asp Ile Pro Gly Thr
                85                  90                  95

Thr Asp Val Ser Phe Gly Lys Glu Ile Val Gly Tyr Glu Ser Pro Lys
            100                 105                 110
Pro Val Ile Gly Ile His Arg Tyr Val Phe Ile Leu Phe Lys Gln Arg
        115                 120                 125
Gly Arg Gln Thr Val Arg Pro Pro Ser Ser Arg Asp His Phe Asn Thr
    130                 135                 140
Arg Arg Phe Ser Glu Glu Asn Gly Leu Gly Leu Pro Val Ala Ala Val
145                 150                 155                 160
Tyr Phe Asn Ala Gln Arg Glu Thr Ala Ala Arg Arg Arg
                165                 170

<210> 159
<211> 225
<212> DNA
<213> Glycine max

<400> 159
gaagagattg tctcctatga aagtccacgt ccaatagtag ggattcatcg aatagttttt 60
gtgttatttc gtcagctgcg tagactaact ctgcaacctc caggctggcg ccagaatttc 120
aacactagag actttgctga gatttataat cttggattac cagtagcggc catgtacttc 180
aactgtaaac gagaaaatga tcaaagcagt ggaagaagaa gataa 225

<210> 160
<211> 74
<212> PRT
<213> Glycine max

<400> 160
Glu Glu Ile Val Ser Tyr Glu Ser Pro Arg Pro Ile Val Gly Ile His
1               5                   10                  15
Arg Ile Val Phe Val Leu Phe Arg Gln Leu Arg Arg Leu Thr Leu Gln
            20                  25                  30
Pro Pro Gly Trp Arg Gln Asn Phe Asn Thr Arg Asp Phe Ala Glu Ile
        35                  40                  45
Tyr Asn Leu Gly Leu Pro Val Ala Ala Met Tyr Phe Asn Cys Lys Arg
    50                  55                  60
Glu Asn Asp Gln Ser Ser Gly Arg Arg Arg
65                  70

<210> 161
<211> 228
<212> DNA
<213> Glycine max

<400> 161
cacgaggttg taacatatga aagtccgcga ccgatgatgg ggattcatcg tttagtgttt 60
gtgttatttc gtcaactggg tagggaaaca gtgtatgcac caggatggcg ccagaatttc 120
aacactagag aatttgctga actctacaac cttggattgc cagttgctgc tgtctatttc 180
aacattcaga gggaatctgg ctctggtgga agaaggttat accattga 228

<210> 162
<211> 75
<212> PRT
<213> Glycine max

<400> 162
His Glu Val Val Thr Tyr Glu Ser Pro Arg Pro Met Met Gly Ile His
1               5                   10                  15

Arg Leu Val Phe Val Leu Phe Arg Gln Leu Gly Arg Glu Thr Val Tyr
            20                  25                  30
Ala Pro Gly Trp Arg Gln Asn Phe Asn Thr Arg Glu Phe Ala Glu Leu
        35                  40                  45
Tyr Asn Leu Gly Leu Pro Val Ala Ala Val Tyr Phe Asn Ile Gln Arg
    50                  55                  60
Glu Ser Gly Ser Gly Gly Arg Arg Leu Tyr His
65                  70                  75

<210> 163
<211> 225
<212> DNA
<213> Glycine max

<400> 163
ggtaacgagg ttgtaagcta tgaaagccca cgacccacga tggggattca tcggttggtg 60
tttgtgttat tccgtcaaca gtttagacag agggtgtatg ctcctggatg gcgacaaaat 120
ttcaatacca gagaatttgc tgaactttac aaccttggat tgccggttgc tgctgtcttc 180
ttcaactgtc agagggaaag tggctctggt ggtagaacat tttga                 225

<210> 164
<211> 74
<212> PRT
<213> Glycine max

<400> 164
Gly Asn Glu Val Val Ser Tyr Glu Ser Pro Arg Pro Thr Met Gly Ile
 1               5                  10                  15
His Arg Leu Val Phe Val Leu Phe Arg Gln Gln Phe Arg Gln Arg Val
            20                  25                  30
Tyr Ala Pro Gly Trp Arg Gln Asn Phe Asn Thr Arg Glu Phe Ala Glu
        35                  40                  45
Leu Tyr Asn Leu Gly Leu Pro Val Ala Ala Val Phe Phe Asn Cys Gln
    50                  55                  60
Arg Glu Ser Gly Ser Gly Gly Arg Thr Phe
65                  70

<210> 165
<211> 147
<212> DNA
<213> Glycine max

<400> 165
atggctgcat ccggggatcc cctattggtt ggtcgcgtga taggtgatgt ggtagatatg 60
ttcattcctt ccttcaacat gttcgtttac tttgggtcgg agcatgtcac aaatggctat 120
gacattaagc catccatggc cataagc                                     147

<210> 166
<211> 49
<212> PRT
<213> Glycine max

<400> 166
Met Ala Ala Ser Gly Asp Pro Leu Leu Val Gly Arg Val Ile Gly Asp
 1               5                  10                  15
Val Val Asp Met Phe Ile Pro Ser Phe Asn Met Phe Val Tyr Phe Gly
            20                  25                  30
Ser Glu His Val Thr Asn Gly Tyr Asp Ile Lys Pro Ser Met Ala Ile
        35                  40                  45

Ser

<210> 167
<211> 486
<212> DNA
<213> Helianthus argophyllus

<400> 167
catatcagca tgtcccttgt cgtagggcgg gtgataggtg atgtcgtcga ccaattcaca 60
ccaagcgtgt cgatggatgt agtctataat ccccagtgcc cggtcttaaa cggccatgag 120
atcaagccta atctcattgc cactaaacct cgtgttaata tcggcggtgt tgacatgaga 180
tcatcttata ctcttatcat gactgacccc gatgctccaa gtccaagtga cccatacttg 240
agagaacatc ttcattggat tgtcacagac attcctggta caactgaagc aacttttgga 300
agggagattg ggagctatga aaaaccaaag ccagtgatag gaatccatcg ctatgtgttc 360
ttattgctca agcaaagagc taggcagtcg gggaggcgac cagttgtgcg agatcgattc 420
aacactcgtg ccttctctca agaaagagac ttggggttac ctgttgctgc tagctacttc 480
cttggg 486

<210> 168
<211> 162
<212> PRT
<213> Helianthus argophyllus

<400> 168
His Ile Ser Met Ser Leu Val Val Gly Arg Val Ile Gly Asp Val Val
1               5                   10                  15
Asp Gln Phe Thr Pro Ser Val Ser Met Asp Val Val Tyr Asn Pro Gln
            20                  25                  30
Cys Pro Val Leu Asn Gly His Glu Ile Lys Pro Asn Leu Ile Ala Thr
        35                  40                  45
Lys Pro Arg Val Asn Ile Gly Gly Val Asp Met Arg Ser Ser Tyr Thr
    50                  55                  60
Leu Ile Met Thr Asp Pro Asp Ala Pro Ser Pro Ser Asp Pro Tyr Leu
65                  70                  75                  80
Arg Glu His Leu His Trp Ile Val Thr Asp Ile Pro Gly Thr Thr Glu
                85                  90                  95
Ala Thr Phe Gly Arg Glu Ile Gly Ser Tyr Glu Lys Pro Lys Pro Val
            100                 105                 110
Ile Gly Ile His Arg Tyr Val Phe Leu Leu Leu Lys Gln Arg Ala Arg
        115                 120                 125
Gln Ser Gly Arg Arg Pro Val Val Arg Asp Arg Phe Asn Thr Arg Ala
    130                 135                 140
Phe Ser Gln Glu Arg Asp Leu Gly Leu Pro Val Ala Ala Ser Tyr Phe
145                 150                 155                 160
Leu Gly

<210> 169
<211> 522
<212> DNA
<213> Helianthus species

<400> 169
cccaagtgtg ttagcatgtc gcttgcagta gggagggtga ttggagatgt cgttgaccca 60
ttcacaccga gtgtgacgat ggaagtagcg tataactccc attacacggt ctctagtggg 120
cacgagctga tgcctaatat cattacttct aaacctcaag ttcatattgg cggtgttgac 180
atgcgatctg cttatactat tatcttgact gacccggatg cacccagtcc gagtgatcct 240
tacttgagag aacatctcca ttggatcgtc acagacattc ctggcacaac tgatgcaact 300

tttggaaggg agattgtgag ctatgaaaaa ccgaatccac ttataggcat ccaccgatac 360
gttttcttac tattcaaaca gagagcaagg aaatcagtta ggccacccgc ttccagagat 420
cagttcaata cacggaactt ctctcaagaa aacgacttag ggttaccggt tgctgctgtc 480
tacttcaatg ctcaaagagc aaatgccgca cgtagaagat aa 522

<210> 170
<211> 173
<212> PRT
<213> Helianthus species

<400> 170
Pro Lys Cys Val Ser Met Ser Leu Ala Val Gly Arg Val Ile Gly Asp
1               5                   10                  15
Val Val Asp Pro Phe Thr Pro Ser Val Thr Met Glu Val Ala Tyr Asn
            20                  25                  30
Ser His Tyr Thr Val Ser Ser Gly His Glu Leu Met Pro Asn Ile Ile
        35                  40                  45
Thr Ser Lys Pro Gln Val His Ile Gly Gly Val Asp Met Arg Ser Ala
    50                  55                  60
Tyr Thr Ile Ile Leu Thr Asp Pro Asp Ala Pro Ser Pro Ser Asp Pro
65                  70                  75                  80
Tyr Leu Arg Glu His Leu His Trp Ile Val Thr Asp Ile Pro Gly Thr
                85                  90                  95
Thr Asp Ala Thr Phe Gly Arg Glu Ile Val Ser Tyr Glu Lys Pro Asn
            100                 105                 110
Pro Leu Ile Gly Ile His Arg Tyr Val Phe Leu Leu Phe Lys Gln Arg
        115                 120                 125
Ala Arg Lys Ser Val Arg Pro Pro Ala Ser Arg Asp Gln Phe Asn Thr
    130                 135                 140
Arg Asn Phe Ser Gln Glu Asn Asp Leu Gly Leu Pro Val Ala Ala Val
145                 150                 155                 160
Tyr Phe Asn Ala Gln Arg Ala Asn Ala Ala Arg Arg Arg
                165                 170

<210> 171
<211> 339
<212> DNA
<213> Helianthus species

<400> 171
atgtcgagga gggagaggga cccgttggtc gttggacgtg tgataggaga tgttcttgat 60
agtttcacaa agtcgattaa ccttacgatt tcttacaacg acagggaagt tagcaacggg 120
tgcacactaa aaccctctca ggttgttaac cagcctcggg ttgatattgg aggtgacgac 180
ctacgagctt ttcacacttt agtcatggtg gatcctgatc tcccaagtcc aagtgaccct 240
aaccttaggg aatacttgca ttggttggtg actgatattc cagcgaccac tgggagcacg 300
ttttggtcaa gaaagttggt gtgctatgag agtccaagg 339

<210> 172
<211> 113
<212> PRT
<213> Helianthus species

<400> 172
Met Ser Arg Arg Glu Arg Asp Pro Leu Val Val Gly Arg Val Ile Gly
1               5                   10                  15
Asp Val Leu Asp Ser Phe Thr Lys Ser Ile Asn Leu Thr Ile Ser Tyr
            20                  25                  30
Asn Asp Arg Glu Val Ser Asn Gly Cys Thr Leu Lys Pro Ser Gln Val
        35                  40                  45

Val Asn Gln Pro Arg Val Asp Ile Gly Gly Asp Asp Leu Arg Ala Phe
    50                  55                  60
His Thr Leu Val Met Val Asp Pro Asp Leu Pro Ser Pro Ser Asp Pro
65                  70                  75                  80
Asn Leu Arg Glu Tyr Leu His Trp Leu Val Thr Asp Ile Pro Ala Thr
                85                  90                  95
Thr Gly Ser Thr Phe Trp Ser Arg Lys Leu Val Cys Tyr Glu Ser Pro
            100                 105                 110
Arg

<210> 173
<211> 534
<212> DNA
<213> Arabidopsis thaliana

<400> 173
atggagaata tgggaactag agtgatagag ccattgataa tggggagagt ggtaggagat 60
gttcttgatt tcttcactcc aacaactaag atgaatgtta gttataacaa gaagcaagtc 120
tccaatggcc atgagctctt tccttcttct gtttcctcca agcctagggt tgagatccat 180
ggtggtgatc tcagatcctt cttcactttg gtgatgatag acccagatgt tccaggtcct 240
agtgacccct ttctaaaaga acacctgcac tggatcgtta caaacattcc cggcacaaca 300
gatgctacgt ttggcaaaga ggtggtgagc tatgaattgc caaggccaag catagggata 360
cataggtttg tgtttgttct gttcaggcag aagcaaagac gtgttatctt tcctaatatc 420
ccttcgagag atcacttcaa cactcgtaaa tttgcggtcg agtatgatct tggtctccct 480
gtcgcggccg tcttctttaa cgcacaaaga gaaaccgctg cacgcaaacg ctag       534

<210> 174
<211> 177
<212> PRT
<213> Arabidopsis thaliana

<400> 174
Met Glu Asn Met Gly Thr Arg Val Ile Glu Pro Leu Ile Met Gly Arg
 1               5                  10                  15
Val Val Gly Asp Val Leu Asp Phe Phe Thr Pro Thr Thr Lys Met Asn
            20                  25                  30
Val Ser Tyr Asn Lys Lys Gln Val Ser Asn Gly His Glu Leu Phe Pro
        35                  40                  45
Ser Ser Val Ser Ser Lys Pro Arg Val Glu Ile His Gly Gly Asp Leu
    50                  55                  60
Arg Ser Phe Phe Thr Leu Val Met Ile Asp Pro Asp Val Pro Gly Pro
65                  70                  75                  80
Ser Asp Pro Phe Leu Lys Glu His Leu His Trp Ile Val Thr Asn Ile
                85                  90                  95
Pro Gly Thr Thr Asp Ala Thr Phe Gly Lys Glu Val Val Ser Tyr Glu
            100                 105                 110
Leu Pro Arg Pro Ser Ile Gly Ile His Arg Phe Val Phe Val Leu Phe
        115                 120                 125
Arg Gln Lys Gln Arg Arg Val Ile Phe Pro Asn Ile Pro Ser Arg Asp
    130                 135                 140
His Phe Asn Thr Arg Lys Phe Ala Val Glu Tyr Asp Leu Gly Leu Pro
145                 150                 155                 160
Val Ala Ala Val Phe Phe Asn Ala Gln Arg Glu Thr Ala Ala Arg Lys
                165                 170                 175
Arg

<210> 175

<211> 528
<212> DNA
<213> Arabidopsis thaliana

<400> 175
atggccagga tttcctcaga cccgcttatg gttgggagag tgatcggaga cgttgtggac 60
aattgtttgc aggcagtgaa aatgacggtg acctataatt ctgacaagca agtctacaat 120
ggccatgaac ttttcccttc tgtagttaca tacaaaccta aggttgaagt tcatgggggt 180
gacatgagat cattcttcac tttggttatg actgatcctg atgttcctgg acctagtgat 240
ccttatctga gagagcactt gcactggatt gttaccgata tcccggggac gactgatgta 300
tcatttggta aagagataat cgggtacgag atgcctcggc caaacatagg gatccaccgc 360
tttgtgtatt tgttgttcaa gcagacccgt agaggaagtg tggtgtctgt gccatcttac 420
agagaccaat tcaacactcg agagtttgct catgagaacg atcttggcct ccccgtcgcg 480
gctgttttct tcaactgcca gcgtgagacc gccgctagac gccgttga 528

<210> 176
<211> 175
<212> PRT
<213> Arabidopsis thaliana

<400> 176
Met Ala Arg Ile Ser Ser Asp Pro Leu Met Val Gly Arg Val Ile Gly
1               5                   10                  15
Asp Val Val Asp Asn Cys Leu Gln Ala Val Lys Met Thr Val Thr Tyr
            20                  25                  30
Asn Ser Asp Lys Gln Val Tyr Asn Gly His Glu Leu Phe Pro Ser Val
        35                  40                  45
Val Thr Tyr Lys Pro Lys Val Glu Val His Gly Gly Asp Met Arg Ser
    50                  55                  60
Phe Phe Thr Leu Val Met Thr Asp Pro Asp Val Pro Gly Pro Ser Asp
65                  70                  75                  80
Pro Tyr Leu Arg Glu His Leu His Trp Ile Val Thr Asp Ile Pro Gly
                85                  90                  95
Thr Thr Asp Val Ser Phe Gly Lys Glu Ile Ile Gly Tyr Glu Met Pro
            100                 105                 110
Arg Pro Asn Ile Gly Ile His Arg Phe Val Tyr Leu Leu Phe Lys Gln
        115                 120                 125
Thr Arg Arg Gly Ser Val Val Ser Val Pro Ser Tyr Arg Asp Gln Phe
    130                 135                 140
Asn Thr Arg Glu Phe Ala His Glu Asn Asp Leu Gly Leu Pro Val Ala
145                 150                 155                 160
Ala Val Phe Phe Asn Cys Gln Arg Glu Thr Ala Ala Arg Arg Arg
                165                 170                 175

<210> 177
<211> 534
<212> DNA
<213> Arabidopsis thaliana

<400> 177
atgtcaagag aaatagagcc actaatagtg ggaagagtga taggagatgt actcgaaatg 60
tttaatccaa gtgtgacaat gagagtcact ttcaattcca acacaatcgt atccaatggt 120
cacgagctcg cgccttctct tctcctctct aagcctcgcg ttgagatcgg tggccaagat 180
cttcgttcct tcttcacctt aatcatgatg gaccccgatg ccccgagtcc tagtaatcct 240
tatatgcgtg aatatctgca ttggatggtg acagatattc ccgggacaac cgatgcttct 300
tttgggagag agatagtgag atatgagacg cctaaaccgg tggcgggaat acacagatac 360
gtctttgcgc tattcaaaca gagagggagg caagctgtga aggcagcgcc ggaaactaga 420
gagtgtttca acacaaacgc tttctcttct tactttggtc tttctcaacc tgttgctgct 480
gtttacttca acgcccaacg tgaaactgct cctcgacgac gtccttctta ttaa 534

<210> 178
<211> 177
<212> PRT
<213> Arabidopsis thaliana

<400> 178
Met Ser Arg Glu Ile Glu Pro Leu Ile Val Gly Arg Val Ile Gly Asp
1               5                   10                  15
Val Leu Glu Met Phe Asn Pro Ser Val Thr Met Arg Val Thr Phe Asn
            20                  25                  30
Ser Asn Thr Ile Val Ser Asn Gly His Glu Leu Ala Pro Ser Leu Leu
        35                  40                  45
Leu Ser Lys Pro Arg Val Glu Ile Gly Gly Gln Asp Leu Arg Ser Phe
    50                  55                  60
Phe Thr Leu Ile Met Met Asp Pro Asp Ala Pro Ser Pro Ser Asn Pro
65                  70                  75                  80
Tyr Met Arg Glu Tyr Leu His Trp Met Val Thr Asp Ile Pro Gly Thr
                85                  90                  95
Thr Asp Ala Ser Phe Gly Arg Glu Ile Val Arg Tyr Glu Thr Pro Lys
            100                 105                 110
Pro Val Ala Gly Ile His Arg Tyr Val Phe Ala Leu Phe Lys Gln Arg
        115                 120                 125
Gly Arg Gln Ala Val Lys Ala Ala Pro Glu Thr Arg Glu Cys Phe Asn
    130                 135                 140
Thr Asn Ala Phe Ser Ser Tyr Phe Gly Leu Ser Gln Pro Val Ala Ala
145                 150                 155                 160
Val Tyr Phe Asn Ala Gln Arg Glu Thr Ala Pro Arg Arg Arg Pro Ser
                165                 170                 175
Tyr

<210> 179
<211> 528
<212> DNA
<213> Arabidopsis thaliana

<400> 179
atgtctataa atataagaga ccctcttata gtaagcagag ttgttggaga cgttcttgat 60
ccgtttaata gatcaatcac tctaaaggtt acttatggcc aaagagaggt gactaatggc 120
ttggatctaa ggccttctca ggttcaaaac aagccaagag ttgagattgg tggagaagac 180
ctcaggaact tctatacttt ggttatggtg gatccagatg ttccaagtcc tagcaaccct 240
cacctccgag aatatctcca ttggttggtg actgatatcc ctgctacaac tggaacaacc 300
tttggcaatg agattgtgtg ttacgaaaat ccaagtccca ctgcaggaat tcatcgtgtc 360
gtgtttatat tgtttcgaca gcttggcagg caaacagtgt atgcaccagg gtggcgccag 420
aacttcaaca ctcgcgagtt tgctgagatc tacaatctcg gccttcccgt ggccgcagtt 480
ttctacaatt gtcagaggga gagtggctgc ggaggaagaa gactttag 528

<210> 180
<211> 175
<212> PRT
<213> Arabidopsis thaliana

<400> 180
Met Ser Ile Asn Ile Arg Asp Pro Leu Ile Val Ser Arg Val Val Gly
1               5                   10                  15
Asp Val Leu Asp Pro Phe Asn Arg Ser Ile Thr Leu Lys Val Thr Tyr
            20                  25                  30
Gly Gln Arg Glu Val Thr Asn Gly Leu Asp Leu Arg Pro Ser Gln Val
        35                  40                  45

Gln Asn Lys Pro Arg Val Glu Ile Gly Gly Glu Asp Leu Arg Asn Phe
    50                  55                  60
Tyr Thr Leu Val Met Val Asp Pro Asp Val Pro Ser Pro Ser Asn Pro
65                  70                  75                  80
His Leu Arg Glu Tyr Leu His Trp Leu Val Thr Asp Ile Pro Ala Thr
                85                  90                  95
Thr Gly Thr Thr Phe Gly Asn Glu Ile Val Cys Tyr Glu Asn Pro Ser
            100                 105                 110
Pro Thr Ala Gly Ile His Arg Val Val Phe Ile Leu Phe Arg Gln Leu
        115                 120                 125
Gly Arg Gln Thr Val Tyr Ala Pro Gly Trp Arg Gln Asn Phe Asn Thr
    130                 135                 140
Arg Glu Phe Ala Glu Ile Tyr Asn Leu Gly Leu Pro Val Ala Ala Val
145                 150                 155                 160
Phe Tyr Asn Cys Gln Arg Glu Ser Gly Cys Gly Gly Arg Arg Leu
                165                 170                 175

<210> 181
<211> 528
<212> DNA
<213> Arabidopsis thaliana

<400> 181
atgtctttaa gtcgtagaga tcctcttgtg gtcggcagtg ttgttggaga tgttcttgat 60
cctttcacga ggttggtctc tcttaaggtc acttatggcc atagagaggt tactaatggc 120
ttggatctaa ggccttctca agttctgaac aaaccaatag tggagattgg aggagacgac 180
ttcagaaatt tctacacctt ggttatggtg gatccagatg tgccgagtcc aagcaaccct 240
caccaacgag aatatctcca ctggttggtg actgatatac ctgccaccac tggaaatgcc 300
tttggcaatg aggtggtgtg ctacgagagt ccacgtcccc cctcgggaat tcatcgtatt 360
gtgttggtat tgttccggca actcggaaga caaacggttt atgcaccggg gtggcgccaa 420
cagttcaaca ctcgtgagtt tgctgagatc tacaatcttg gtcttcctgt ggctgcctct 480
tacttcaact gccagaggga gaatggctgt gggggaagaa gaacgtag             528

<210> 182
<211> 175
<212> PRT
<213> Arabidopsis thaliana

<400> 182
Met Ser Leu Ser Arg Arg Asp Pro Leu Val Val Gly Ser Val Val Gly
1               5                   10                  15
Asp Val Leu Asp Pro Phe Thr Arg Leu Val Ser Leu Lys Val Thr Tyr
            20                  25                  30
Gly His Arg Glu Val Thr Asn Gly Leu Asp Leu Arg Pro Ser Gln Val
        35                  40                  45
Leu Asn Lys Pro Ile Val Glu Ile Gly Gly Asp Asp Phe Arg Asn Phe
    50                  55                  60
Tyr Thr Leu Val Met Val Asp Pro Asp Val Pro Ser Pro Ser Asn Pro
65                  70                  75                  80
His Gln Arg Glu Tyr Leu His Trp Leu Val Thr Asp Ile Pro Ala Thr
                85                  90                  95
Thr Gly Asn Ala Phe Gly Asn Glu Val Val Cys Tyr Glu Ser Pro Arg
            100                 105                 110
Pro Pro Ser Gly Ile His Arg Ile Val Leu Val Leu Phe Arg Gln Leu
        115                 120                 125
Gly Arg Gln Thr Val Tyr Ala Pro Gly Trp Arg Gln Gln Phe Asn Thr
    130                 135                 140
Arg Glu Phe Ala Glu Ile Tyr Asn Leu Gly Leu Pro Val Ala Ala Ser
145                 150                 155                 160

Tyr Phe Asn Cys Gln Arg Glu Asn Gly Cys Gly Gly Arg Arg Thr
                165                 170                 175

<210> 183
<211> 522
<212> DNA
<213> Arabidopsis thaliana

<400> 183
atggcggctt ctgttgatcc tttggtggtc ggaagagtga tcggagatgt gttggacatg 60
ttcatcccaa ccgccaatat gtctgtctac tttggcccca aacacatcac taacggctgc 120
gagatcaaac cctccaccgc agtcaatcct ccaaaagtca acatctcggg ccattccgat 180
gagctttaca ctctcgtgat gactgacccg gacgcaccta gcccaagcga gccgaacatg 240
agagaatggg tccactggat tgtcgtggat attcccggag gcacaaatcc ctcaagagga 300
aaagagatac ttccatacat ggaaccaagg ccaccagtgg ggattcaccg ttacatattg 360
gtacttttcc ggcaaaactc accggtgggt ctgatggtgc agcagcctcc atcacgagcc 420
aatttcagca cacgaatgtt cgctggacat ttcgatcttg gtctacctgt ggccactgtc 480
tatttcaacg cccaaaagga acctgcttca cgcagacgct ag                    522

<210> 184
<211> 173
<212> PRT
<213> Arabidopsis thaliana

<400> 184
Met Ala Ala Ser Val Asp Pro Leu Val Val Gly Arg Val Ile Gly Asp
 1               5                  10                  15
Val Leu Asp Met Phe Ile Pro Thr Ala Asn Met Ser Val Tyr Phe Gly
            20                  25                  30
Pro Lys His Ile Thr Asn Gly Cys Glu Ile Lys Pro Ser Thr Ala Val
        35                  40                  45
Asn Pro Pro Lys Val Asn Ile Ser Gly His Ser Asp Glu Leu Tyr Thr
    50                  55                  60
Leu Val Met Thr Asp Pro Asp Ala Pro Ser Pro Ser Glu Pro Asn Met
65                  70                  75                  80
Arg Glu Trp Val His Trp Ile Val Val Asp Ile Pro Gly Gly Thr Asn
                85                  90                  95
Pro Ser Arg Gly Lys Glu Ile Leu Pro Tyr Met Glu Pro Arg Pro Pro
            100                 105                 110
Val Gly Ile His Arg Tyr Ile Leu Val Leu Phe Arg Gln Asn Ser Pro
        115                 120                 125
Val Gly Leu Met Val Gln Gln Pro Pro Ser Arg Ala Asn Phe Ser Thr
    130                 135                 140
Arg Met Phe Ala Gly His Phe Asp Leu Gly Leu Pro Val Ala Thr Val
145                 150                 155                 160
Tyr Phe Asn Ala Gln Lys Glu Pro Ala Ser Arg Arg Arg
                165                 170